# EUROPÄISCHE PATENTSCHRIFT

(11) **EP 2 029 591 B1**
(45) Veröffentlichungstag und Bekanntmachung des Hinweises auf die Patenterteilung: **07.09.2011**
(21) Anmeldenummer: 07725745.9
(22) Anmeldetag: 01.06.2007
(51) Int. Cl.: C07D 471/04, A61K 31/4375, A61P 9/00

(54) **SUBSTITUIERTE 4-ARYL-1,4-DIHYDRO-1,6-NAPHTHYRIDINE UND IHRE VERWENDUNG**
SUBSTITUTED 4-ARYL-1,4-DIHYDRO-1,6-NAPHTHYRIDINES AND USE THEREOF
4-ARYL-1,4-DIHYDRO-1,6-NAPHTYRIDINE SUBSTITUÉE ET SON UTILISATION

(30) Priorität: 07.06.2006 DE 102006026585
(43) Veröffentlichungstag der Anmeldung: 04.03.2009
(73) Patentinhaber: Bayer Pharma Aktiengesellschaft, 13353 Berlin (DE)
(72) Erfinder: FIGUEROA PEREZ, Santiago, 51373 Leverkusen (DE); KOLKHOF, Peter, 42113 Wuppertal (DE); BÄRFACKER, Lars, 46047 Oberhausen (DE); FLAMME, Ingo, 51580 Reichshof (DE); SCHLEMMER, Karl-Heinz, 42113 Wuppertal (DE); GROSSER, Rolf, 51373 Leverkusen (DE); MÜNTER, Klaus, 42489 Wülfrath (DE); KNORR, Andreas, 40699 Erkrath (DE); NITSCHE, Adam, 50259 Pulheim (DE)
(86) Internationale Anmeldenummer: PCT/EP2007/004865
(87) Internationale Veröffentlichungsnummer: WO 2007/140934

(56) Entgegenhaltungen:
- EP-A1- 0 173 933
- WO-A-02/10164
- WO-A-2005/087740
- WO-A-2005/097118

## Beschreibung

Die vorliegende Anmeldung betrifft neue substituierte 4-Aryl-1,4-dihydro-1,6-naphthyridine, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von kardiovaskulären Erkrankungen.

Aldosteron spielt eine Schlüsselrolle in der Aufrechterhaltung der Flüssigkeits- und Elektrolythomöostase, indem es im Epithel des distalen Nephrons die Natriumretention und Kaliumsekretion fördert, was zur Konstanthaltung des Extrazellulärvolumens und damit zur Blutdruckregulation beiträgt. Daneben entfaltet Aldosteron direkte Effekte auf die Struktur und Funktion des Herz- und Gefäßsystems, wobei die dafür zugrunde liegenden Mechanismen noch nicht erschöpfend geklärt sind [R.E. Booth, J.P. Johnson, J.D. Stockand, Adv. Physiol. Educ. 26 (1),8-20 (2002)].

Aldosteron ist ein Steroidhormon, das in der Nebennierenrinde gebildet wird. Seine Produktion wird indirekt ganz wesentlich in Abhängigkeit von der Nierendurchblutung reguliert. Jede Abnahme der Nierendurchblutung führt in der Niere zu einer Ausschüttung des Enzyms Renin in den Blutkreislauf. Dieses wiederum aktiviert die Bildung von Angiotensin II, das einerseits verengend auf die arteriellen Blutgefäße wirkt, andererseits aber auch die Bildung von Aldosteron in der Nebennierenrinde stimuliert. Somit fungiert die Niere als Blutdruck- und damit indirekter Volumen-Sensor im Blutkreislauf und wirkt über das Renin-Angiotensin-Aldosteron-System kritischen Volumenverlusten entgegen, indem einerseits der Blutdruck gesteigert wird (Angiotensin II-Wirkung), andererseits durch verstärkte Rückresorption von Natrium und Wasser in der Niere der Füllungszustand des Gefäßsystems wieder ausgeglichen wird (Aldosteron-Wirkung).

Dieses Regelsystem kann in vielfältiger Weise krankhaft gestört sein. So führt eine chronische Minderdurchblutung der Nieren (z.B. infolge einer Herzinsuffizienz und des hierdurch verursachten Blutrückstaus im Venensystem) zu einer chronisch überhöhten Ausschüttung von Aldosteron. Diese wiederum zieht eine Expansion des Blutvolumens nach sich und verstärkt hiermit die Herzschwäche durch ein Volumenüberangebot an das Herz. Eine Stauung von Blut in den Lungen mit Atemnot und Ödembildung in den Extremitäten sowie Aszites und Pleuraergüsse können die Folge sein; die Nierendurchblutung sinkt weiter ab. Überdies führt die übersteigerte Aldosteron-Wirkung zu einer Minderung der Kalium-Konzentration im Blut und in der Extrazellulärflüssigkeit. In ohnehin vorgeschädigten Herzmuskeln kann die Unterschreitung eines kritischen Mindestwertes tödlich endende Herzrhythmusstörungen auslösen. Hierin dürfte eine der Hauptursachen des häufig bei Patienten mit Herzinsuffizienz eintretenden *plötzlichen Herztodes* zu suchen sein.

Zusätzlich wird Aldosteron auch für eine Reihe der typischerweise bei Herzinsuffizienten zu beobachtenden Umbauprozesse des Herzmuskels verantwortlich gemacht. Somit ist der *Hyperaldosteronismus* eine entscheidende Komponente in der Pathogenese und Prognose der Herzinsuffizienz, die ursprünglich durch unterschiedliche Schädigungen, wie z.B. einen Herzinfarkt, eine Herzmuskelentzündung oder Bluthochdruck, ausgelöst werden kann. Diese Annahme wird durch die Tatsache erhärtet, dass in umfangreichen klinischen Studien in Patientenkollektiven mit chronischer Herzinsuffizienz bzw. nach akutem Myokardinfarkt durch Anwendung von Aldosteron-Antagonisten die Gesamtmortalität deutlich gesenkt wurde [B. Pitt, F. Zannad, W.J. Remme et al., N. Engl. J. Med. 341, 709-717 (1999); B. Pitt, W. Remme, F. Zannad et al., N. Engl. J. Med. 348, 1309-1321 (2003)]. Dies konnte unter anderem durch eine Senkung der Inzidenz des plötzlichen Herztodes erreicht werden.

Neueren Studien zufolge findet man auch bei einem nicht unerheblichen Teil der Patienten, die unter einer essentiellen Hypertonie leiden, eine sogenannte normokaliämische Variante des primären Hyperaldosteronismus [Prävalenz bis 11% aller Hypertoniker: L. Seiler und M. Reincke, *Der Aldosteron-Renin-Quotient bei sekundärer Hypertonie,* Herz 2.8 686-691 (2003)]. Als beste Diagnostikmethode dient beim normokaliämischen Hyperaldosteronismus der Aldosteron/Renin-Quotient der entsprechenden Plasmakonzentrationen, so dass auch relative Aldosteron-Erhöhungen in Bezug auf die Renin-Plasmakonzentrationen diagnostizierbar und letztlich therapierbar werden. Deshalb ist ein im Zusammenhang mit einer essentiellen Hypertonie diagnostizierter Hyperaldosteronismus ein Ansatzpunkt für eine kausale und prophylaktisch sinnvolle Therapie.

Weitaus seltener als die oben aufgeführten Formen des Hyperaldosteronismus sind solche Krankheitsbilder, bei denen die Störung entweder in den Hormon-produzierenden Zellen der Nebenniere selbst zu finden ist oder deren Anzahl oder Masse durch eine Hyperplasie oder Wucherung vermehrt ist. Adenome oder diffuse Hyperplasien der Nebennierenrinde sind die häufigste Ursache des auch als *Conn-Syndrom* bezeichneten primären Hyperaldosteronismus, dessen Leitsymptome Hypertonie und hypokaliämische Alkalose sind. Auch hier steht neben der chirurgischen Entfernung des krankhaften Gewebes die medikamentöse Therapie mit Aldosteron-Antagonisten im Vordergrund [H.A. Kühn und J. Schirmeister (Hrsg.), Innere Medizin, 4. Aufl., Springer Verlag, Berlin, 1982].

Ein anderes typischerweise mit Erhöhung der Aldosteron-Konzentration im Plasma einhergehendes Krankheitsbild ist die fortgeschrittene Leberzirrhose. Die Ursache der Aldosteronerhöhung liegt hier vorwiegend im infolge der Leberfunktionsstörung eingeschränkten Abbau des Aldosterons. Volumenüberlastung, Ödeme und Hypokaliämie sind die typischen Folgen, die in der klinischen Praxis durch Aldosteron-Antagonisten erfolgreich gelindert werden können.

Die Wirkungen von Aldosteron werden über den in den Zielzellen intrazellulär lokalisierten Mineralokorticoid-Rezeptor vermittelt. Die bislang verfügbaren Aldosteron-Antagonisten besitzen wie Aldosteron selbst eine Steroid-Grundstruktur. Begrenzt wird die Anwendbarkeit derartiger steroidaler Antagonisten durch ihre Wechselwirkungen mit den Rezeptoren anderer Steroidhormone, die zum Teil zu erheblichen Nebenwirkungen wie Gynäkomastie und Impotenz und zum Abbrechen der Therapie führen [M.A. Zaman, S. Oparil, D.A. Calhoun, Nature Rev. Drug Disc. 1, 621-636 (2002)].

Die Anwendung wirkstarker, nicht-steroidaler und für den Mineralokorticoid-Rezeptor selektiver Antagonisten bietet die Möglichkeit, dieses Nebenwirkungsprofil zu umgehen und dadurch einen deutlichen Therapievorteil zu erzielen.

Aufgabe der vorliegenden Erfindung ist die Bereitstellung neuer Verbindungen, die als selektive Mineralokorticoid-Rezeptor-Antagonisten für die Behandlung von Erkrankungen, insbesondere von kardiovaskulären Erkrankungen, eingesetzt werden können.

In EP 0 133 530-A, EP 0 173 933-A, EP 0 189 898-A und EP 0 234 516-A werden 4-Aryl-substituierte 1,4-Dihydro-1,6-naphthyridine bzw. -naphthyridinone mit Calcium-antagonistischer Wirkung zur Behandlung von Gefäßerkrankungen offenbart. Über das pharmakologische Profil dieser Verbindungen wird unter anderem in G. Werner et al., Naunyn-Schmiedeberg's Arch. Pharmacol. 344 (3), 337-344 (1991) berichtet. Weiterhin werden 1,4-Dihydro-1,6-naphthyridin-Derivate in WO 02/10164 als Kaliumkanal-Öffner zur Behandlung unterschiedlicher, vor allem urologischer Erkrankungen beansprucht. 4-Fluorenonyl- bzw. 4-Chromenonyl-1,4-dihydropyridin-Derivate als Mineralokorticoid-Rezeptor-Antagonisten werden in WO 2005/087740 und WO 2007/009670 beschrieben. In WO 2006/066011 werden 4-Aryl-3-cyano-1,4-dihydropyridin-5-carbonsäureester und -amide als zum Teil duale Modulatoren von Steroidhormon-Rezeptoren und des L-Typ-Calciumkanals offenbart, und in WO 2005/097118 werden Verbindungen mit 4-Aryl-1,4-dihydropyridin-Kemstruktur als Aldosteron-Rezeptor-Antagonisten beansprucht.

Gegenstand der vorliegenden Erfindung sind Verbindungen der allgemeinen Formel (I) 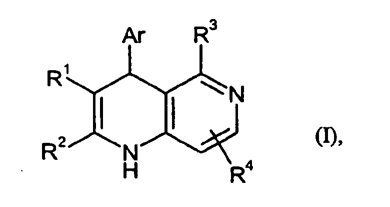 in welcher
Ar für eine Gruppe der Formel 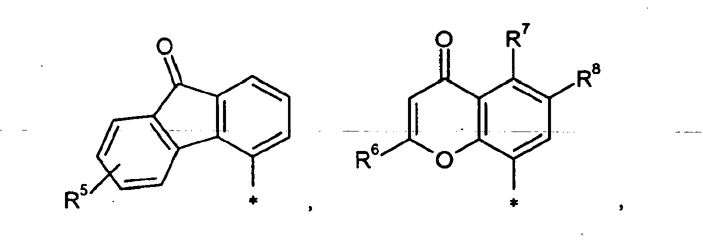 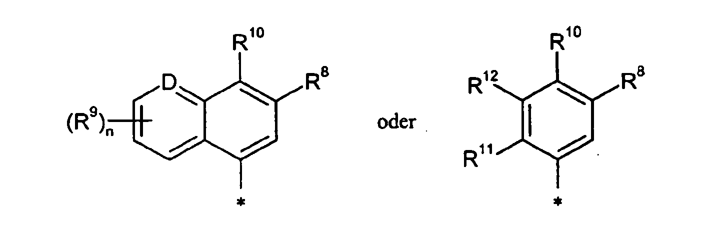 steht, worin
- *: die Verknüpfungsstelle bedeutet,
- R⁵: Wasserstoff oder Halogen bedeutet,
- R⁶: Methyl oder Ethyl bedeutet,
- R⁷: Wasserstoff, Fluor, Chlor, Cyano, Nitro, Trifluormethyl oder (C₁-C₄)-Alkyl bedeu- tet,
- R⁸: Wasserstoff oder Fluor bedeutet,
- R⁹: Halogen, (C₁C₄)-Alkyl, Trifluormethyl, (C₁-C₄)-Alkoxy oder Trifluormethoxy be- deutet,
- R¹⁰: Cyano oder Nitro bedeutet,
- R¹¹: Wasserstoff, Halogen, (C₁-C₄)-Alkyl, (C₁-C₄)-Alkoxy, (C₁-C₄)-Alkylthio oder Di- (C₁-C₄)-alkylamino, wobei die Alkylgruppe in den genannten (C₁-C₄)-Alkyl-, (C₁- C₄)-Alkoxy- und (C₁-C₄)-Alkylthio-Resten jeweils bis zu dreifach mit Fluor substi- tuiert sein kann,
oder

Phenyl, welches mit Halogen, (C₁-C₄)-Alkyl oder Trifluormethyl substituiert sein kann, bedeutet,
- R¹²: Wasserstoff, Halogen oder (C₁-C₄)-Alkyl bedeutet,
- D: CH, C-R⁹ oder N bedeutet
und
- n: die Zahl 0, 1 oder 2 bedeutet,
wobei für den Fall, dass der Substituent R⁹ mehrfach auftritt, seine Bedeutungen gleich oder verschieden sein können,
- R¹: für Cyano, Nitro oder eine Gruppe der Formel -C(=O)-R¹³ steht, worin

- R¹³: (C₁-C₆)-Alkyl, das mit (C₃-C₇)-Cycloalkyl oder ein- bis dreifach mit Fluor substitu- iert sein kann, oder Phenyl, das mit Halogen, Cyano, (C₁-C₄)-Alkyl, Trifluor- methyl, (C₁-C₄)-Alkoxy oder Trifluormethoxy substituiert sein kann, oder (C₃-C₇)- Cycloalkyl bedeutet,

- R²: für (C₁-C₄)-Alkyl, Trifluormethyl, Cyclopropyl, Cyclobutyl, (C₁-C₄)-Alkoxy oder (C₁-C₄)- Alkylthio steht,
- R³: für (C₁-C₆)-Alkyl, (C₁-C₆)-Alkoxy, Trifluormethoxy, (C₁-C₆)-Atkylthio, Amino, Mono-(C₁- C₆)-alkylamino oder eine Gruppe der Formel -O-SO₂-R¹⁴ steht, wobei die genannten (C₁-C₆)-Alkyl-, (C₁-C₆)-Alkoxy- und (C₁-C₆)-Alkylthio-Reste jeweils mit (C₃-C₇)-Cycloalkyl substituiert sein können
und
- R¹⁴: (C₁-C₆)-Alkyl, Trifluormethyl, (C₃-C₇)-Cycloalkyl, Phenyl oder 5- oder 6-gliedri- ges Heteroaryl mit bis zu zwei Heteroatomen aus der Reihe N, O und/oder S be- deutet, wobei Phenyl und Heteroaryl ihrerseits jeweils ein- oder zweifach, gleich oder ver- schieden, mit Halogen, Cyano, Nitro, (C₁-C₄)-Alkyl, Trifluormethyl, (C₁-C₄)- Alkoxy und/oder Trifluormethoxy substituiert sein können,
und
- R⁴: für Wasserstoff, Fluor, (C₁-C₄)-Alkyl, Trifluormethyl, (C₁-C₄)-Alkoxy, Trifluormethoxy, (C₁-C₄)-Alkylthio, Amino, Mono-(C₁-C₄)-alkylamino oder Di-(C₁-C₄)-alkylamino steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:
(C₁-C₆)-Alkyl, (C₁-C₄)-Alkyl und (C₁-C₃)-Alkyl stehen im Rahmen der Erfindung für einen geradkettigen oder verzweigten Alkylrest mit 1 bis 6, 1 bis 4 bzw. 1 bis 3 Kohlenstoffatomen. Bevorzugt ist ein geradkettiger oder verzweigter Alkylrest mit 1 bis 4, besonders bevorzugt mit 1 bis 3 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl, iso-Pentyl und n-Hexyl.
(C₃-C₇)-Cycloalkyl und (C₃-C₆-Cycloalkyl stehen im Rahmen der Erfindung für eine monocyclische, gesättigte Cycloalkylgruppe mit 3 bis 7 bzw. 3 bis 6 Kohlenstoffatomen. Bevorzugt ist ein Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.
(C₁-C₆)-Alkoxy, (C₁-C₄)-Alkoxy und (C₁-C₃)-Alkoxy stehen im Rahmen der Erfindung für einen geradkettigen oder verzweigten Alkoxyrest mit 1 bis 6, 1 bis 4 bzw. 1 bis 3 Kohlenstoffatomen. Bevorzugt ist ein geradkettiger oder verzweigter Alkoxyrest mit 1 bis 4, besonders bevorzugt mit 1 bis 3 Kohlenstoffatomen. Beispielhaft und vorzugsweise seien genannt: Methoxy, Ethoxy, n-Propoxy, Isopropoxy, n-Butoxy, tert.-Butoxy, n-Pentoxy und n-Hexoxy.
(C₁-C₆)-Alkylthio und (C₁C₄)-Alkylthio stehen im Rahmen der Erfindung für einen geradkettigen oder verzweigten Alkylthiorest mit 1 bis 6 bzw. 1 bis 4 Kohlenstoffatomen. Bevorzugt ist ein geradkettiger oder verzweigter Alkylthiorest mit 1 bis 4 Kohlenstoffatomen. Beispielhaft und vorzugsweise seien genannt: Methylthio, Ethylthio, n-Propylthio, Isopropylthio, n-Butylthio, tert.-Butylthio, n-Pentylthio und n-Hexylthio.
Mono-(C₁-C₆)-alkylamino und Mono-(C₁-C₄)-alkylamino stehen im Rahmen der Erfmdung für eine Amino-Gruppe mit einem geradkettigen oder verzweigten Alkylsubstituenten, der 1 bis 6 bzw. 1 bis 4 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, n-Propylamino, Isopropylamino, n-Butylamino, tert.-Butylamino, n-Pentylamino und n-Hexylamino.
Di-(C₁-C₆)-alkylamino und Di-(C₁-C₄)-alkylamino stehen im Rahmen der Erfindung fiir eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N*,*N*-Dimethylamino, *N*,*N*-Diethylamino, *N*-Ethyl-*N*-methylamino, *N*-Methyl-*N*-n-propylamino, *N*-Isopropyl-*N*-n-propylamino, *N*-tert.-Butyl-*N*-methylamino, *N*-Ethyl-*N*-n-pentylamino und *N*-n-Hexyl-*N*-methylamino.
5- oder 6-gliedriges Heteroaryl steht im Rahmen der Erfmdung für einen aromatischen Heterocyclus (Heteroaromaten) mit 5 oder 6 Ringatomen, der ein oder zwei Ring-Heteroatome aus der Reihe N, O und/oder S enthält und über ein Ring-Kohlenstoffatom verknüpft ist. Beispielhaft und vorzugsweise seien genannt: Furyl, Pyrrolyl, Thienyl, Pyrazolyl, Imidazolyl, Thiazolyl, Oxazolyl, Isoxazolyl, Isothiazolyl, Pyridyl, Pyrimidinyl, Pyridazinyl und Pyrazinyl.
Halogen schließt im Rahmen der Erfindung Fluor, Chlor, Brom und Iod ein. Bevorzugt sind Fluor oder Chlor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt sind Verbindungen der Formel (I), in welcher
- Ar: für eine Gruppe der Formel 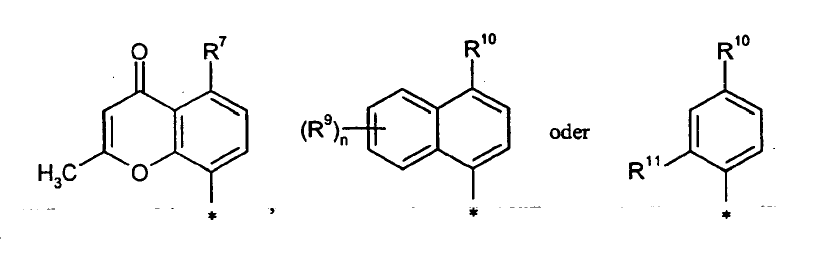
steht, worin
- *: die Verknüpfungsstelle bedeutet,
- R⁷: Wasserstoff, Fluor, Chlor oder Cyano bedeutet,
- R⁹: Fluor, Chlor, Methyl oder Ethyl bedeutet,
- R¹⁰: Cyano oder Nitro bedeutet,
- R¹¹: Chlor, Brom, (C₁-C₄)-Alkyl, Trifluormethyl, (C₁-C₄)-Alkoxy, Trifluormethoxy, (C₁-C₄)-Alkylthio oder Trifluormethylthio bedeutet,
und
- n: die Zahl 0 oder 1 bedeutet,

- R¹: für Cyano, Acetyl oder Trifluoracetyl steht,
- R²: für Methyl oder Trifluormethyl steht,
- R³: für (C₁-C₄)-Alkoxy, Trifluormethoxy oder eine Gruppe der Formel -O-SO₂-R¹⁴ steht, worin

- R¹⁴: (C₁C₄)-Alkyl, Trifluormethyl, (C₃-C₆)-Cycloalkyl, Phenyl oder Thienyl bedeutet, wobei Phenyl und Thienyl ihrerseits jeweils ein- oder zweifach, gleich oder ver- schieden, mit Fluor, Chlor, Cyano, Methyl, Trifluormethyl, Methoxy und/oder Tri- fluormethoxy substituiert sein können,
und
- R⁴: für Wasserstoff, Fluor oder Methyl steht,
sowie ihre Salze, Solvate und Solvate der Salze.

Besonders bevorzugt sind Verbindungen der Formel (I), in welcher
- Ar: für eine Gruppe der Formel 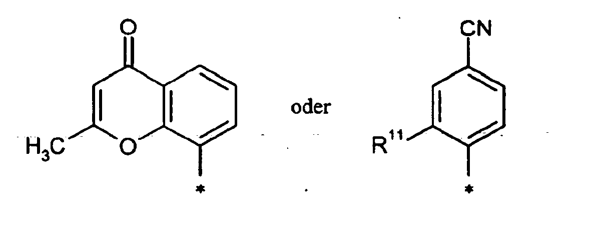 steht, worin
*
die Verknüpfungsstelle
und
R¹¹ Ethyl, Methoxy oder Trifluormethoxy bedeutet,
- R¹: für Cyano oder Acetyl steht,
- R²: für Methyl oder Trifluormethyl steht,
- R³: für (C₁-C₃)-Alkoxy oder eine Gruppe der Formel -O-SO₂-R¹⁴ steht, worin
R¹⁴ (C₁-C₃)-Alkyl bedeutet,
und
- R⁴: für Wasserstoff oder Methyl steht,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), in welchen R³ für gegebenenfalls mit (C₃-C₇)-Cycloalkyl substituiertes (C₁-C₆)-Alkoxy, Trifluormethoxy oder eine Gruppe der Formel -O-SO₂-R¹⁴ steht, worin R¹⁴ die oben angegebene Bedeutung hat, dadurch gekennzeichnet, dass man eine Verbindung der Formel (II) 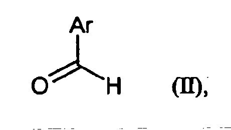 in welcher Ar die oben angegebene Bedeutung hat,
entweder
[A] in einem einstufigen (Eintopf-Reaktion) oder zweistufigen Verfahren mit einer Verbindung der Formel (III) 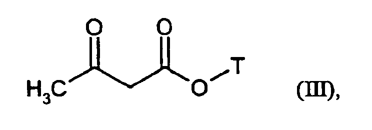 in welcher
   - T: für Methyl oder Ethyl steht,
   und einer Verbindung der Formel (IV) 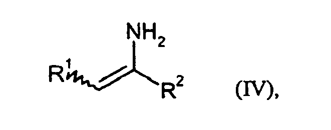 in welcher R¹ und R² die oben angegebenen Bedeutungen haben,
   zu einer Verbindung der Formel (V) 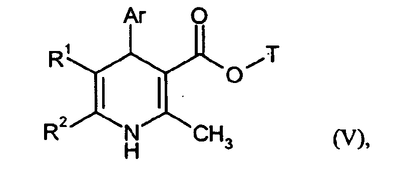 in welcher Ar, R¹, R² und T jeweils die oben angegebenen Bedeutungen haben,
   umsetzt und diese dann in einem inerten Lösungsmittel mit s-Triazin in Gegenwart einer Base zu einer Verbindung der Formel (VIa) 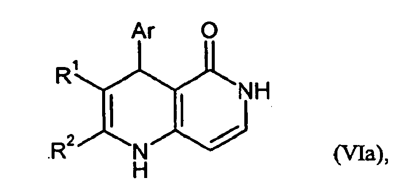 in welcher Ar, R¹ und R² jeweils die oben angegebenen Bedeutungen haben,
   reagiert
   oder
[B] in einem einstufigen (Eintopf-Reaktion) oder zweistufigen Verfahren mit einer Verbindung der Formel (VII) 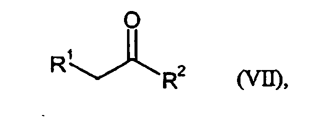 in welcher R¹ und R² die oben angegebenen Bedeutungen haben,
   und einer Verbindung der Formel (VIII) 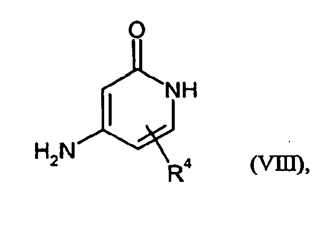 in welcher R⁴ die oben angegebene Bedeutung hat,
   zu einer Verbindung der Formel (VI) 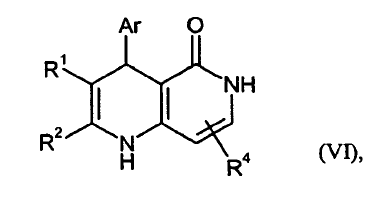 in welcher Ar, R¹, R² und R⁴ jeweils die oben angegebenen Bedeutungen haben,
   umsetzt und anschließend die Verbindungen der Formel (VIa) bzw. (VI) in einem inerten Lösungsmittel gegebenenfalls in Gegenwart einer Base mit einer Verbindung der Formel (IX) oder einem Trialkyloxonium-Salz der Formel (X) 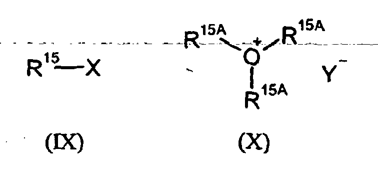 in welchen
   - R¹⁵: für (C₁-C₆)-Alkyl, das mit (C₃-C₇)-Cycloalkyl substituiert sein kann, oder für Trifluor- methyl steht,
   - R^{15A}: für Methyl oder Ethyl steht,
   - X: für eine Abgangsgruppe, wie beispielsweise Halogen, Mesylat, Tosylat oder Triflat, steht
   und
   - Y⁻: für ein nicht-nukleophiles Anion, wie beispielsweise Tetrafluoroborat, steht,
   oder in Gegenwart einer Säure mit einem Orthoameisensäureester der Formel (XI) 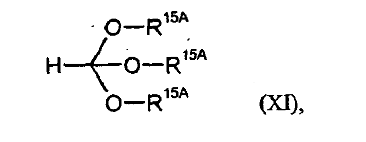 in welcher R^{15A} die oben angegebene Bedeutung hat,
   zu Verbindungen der Formel (I-A) 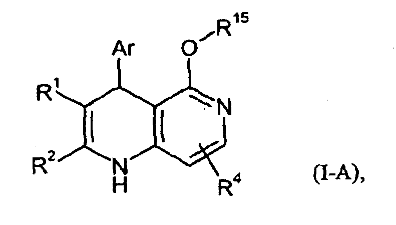 in welcher Ar, R¹, R², R⁴ und R¹⁵ jeweils die oben angegebenen Bedeutungen haben, alkyliert
   oder die Verbindungen der Formel (VIa) bzw. (VI) in einem inerten Lösungsmittel in Gegenwart einer Base mit einer Verbindung der Formel (XII) 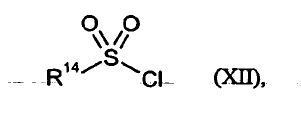 in welcher R¹⁴ die oben angegebene Bedeutung hat,
   zu Verbindungen der Formel (I-B) 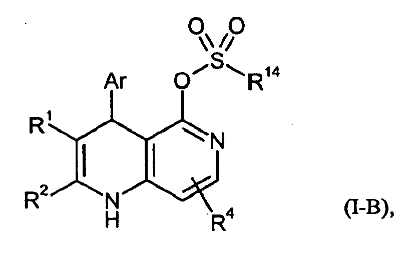 in welcher Ar, R¹, R², R⁴ und R¹⁴ jeweils die oben angegebenen Bedeutungen haben,
   umsetzt
   und gegebenenfalls die resultierenden Verbindungen der Formel (I-A) bzw. (I-B) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

Die Verfahrensschritte (II) + (III) + (IV) → (V) und (II) + (VII) + (VIII) → (VI) werden im Allgemeinen in einem inerten Lösungsmittel in einem Temperaturbereich von +20°C bis zum Siedepunkt des Lösungsmittels bei Normaldruck durchgeführt.

Als inerte Lösungsmittel eignen sich hierfür beispielsweise Alkohole wie Methanol, Ethanol, n-Propanol, Isopropanol, n-Butanol oder *tert*.-Butanol, Halogenkohlenwasserstoffe wie Dichlormethan, Trichlormethan, Tetrachlormethan, Trichlorethan oder 1,2-Dichlorethan, oder andere Lösungsmittel wie Acetonitril, Tetrahydrofuran, Dioxan, 1,2-Dimethoxyethan, Hexan, Benzol, Toluol, Chlorbenzol, Pyridin oder Eisessig. Bevorzugt werden die Umsetzungen in Dichlormethan, Toluol, Ethanol oder Isopropanol bei der jeweiligen Rückfluss-Temperatur unter Normaldruck durchgeführt.

Die Verfahrensschritte (II) + (III) + (IV) → (V) und (II) + (VII) + (VIII) → (VI) können gegebenenfalls vorteilhaft in Gegenwart einer Säure, einer SäureBase-Kombination und/oder eines wasserentziehenden Mittels, wie beispielsweise Molekularsieb, erfolgen. Als Säuren eignen sich beispielsweise Essigsäure, Trifluoressigsäure, Methansulfonsäure oder p-Toluolsulfonsäure; als Basen sind insbesondere Piperidin oder Pyridin geeignet [vgl. nachfolgendes Reaktionsschema 8; zur Synthese von 1,4-Dihydropyridinen vgl. auch D.M. Stout, A.I. Meyers, Chem. Rev. 1982, 82, 223-243; H. Meier et al., Liebigs Ann. Chem. 1977, 1888; H. Meier et al., ibid. 1977, 1895; H. Meier et al., ibid. 1976, 1762; F. Bossert et al., Angew. Chem. 1981, 93, 755].

Die Umsetzung mit s-Triazin im Verfahrensschritt (V) → (VIa) wird bevorzugt in *N*,*N*-Dimethylformamid unter Verwendung von Natriumhydrid als Base durchgeführt [vgl. J. Kleinschroth et al., Synthesis 1986, 859-860].

Gegebenenfalls kann eine Trennung der Enantiomere und/oder Diastereomere bereits auf der Stufe der Intermediate (VIa) bzw. (VI) erfolgen, welche dann separat den nachfolgenden Umsetzungen unterworfen werden (vgl. Reaktionsschema 9).

Inerte Lösungsmittel für die Verfahrensschritte (VI) + (IX) → (I-A), (VI) + (X) → (I-A) und (VI) + (XII) → (I-B) sind beispielsweise Ether wie Diethylether, Methyl-*tert*.-butylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethan, Tetrachlorethan, Trichlorethylen, Chlorbenzol oder Chlortoluol, oder andere Lösungsmittel wie *N*,*N*-Dimethylformamid (DMF), Dimethylsulfoxid (DMSO), *N*,*N*'-Dimethylpropylenharnstoff (DMPU), *N-*Methylpyrrolidon (NMP), Pyridin oder Acetonitril. Ebenso ist es möglich, Gemische der genannten Lösungsmittel zu verwenden. Bevorzugt werden im Verfahrensschritt (VI) + (IX) → (I-A) Tetrahydrofuran oder Dimethylformamid, im Verfahrensschritt (VI) + (X) → (I-A) Dichlormethan und im Verfahrensschritt (VI) + (XII) → (I-B) Pyridin eingesetzt.

Die Verfahrensvariante (VI) + (XI) → (I-A) wird vorzugsweise mit einem großen Überschuss an Orthoameisensäureester ohne Zusatz eines weiteren Lösungsmittels durchgeführt; als Reaktionskatalysator sind beispielsweise starke anorganische Säuren wie Schwefelsäure von Vorteil [vgl. z.B. I.I. Barabanov et al., Russ. Chem. Bl. 47 (11), 2256-2261 (1998)].

Als Basen für den Verfahrensschritt (VI) + (IX) → (I-A) eignen sich insbesondere Alkali- oder Erdalkalicarbonate wie Lithium-, Natrium-, Kalium-, Calcium- oder Cäsiumcarbonat, Alkalihydride wie Natrium- oder Kaliumhydrid, Amide wie Lithium-, Natrium- oder Kalium-bis-(trimethylsilyl)amid oder Lithiumdiisopropylamid, metallorganische Verbindungen wie Butyllithium oder Phenyllithium, oder auch Phosphazen-Basen wie beispielsweise P2-t-Bu oder P4-t-Bu [so genannte "Schwesinger-Basen", vgl. R. Schwesinger, H. Schlemper, Angew. Chem. Int. Ed. Engl. 26, 1167 (1987); T. Pietzonka, D. Seebach, Chem. Ber. 124, 1837 (1991)]. Bevorzugt wird Natriumhydrid oder die Phosphazen-Base P4-t-Bu verwendet.

Als Basen für den Verfahrensschritt (VI) + (XII) → (I-B) eignen sich insbesondere Alkali- oder Erdalkalicarbonate wie Lithium-, Natrium-, Kalium-, Calcium- oder Cäsiumcarbonat, Alkalihydride wie Natrium- oder Kaliumhydrid, metallorganische Verbindungen wie Butyllithium oder Phenyllithium, oder organische Amine wie Triethylamin, *N*-Methylmorpholin, *N*-Methylpiperidin, *N*,*N*-Diisopropylethylamin, Pyridin, 1,5-Diazabicyclo[4.3.0]non-5-en (DBN), 1,8-Diazabicyclo-[5.4.0]undec-7-en (DBU) oder 1,4-Diazabicyclo[2.2.2]octan (DABCO^{®}). Bevorzugt wird Pyridin verwendet, das gleichzeitig auch als Lösungsmittel dient.

Der Verfahrensschritt (VI) + (X) → (I-A) wird im Allgemeinen ohne Zusatz einer Base durchgeführt,

Die Umsetzungen (VI) + (IX) → (I-A), (VI) + (X) → (I-A) und (VI) + (XII) → (I-B) erfolgen im Allgemeinen in einem Temperaturbereich von -20°C bis +100°C, bevorzugt bei 0°C bis +50°C; die Verfahrensvariante (VI) + (XI) → (I-A) wird in der Regel in einem Temperaturbereich von +100°C bis +150°C durchgeführt. Die Reaktionen können bei normalem, bei erhöhtem oder bei vermindertem Druck erfolgen (z.B. von 0.5 bis 5 bar); im Allgemeinen arbeitet man bei Normaldruck.

Die Verbindungen der Formel (II) sind kommerziell erhältlich, literaturbekannt oder können in Analogie zu literaturbekannten Verfahren hergestellt werden (vgl. nachfolgende Reaktionsschemata 1-7). Die Verbindungen der Formeln (III), (IV), (VII), (IX), (X), (XI) und (XII) sind vielfach kommerziell erhältlich, literaturbekannt oder nach literaturbekannten Methoden herstellbar.

Die Verbindungen der Formel (VIII) sind literaturbeschrieben oder können in Analogie zu literaturbekannten Verfahren erhalten werden [vgl. z.B. T. Searls, L.W. McLaughlin, Tetrahedron 55, 11985-11996 (1999); D. McNamara, P.D. Cook, J. Med. Chem. 30, 340-347 (1987); S. Nesnow, C. Heidelberger, J. Heterocycl. Chem. 12, 941-944 (1975); N.C. Hung, E. Bisagni, Synthesis 1984, 765-766].

Die erfindungsgemäßen Verbindungen der Formel (I), in welchen R³ für (C₁-C₆)-Alkyl oder Mono-(C₁-C₆)-alkylamino steht, können in Analogie zu literaturbekannten Methoden beispielsweise ausgehend von Verbindungen der Formel (I-B), in welchen R¹⁴ für Trifluormethyl steht, erhalten werden (siehe Reaktionsschema 10).

Die erfindungsgemäßen Verbindungen der Formel (I), in welchen R³ für (C₁-C₆)-Alkylthio steht, können in Analogie zu literaturbekannten Methoden beispielsweise ausgehend von Verbindungen der Formel (VI) erhalten werden (siehe Reaktionsschema 11).

Die Herstellung der erfindungsgemäßen Verbindungen kann durch die folgenden Syntheseschemata veranschaulicht werden: 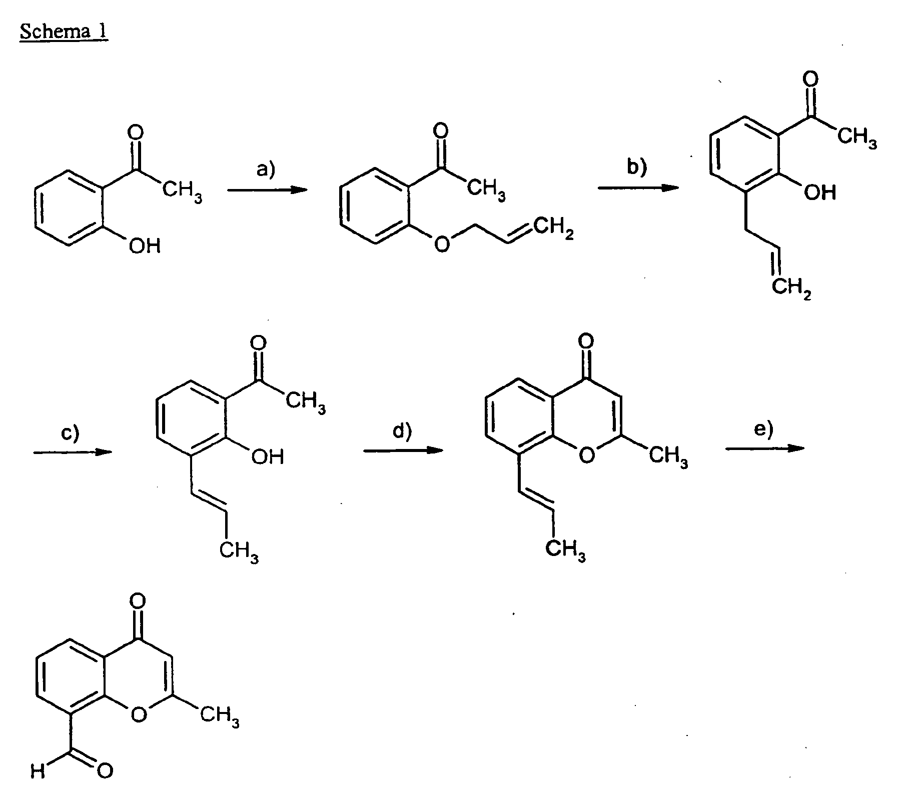 [a): Allylbromid, Kaliumcarbonat, cat. Kaliumiodid, Aceton, Rückfluss; b): 230°C, 4 h; c): Bis-(benzonitril)dichlorpalladium(II), Toluol, 120°C, 16 h; d): Acetylchlorid, Natriumhydrid, THF, 10-25°C, 16 h; e): 1. Ozon, Dichlormethan, -60°C, 30 min; 2. Dimethylsulfid]. 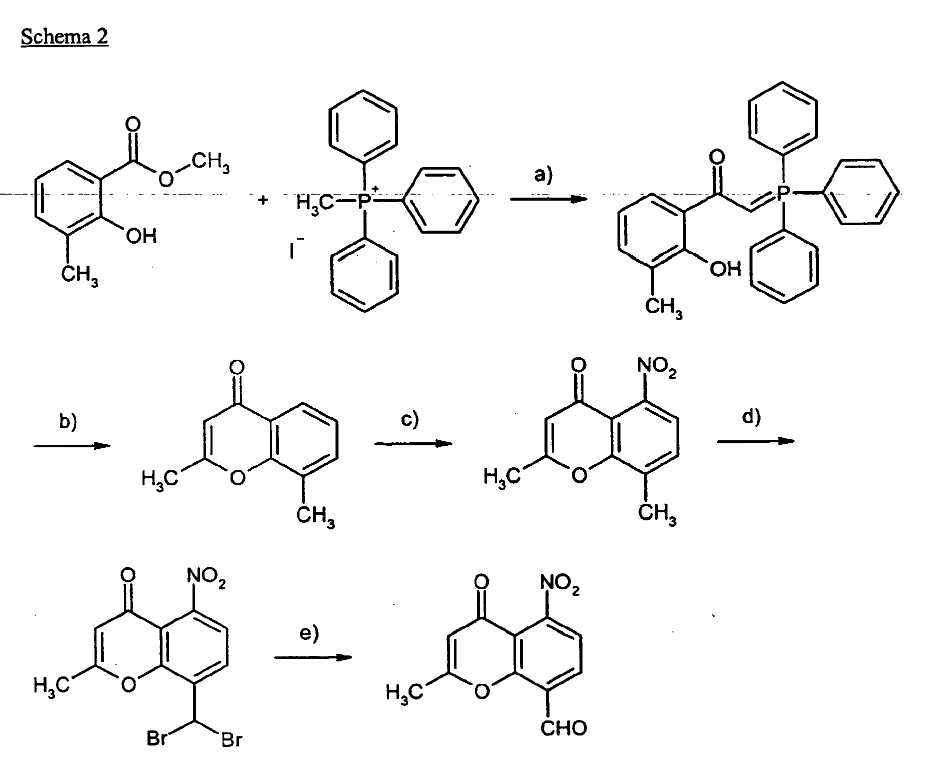 [a): n-Butyllithium, THF, 60°C, 3 h; b): Essigsäureanhydrid, Pyridin, Rückfluss, 6 h; c): konz. H₂SO₄, HNO₃, 0°C, 1 h; d): *N*-Bromsuccinimid, AIBN, Tetrachlorkohlenstoff, Rückfluss; e): *N-*Methylmorpholin-*N*-oxid, Acetonitril, Rückfluss]. 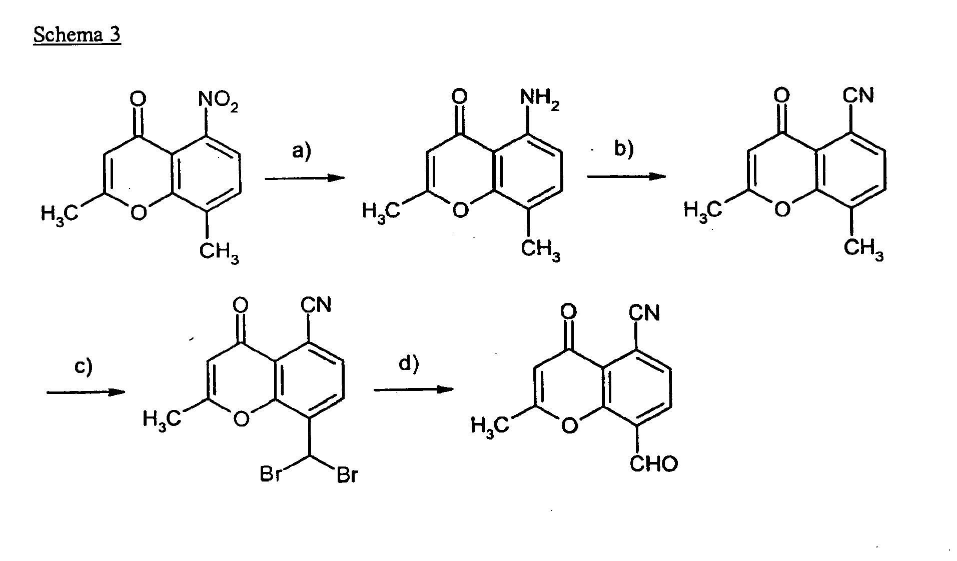 [a): Zinn(II)chlorid-Dihydrat, Ethylacetat, 70°C; b): 1. Natriumnitrit, Schwefelsäure, 0°C, 1.5 h; 2. Kupfer(I)cyanid, Natriumcyanid, Wasser/Ethylacetat, 0°C, 45 min; c): *N*-Bromsuccinimid, AIBN, Tetrachlorkohlenstoff, Rückfluss; d): *N*-Methylmorpholin-*N*-oxid, Acetonitril, Rückfluss]. 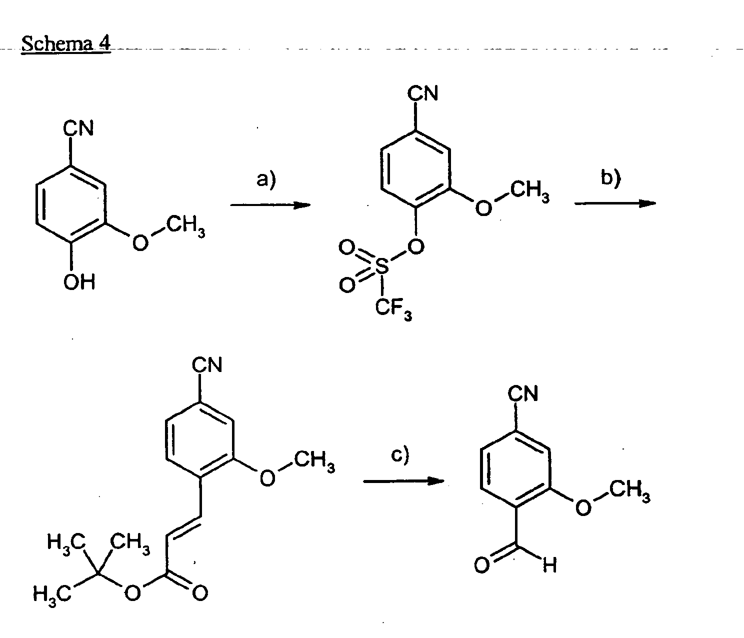 [a): Trifluormethansulfonsäureanhydrid, Pyridin, 0°C → RT, 30 min; b): Acrylsäure-*tert*.-butylester, Bis(triphenylphosphin)dichlorpalladium(II), DMF, 120°C, 24 h; c): cat. Osmiumtetroxid, cat. Benzyltriethylammoniumchlorid, Natriumperiodat, THF/Wasser, 20-25°C, 2 h]. 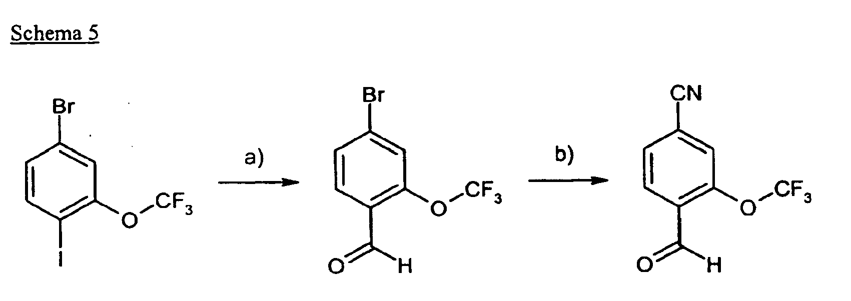 [a): n-Butyllithium, THF, -78°C, dann *N*-Formylmorpholin; b): Zinkcyanid, Tetrakis(triphenylphosphin)palladium(0), DMF, Mikrowelle 250°C / 5 min]. 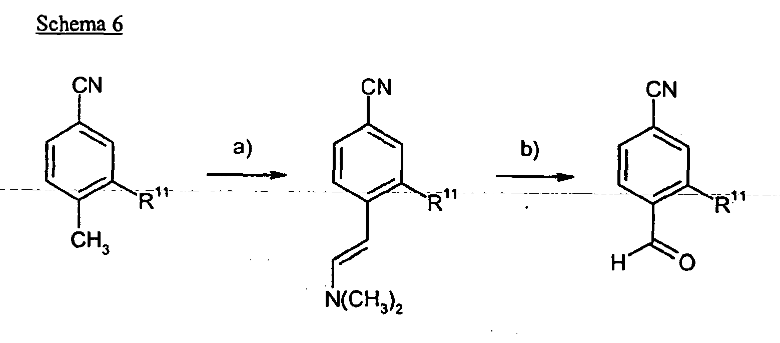 [a): *N*,*N*-Dimethylformamid-dimethylacetal, DMF, 140-180°C; b): Natriumperiodat, THF/Wasser]. 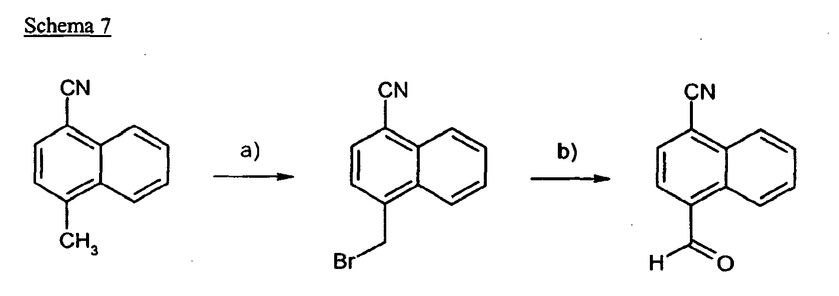 [a): *N*-Bromsuccinimid, 2,2'-Azobis-2-methylpropannitril, Tetrachlormethan, Rückfluss; b): *N-*Methylmorpholin-*N*-oxid, Acetonitril, 3Å-Molekularsieb]. 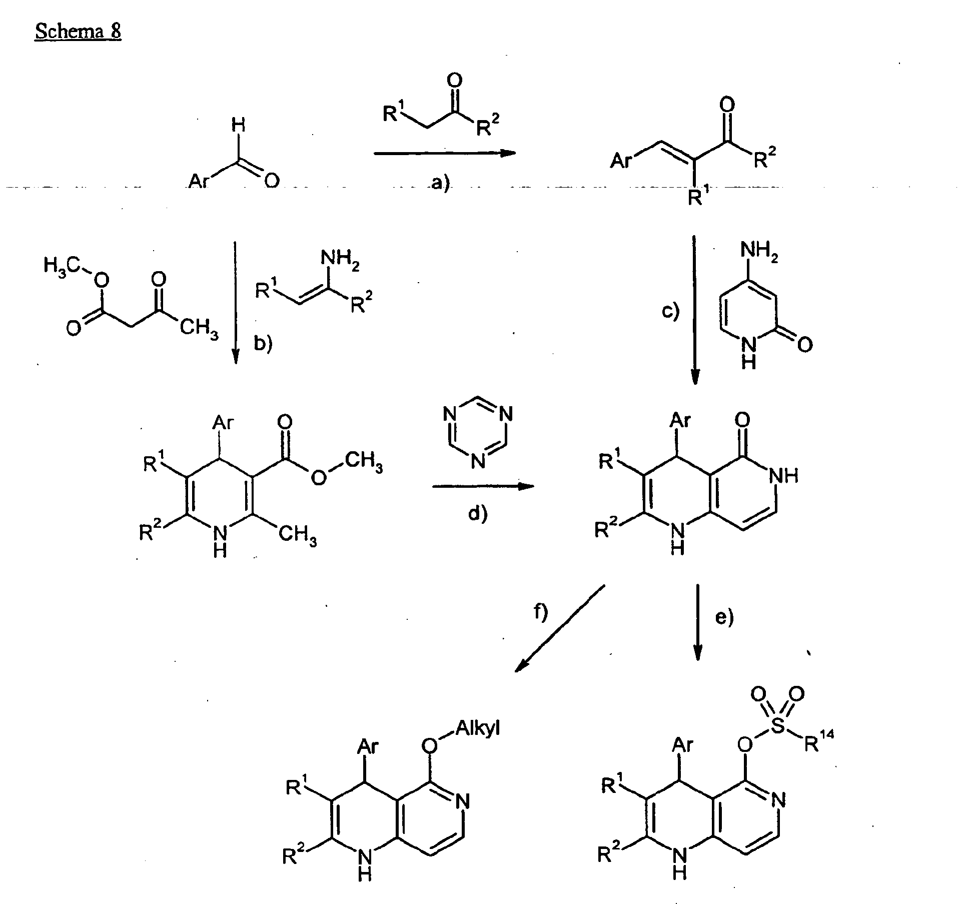 [a): cat. Piperidin, Essigsäure, Dichlormethan, Rückfluss, 24 h; b): Essigsäure, Isopropanol, Rückfluss, 12 h; c): Isopropanol, Rückfluss, 4 d; d): Natriumhydrid, DMF, 100°C, 12 h; e): R¹⁴-SO₂-Cl, Pyridin, RT, 1-3 h; f): Alkyltriflat, Phosphazen-P4-Base, THF, RT, 10 min; oder Alkyliodid, Natriumhydrid, DMF, RT, 10 h; oder Trialkyloxoniumtetrafluoroborat, Dichlormethan, RT, 12 h; oder Trialkylorthoformiat, cat. Schwefelsäure, 100-150°C, 12-36 h]. 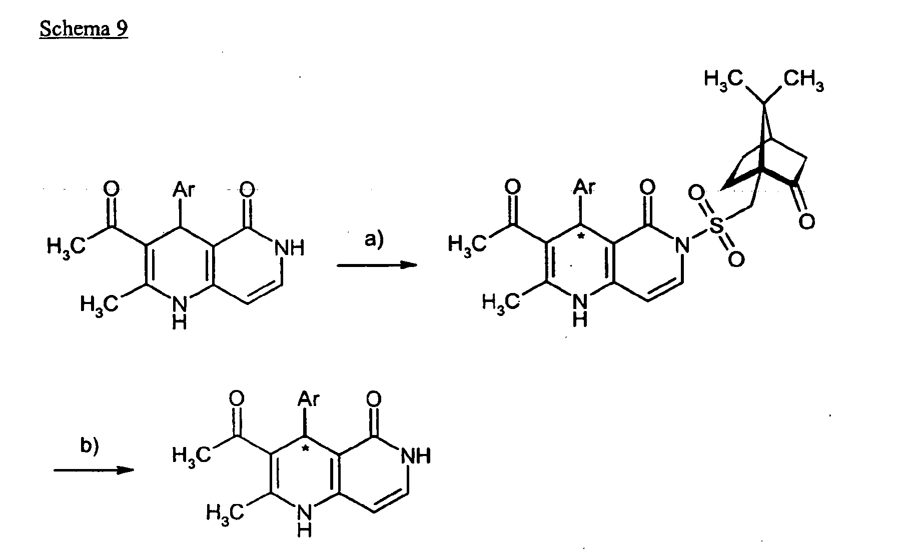 [a): 1. (1S)-(+)-Campher-10-sulfonsäurechlorid, Lithium-*tert*.-butylat, DMF, RT, 30 min; 2. Diastereomerentrennung; b): Natriumcarbonat, Methanol/Wasser, RT, 12 h]. 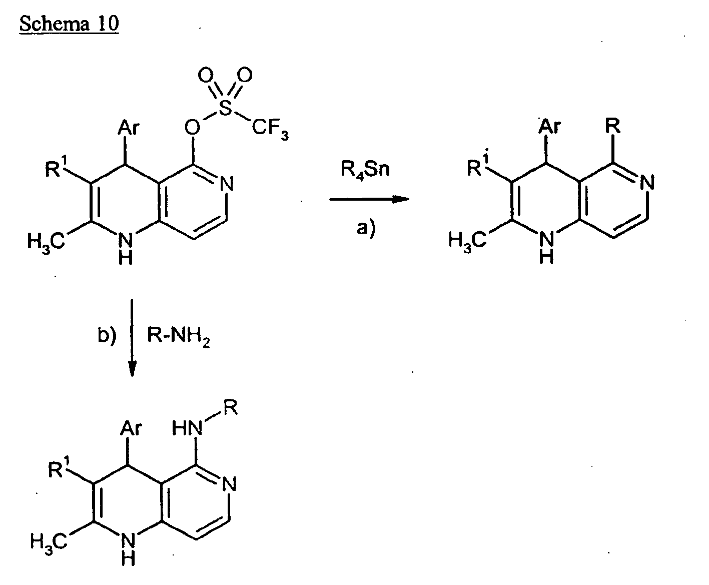 [R = Alkyl; a): Pd(PPh₃)₄, LiCl, 2,6-Di-*tert*.-butyl-4-methylphenol, Dioxan; vgl. z.B. J.A. Robl, Synthesis 1991, 56-58. b): K₂CO₃, DMF; vgl. z.B. Hinschberger et al., Pharm. Pharmacol. Commun. 2000, 6, 67-72]. 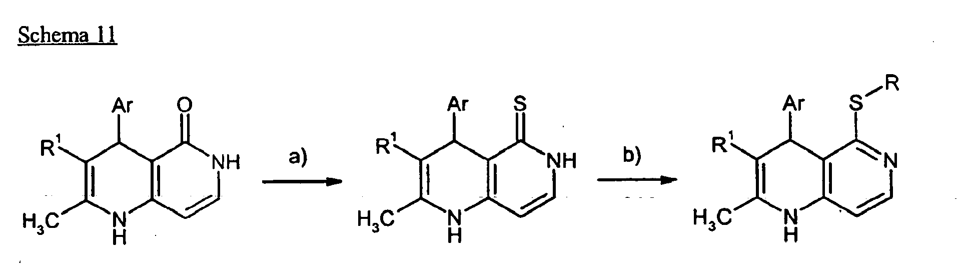 [R = Alkyl; a): P₂S₅, Pyridin; vgl. z.B. Kock et al., Synthesis 2005, 1052-1054. b): R-I,K₂CO₃, Aceton].

Die erfindungsgemäßen Verbindungen wirken als Antagonisten des Mineralokorticoid-Rezeptors und zeigen ein nicht vorhersehbares, wertvolles pharmakologisches Wirkspektrum. Sie eignen sich daher zur Verwendung als Arzneimittel zur Behandlung und/oder Prophylaxe von Krankheiten bei Menschen und Tieren.

Die erfindungsgemäßen Verbindungen sind geeignet für die Prophylaxe und/oder Behandlung von verschiedenen Erkrankungen und krankheitsbedingten Zuständen, insbesondere von Erkrankungen, die entweder durch eine Erhöhung der Aldosteron-Konzentration im Plasma oder durch eine Veränderung der Aldosteron-Plasmakonzentration relativ zur Renin-Plasmakonzentration gekennzeichnet sind oder mit diesen Veränderungen einhergehen. Beispielsweise seien genannt: idiopathischer primärer Hyperaldosteronismus, Hyperaldosteronismus bei Nebennierenhyperplasie, Nebennierenadenomen und/oder Nebennierencarzinomen, Hyperaldosteronismus bei Leberzirrhose, Hyperaldosteronismus bei Herzinsuffizienz sowie (relativer) Hyperaldosteronismus bei essentieller Hypertonie.

Die erfindungsgemäßen Verbindungen sind aufgrund ihres Wirkmechanismus ferner geeignet für die Prophylaxe des plötzlichen Herztodes bei Patienten, die unter einem erhöhten Risiko stehen, an einem plötzlichen Herztod zu versterben. Dies sind insbesondere Patienten, die z.B. an einer der folgenden Erkrankungen leiden: Hypertonie, Herzinsuffizienz, koronare Herzerkrankung, stabile und instabile Angina pectoris, myokardiale Ischämie, Myokardinfarkt, dilatative Kardiomyopathien, Schock, Arteriosklerose, atriale und ventrikuläre Arrhythmie, transitorische und ischämische Attacken, Hirnschlag, entzündliche kardiovaskuläre Erkrankungen., periphere und kardiale Gefäßerkrankungen, periphere Durchblutungsstörungen, pulmonale Hypertonie, Spasmen der Koronararterien und peripherer Arterien, Thrombosen, thromboembolische Erkrankungen sowie Vaskulitis.

Die erfindungsgemäßen Verbindungen können ferner verwendet werden für die Prophylaxe und/oder Behandlung von Ödembildung wie zum Beispiel pulmonales ödem, renales Ödem oder Herzinsuffizienz-bedingtes Ödem, und von Restenosen wie nach Thrombolysetherapien, percutantransluminalen Angioplastien (PTA) und transluminalen Koronarangioplastien (PTCA), Herztransplantationen und Bypass-Operationen.

Weiterhin eignen sich die erfindungsgemäßen Verbindungen zur Verwendung als Diuretikum und bei Elektrolytstörungen wie zum Beispiel Hyperkalzämie.

Außerdem können die erfindungsgemäßen Verbindungen eingesetzt werden für die Prophylaxe und/oder Behandlung von Diabetes mellitus und diabetischen Folgeerkrankungen wie z.B. Neuropathie und Nephropathie, von akutem und chronischem Nierenversagen sowie der chronischen Niereninsuffizienz.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker und Rho-Kinase-Inhibitoren;
- Diuretika, insbesondere Schleifendiuretika sowie Thiazide und Thiazid-ähnliche Diuretika;
- antithrombotisch wirkende Mittel, beispielhaft und vorzugsweise aus der Gruppe der Thrombozytenaggregationshemmer, der Antikoagulantien oder der profibrinolytischen Substanzen;
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber,_Gallensäure-Reabsorptionshemmer und Lipoprotein(a)-Antagonisten;
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- positiv-inotrop wirksame Verbindungen, wie beispielsweise Herzglycoside (Digoxin), betaadrenerge und dopaminerge Agonisten wie Isoproterenol, Adrenalin, Noradrenalin, Dopamin und Dobutamin;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil, sowie PDE 3-Inhibitoren wie Amrinone und Milrinone;
- natriuretische Peptide, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Calcium-Sensitizer, wie beispielhaft und vorzugsweise Levosimendan;
- Kalium-Supplements;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- NO- und Häm-unabhängige Aktivatoren der Guanylatcyclase, wie insbesondere die in WO 01/19355, WO 01/19776, WO 01/19778, WO 01/19780, WO 02/070462 und WO 02/070510 beschriebenen Verbindungen;
- Inhibitoren der humanen neutrophilen Elastase (HNE), wie beispielsweise Sivelestat oder DX-890 (Reltran);
- die Signaltransduktionskaskade inhibierende Verbindungen, wie beispielsweise Tyrosinkinase-Inhibitoren, insbesondere Sorafenib, Imatinib, Gefitinib und Erlotinib; und/oder
- den Energiestoffwechsel des Herzens beeinflussende Verbindungen, wie beispielhaft und vorzugsweise Etomoxir, Dichloracetat, Ranolazine oder Trimetazidine.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, älpha-Rezeptoren-Blocker, beta-Rezeptoren-Blocker, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095 oder BA-1049, verabreicht.

Unter antithrombotisch wirkenden Mitteln (Antithrombotika) werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705, BAY 60-5521, BAY 78-7499 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- cat.: katalytisch
- CI: chemische Ionisation (bei MS)
- d: Tag(e)
- DC: Dünnschichtchromatographie
- DMF: Dimethylformamid
- DMSO: Dimethylsulfoxid
- d. Th.: der Theorie (bei Ausbeute)
- EI: Elektronenstoß-Ionisation (bei MS)
- ent: Enantiomer / enantiomerenrein
- eq: Äquivalent(e)
- ESI: Elektrospray-Ionisation (bei MS)
- GC-MS: Gaschromatographie-gekoppelte Massenspektrometrie
- h: Stunde(n)
- HPLC: Hochdruck-, Hochleistungsflüssigchromatographie
- konz.: konzentriert
- LC-MS: Flüssigchromatographie-gekoppelte Massenspektrometrie
- min: Minute(n)
- MS: Massenspektrometrie
- NMR: Kernresonanzspektrometrie
- Ph: Phenyl
- R_{f}: Retentionsindex (bei DC)
- Rₜ: Retentionszeit (bei HPLC)
- RT: Raumtemperatur
- THF: Tetrahydrofuran
- v/v: Volumen-zu-Volumen-Verhältnis (einer Lösung)

### LC-MS-. GC-MS- und HPLC-Methoden:

### Methode 1 (LC-MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A →> 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2 (LGMS):

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 3 (LC-MS):

Instrument: Micromass Quattro LCZ mit HPLC Agilent Serie 1100; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 4 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 5 (LC-MS):

Instrument: Micromass Platform LCZ mit HPLC Agilent Serie 1100; Säule: Thermo Hypersil GOLD 3µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 6 (LC-MS):

Instrument: Micromass Platform LCZ mit HPLC Agilent Serie 1100; Säule: Thermo HyPURITY Aquastar 3µ 50 mm x 2.1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A→ 0.2 min 100% A → 2.9 min 30% A → 3.1 min 10% A → 5.5 min 10% A; Ofen: 50°C; Fluss: 0.8 ml/min; UV-Detektion: 210 nm.

### Methode 7 (GC-MS):

Instrument: Micromass GCT, GC 6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

### Methode 8 (BPLC):

Instrument: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Säulentemperatur: 30°C; UV-Detektion: 210 nm.

### Methode 9 (chirale HPLC):

Säule: 250 mm x 46 mm, basierend auf dem chiralen Selektor Poly(*N*-methacryloyl-D-leucin-*tert*.-butylamid); Eluent: Isohexan/Ethylacetat 1:1; Temperatur: 24°C; Fluss: 2 ml/min; UV-Detektion: 260 nm.

### Methode 10 (chirale HPLC):

Säule: Daicel Chiralpak AD-H, 5 µm, 250 mm x 4 mm; Eluent: Isohexan/Isopropanol 80:20; Temperatur: 35°C; Fluss: 2 ml/min; UV-Detektion: 250 nm.

### Methode 11 (GC-MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 12 (LC-MS):

Instrument: Micromass Quattro LCZ mit HPLC Agilent Serie 1100; Säule: Phenomenex Onyx Monolithic C18, 100 mm x 3 mm; Eluent A:11 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### Methode 13 (LC-MS):

Gerätetyp MS: Waters ZQ; Gerätetyp HPLC: Waters Alliance 2795; Säule: Phenomenex Onyx Monolithic C18, 100 mm x 3 mm; Eluent A: 11 Wasser + 0.5 ml 50%-ige Ameisensäure, Eluent B: 1 11 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

### Ausgangsverbindungen und Intermediate:

### Beispiel 1A

### 1-[2-(Allyloxy)phenyl]ethanon

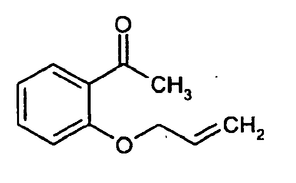

542 g (3.9 mol) 2-Hydroxyacetophenon werden mit 592 g (4.9 mol) Allylbromid, 1000 g (7.2 mol) Kaliumcarbonat und 13.2 g (79 mmol) Kaliumiodid in 2.4 Liter Aceton 24 h lang zum Rückfluss erhitzt. Nach Abkühlen auf Raumtemperatur wird filtriert und das Lösungsmittel im Vakuum entfernt. Der Rückstand wird in Toluol gelöst und mit 10%-iger Natronlauge und Wasser gewaschen. Nach Einengen werden 689 g (98% d. Th.) der Titelverbindung erhalten.
¹H-NMR (300 MHz, CDCl₃): δ = 2.68 (s, 3H), 4.68 (dd, 2H), 5.89 (dd, 2H), 6.09 (m, 1H), 6.99 (dd, 2H), 7.44 (m, 1H), 7.71 (d, 1H).

### Beispiel 2A

### 1-(3-Allyl-2-hydroxyphenyl)ethanon

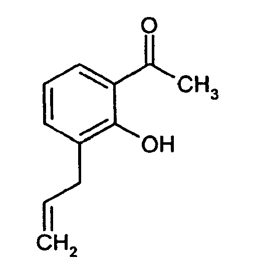

160 g (0.9 mol) 1-[2-(Allyloxy)phenyl]ethanon werden im Metallbad 4 h lang bei 230-240°C gerührt. Nach Abkühlen auf Raumtemperatur wird das Produkt über einen Dünnschichtverdampfer bei 140°C und 0.4 mbar destilliert. Es werden 155 g (97% d. Th.) der Titelverbindung erhalten.
¹H-NMR (300 MHz, CDCl₃): δ = 2.68 (s, 3H), 3.44 (d, 2H), 5.09 (m, 2H), 6.01 (m, 1H), 6.85 (t, 1H), 7.38 (dd, 1H), 7.62 (dd, 1H), 12.61 (s, 1H).

### Beispiel 3A

### 1-{2-Hydroxy-3-[(1E)-prop-1-en-1-yl]phenyl}ethanon

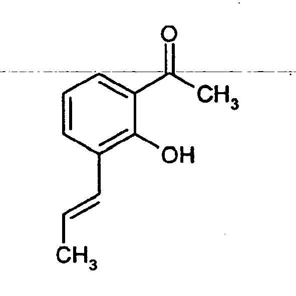

40 g (227 mmol) 1-(3-Allyl-2-hydroxyphenyl)ethanon werden in 120 ml Toluol gelöst und mit 2.17 g (5.6 mmol) Bis(benzonitril)dichlorpalladium(II) versetzt. Die Reaktionsmischung wird über Nacht auf 120°C erhitzt. Nach Abkühlen auf Raumtemperatur wird über Kieselgur filtriert und das Lösungsmittel im Vakuum entfernt. Es werden 20.9 g (95% d.Th.) der Titelverbindung erhalten, welche ohne weitere Reinigung in der nächsten Stufe umgesetzt wird.
LC-MS (Methode 1): Rₜ = 2.36 min; [M+H]⁺ = 177
¹H-NMR (300 MHz, CDCl₃): δ = 1.91 (dd, 3H), 2.63 (s, 3H), 6.32 (m, 1H), 6.73 (dd, 1H), 6.85 (t, 1H), 7.59 (m, 2H), 12.74 (s, 1H).

### Beispiel 4A

### 2-Methyl-8-[(1E)-prop-1-en-1-yl]-4H-chromen-4-on

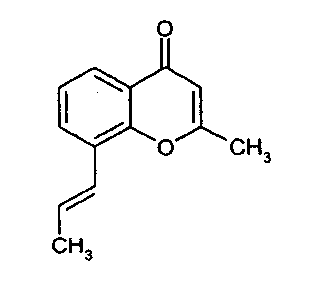

12.52 g (313.2 mmol) 60%-iges Natriumhydrid (Suspension in Mineralöl) werden unter Argon bei 10°C in 300 ml absolutem THF vorgelegt. Zu der Suspension werden 18.4 g (104.4 mmol) 1-{2-Hydroxy-3-[(1*E*)-prop-1-en-1-yl]phenyl}ethanon langsam zugetropft. Nach 15 min werden 9 g (114.9 mmol) Acetylchlorid zugegeben. Die Reaktionsmischung wird über Nacht bei Raumtemperatur gerührt. Man hydrolysiert mit 300 ml Wasser und extrahiert mehrfach mit Ethylacetat. Nach Waschen der organischen Phase mit gesättigter Natriumchlorid-Lösung wird über Natriumsulfat getrocknet. Anschließend wird das Lösungsmittel im Vakuum entfernt. Der Rückstand wird in 200 ml Methanol aufgenommen und mit 50 ml 20%-iger Salzsäure 30 min auf 80°C erhitzt. Anschließend wird das Lösungsmittel im Vakuum entfernt und der Rückstand mit 400 ml Wasser versetzt. Es wird mehrfach mit Dichlormethan extrahiert. Nach Trocknen der organischen Phase über Magnesiumsulfat wird das Lösungsmittel im Vakuum entfernt und der Rückstand mittels Säulenchromatographie gereinigt (Laufmittel: Dichlormethan/Methanol 98:2). Es werden 10.5 g (50.2% d. Th.) der Titelverbindung als gelbes Öl erhalten.
LC-MS (Methode 3): Rₜ = 2.07 min; [M+H]⁺ = 201
¹H-NMR (300 MHz, CDCl₃): δ = 1.98 (dd, 3H), 2.43 (s, 3H), 6.18 (s, 1H), 6.40 (m, 1H), 6.85 (dd, 1H), 7.31 (t, 1H), 7.72 (dd, 1H), 8.05 (dd, 1H).

### Beispiel 5A

### 2-Methyl-4-oxo-4H-chromen-8-carbaldehyd

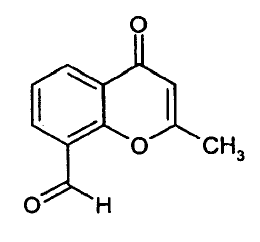

18.5 g (62.8 mmol) 2-Methyl-8-[(1*E*)-prop-1-en-1-yl]-4*H*-chromen-4-on werden in 400 ml Dichlormethan gelöst und auf -60°C abgekühlt. In die Reaktionslösung wird 30 min lang Ozon eingeleitet. Anschließend wird die Reaktionsmischung mit Dimethylsulfid versetzt. Nach Erwärmen auf Raumtemperatur wird das Lösungsmittel im Vakuum entfernt und der Rückstand mit wenig Methanol aufgeschlämmt. Nach Filtration wird der verbleibende Feststoff aus Diethylether umkristallisiert. Es werden 9.1 g (77.4% d. Th.) der Titelverbindung erhalten.
LC-MS (Methode 1): Rₜ = 1.31 min; [M+H]⁺ = 189
¹H-NMR (300 MHz, CDCl₃): δ = 2.48 (s, 3H), 6.27 (s, 1H), 7.51 (m, 1H), 8.21 (dd, 1H), 8.46 (dd, 1H), 10.67 (s, 1H).

### Beispiel 6A

### 3-[(2-Methyl-4-oxo-4H-chromen-8-yl)methylen]pentan-2,4-dion

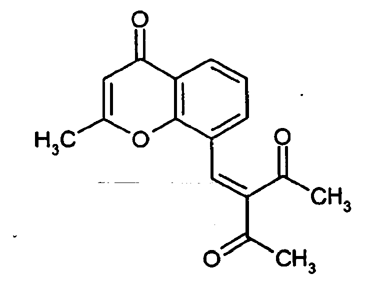

20 g (106 mmol) 2-Methyl-4-oxo-4*H*-chromen-8-carbaldehyd, 12 ml (116 mmol) 2,4-Pentandion, 9.1 ml (159 mmol) Essigsäure und 0.21 ml (2.1 mmol) Piperidin in 400 ml wasserfreiem Dichlormethan werden 24 h lang am Wasserabscheider unter Rückfluss gerührt. Nach dem Abkühlen wird die Reaktionslösung nacheinander mit gesättigter Natriumhydrogencarbonat-Lösung und gesättigter Natriumchlorid-Lösung gewaschen. Die organische Phase wird über Magnesiumsulfat getrocknet und eingeengt. Der Rückstand wird aus Isopropanol umkristallisiert. Man erhält 24.3 g (73% d. Th.) der Titelverbindung als weißen Feststoff.
LC-MS (Methode 4): Rₜ = 1.91 min; [M+H]⁺ = 271
¹H-NMR (300 MHz, DMSO-d₆): δ = 2.24 (s, 3H), 2.44 (s, 3H), 2.54 (s, 3H), 6.33 (s, 1H), 7.49 (t, 1H), 7.64 (dd, 1H), 7.97 (s, 1H), 8.07 (dd, 1H).

### Beispiel 7A

### 3-Acetyl-2-methyl-4-(2-methyl-4-oxo-4H-chromen-8-yl)-4,6-dihydro-1,6-naphthyridin-5(1H)-on

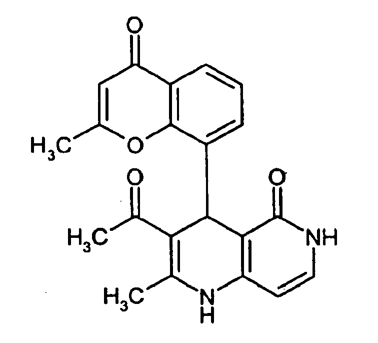

4.00 g (14.79 mmol) 3-[(2-Methyl-4-oxo-4*H*-chromen-8-yl)methylen]pentan-2,4-dion und 1.63 g (14.79 mmol) 4-Aminopyridin-2(1*H*)-on [Searls, T., McLaughlin, L.W., Tetrahedron 55, 11985-11996 (1999)] werden in 40 ml Isopropanol gelöst und 4 Tage lang unter Argon bei Rückflusstemperatur temperiert. Das Gemisch wird danach eingeengt und der Rückstand aus Methanol umkistallisiert. Man erhält 4.1 g (76% d. Th.) der Titelverbindung als gelben Feststoff.
LC-MS (Methode 4): Rₜ = 1.26 min; [M+H]⁺ = 363
¹H-NMR (300 MHz, DMSO-d₆): δ= 2.13 (s, 3H), 2.28 (s, 3H), 2.36 (s, 3H), 5.45 (s, 1H), 5.93 (d, 1H), 6:18 (s, 1H), 7.12 (m, 1H), 7.31 (t, 1H), 7.64 (dd, 1H), 7.78 (dd, 1H), 9.35 (s, 1H), 10.82 (s, 1H).

### Beispiel 8A

### 4-Brom-2-(trifluormethoxy)benzaldehyd

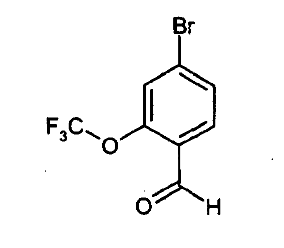

20.00 g (54.51 mmol) 4-Brom-2-(trifluormethoxy)iodbenzol werden in 200 ml THF gelöst und auf -78°C gekühlt. Anschließend werden 26.16 ml (65.41 mmol) einer 2.5 M Lösung von n-Butyllithium in Hexan zugetropft. Es wird 30 min nachgerührt und anschließend 14.43 g (125.37 mmol) N-Formylmorpholin zudosiert. Nachdem vollständiger Umsatz detektiert ist (DC-Kontrolle), wird bei -78°C mit Isopropanol solvolysiert. Nach dem Erwärmen auf Raumtemperatur wird mit Wasser versetzt und zweimal mit Dichlormethan extrahiert. Die vereinigten organischen Phasen werden mit gesättigter Natriumchlorid-Lösung gewaschen, mit Natriumsulfat getrocknet und das Lösungsmittel unter vermindertem Druck abdestilliert. Der Rückstand wird säulenchromatographisch gereinigt (Kieselgel, Laufmittel: Cyclohexan/Essigsäureethylester 5:1). Es werden 11.43 g (78% d. Th.) der Titelverbindung erhalten.
GC-MS (Methode 7): Rₜ = 4.24 min; MS (EIpos): m/z = 270 [M+H]⁺
¹H-NMR (300 MHz, DMSO-d₆): δ = 7.85-7.92 (m, 3H), 10.20 (s, 1H).

### Beispiel 9A

### 4-Formyl-3-(trifluormethoxy)benzonitril

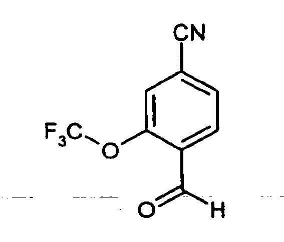

10.63 g (39.51 mmol) 4-Brom-2-(trifluormethoxy)benzaldehyd, 3.43 g (29.24 mmol) Zinkcyanid und 1.37 g (1.19 mmol) Tetrakis(triphenylphosphin)palladium(0) werden in 80 ml DMF gelöst. Anschließend wird die Reaktionsmischung in mehreren Portionen in einer Single Mode-Mikrowelle (Emrys Optimizer, 5 min bei 220°C) umgesetzt. Die vereinigten Ansätze werden mit Wasser versetzt und zweimal mit Toluol extrahiert. Die vereinigten organischen Phasen werden mit gesättigter Natriumchlorid-Lösung gewaschen, mit Natriumsulfat getrocknet und anschließend das Lösungsmittel am Rotationsverdampfer entfernt. Der Rückstand wird säulenchromatographisch aufgereinigt (Kieselgel, Laufmittel: Cyclohexan/Essigsäureethylester 10:1). Es werden 3.32 g (78% d. Th.) der Titelverbindung in 80%-iger Reinheit (nach LC-MS) erhalten.
MS (EIpos): m/z = 215 [M]⁺
¹H-NMR (300 MHz, DMSO-d₆): δ = 7.85-7.91 (m, 3H), 10.20 (s, 1H).

### Beispiel 10A

### 4-(3-Acetyl-2-methyl-5-oxo-1,4,5,6-tetrahydro-1,6-naphthyridin-4-yl)-3-(trifluormethoxy)-benzonitril

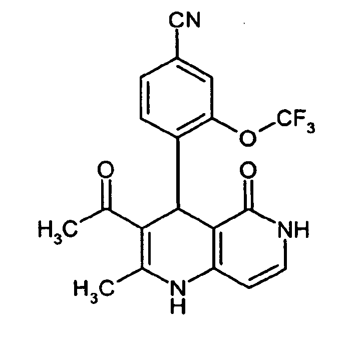

3.32 g (15.43 mmol) 4-Formyl-3-(trifluormethoxy)benzonitril, 1.55 g (15.43 mmol) 2,4-Pentandion, 1.39 g Essigsäure (23.15 mmol) und 262 mg (3.09 mmol) Piperidin werden in 150 ml Dichlormethan gelöst und über Nacht an einem inversen Wasserabscheider unter Rückfluss erhitzt. Nachdem per DC-Kontrolle vollständiger Umsatz detektiert ist, wird mit 100 ml Dichlormethan verdünnt und die Mischung mit Wasser und gesättigter Natriumchlorid-Lösung gewaschen. Das Lösungsmittel wird am Rotationsverdampfer entfernt. Es werden 4.56 g (99% d. Th.) rohes 4-(2-Acetyl-3-oxobut-1-en-1-yl)-3-(trifluormethoxy)benzonitril erhalten, welches ohne weitere Aufreinigung direkt umgesetzt wird.
3.32 g (10.73 mmol) der so erhaltenen Benzylidenverbindung werden in 100 ml Isopropanol gelöst und in der Siedehitze mit 1.31 g (10.73 mmol) 4-Aminopyridin-2(1*H*)-on [Searls, T., McLaughlin, L.W., Tetrahedron 55, 11985-11996 (1999)], gelöst in 60 ml warmem Isopropanol, versetzt (dabei sollte vermieden werden, das 4-Aminopyridin-2(1*H*)-on als Suspension zuzugeben). Nach dreitägigem Rühren bei Rückflusstemperatur wird das Lösungsmittel am Rotationsverdampfer entfernt, der Rückstand in Methanol aufgenommen und der anfallende Feststoff abfiltriert. Es werden 2.40 g (57% d. Th.) der Titelverbindung erhalten.
LC-MS (Methode 4): Rₜ = 1.72 min; MS (EIpos): m/z = 390 [M+H]⁺
¹H-NMR. (300 MHz, DMSO-d₆): δ = 2.12 (s, 3H), 2.27 (s, 3H), 5.29 (s, 1H), 5.89 (d, 1H), 7.14 (t, 1H), 7.58 (d, 1H), 7.65 (br. s, 1H), 7.69 (dd, 1H), 9.34 (s, 1H), 10.91 (s, 1H).

### Beispiel 11A

### Natrium 1-cyanoprop-1-en-2-olat

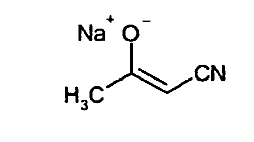

Natrium (7.69 g, 335 mmol) wird portionsweise in 350 ml wasserfreies Methanol eingetragen. Nach dem Abkühlen der Reaktionsmischung auf 25°C wird 5-Methylisoxazol (27.8 g, 335 mmol) langsam und portionsweise zugegeben (exotherme Reaktion). Nach beendeter Zugabe wird der Ansatz für 4 h bei RT gerührt und anschließend eingeengt. Der Rückstand wird mit wenig Diethylether gewaschen, abgesaugt und im Ölpumpenvakuum getrocknet. Man erhält 32.0 g (91% d. Th.) der Titelverbindung.
¹H-NMR (400 MHz, DMSO-d₆): δ = 3.18 (s, 1H), 1.51 (s, 3H).

### Beispiel 12A

### 2-Methyl-4-(2-methyl-4-oxo-4H-chromen-8-yl)-5-oxo-1,4,5,6-tetrahydro-1,6-naphthyridin-3-carbonitril

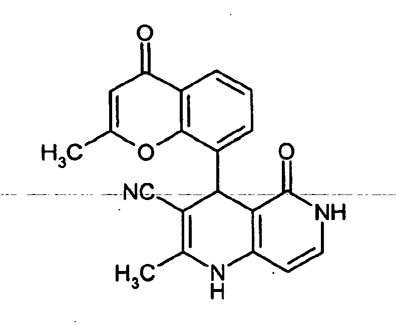

300 mg (1.59 mmol) 2-Methyl-4-oxo-4*H*-chromen-8-carbaldehyd werden mit 167.5 mg (1.59 mmol) Natrium 1-cyanoprop-1-en-2-olat, 175 mg (1.59 mmol) 4-Aminopyridin-2(1*H*)-on [Searls, T., McLaughlin, L.W., Tetrahedron 55, 11985-11996 (1999)] und 137 µl (2.39 mmol) Essigsäure in 12 ml 2-Propanol gelöst und unter Argon 12 h lang unter Rückfluss erhitzt. Nach dem Abkühlen wird die Reaktionsmischung filtriert und der verbleibende Feststoff mit Diethylether (20 ml) gewaschen. Es werden 454 mg (82% d. Th.) der Titelverbindung als weißer Feststoff erhalten.
MS (ESI): [M+H]⁺ = 346.2
¹H-NMR (300 MHz, DMSO-d₆): δ = 2.06 (s, 3H), 2.38 (s, 3H), 5.14 (s, 1H), 5.97 (d, 1H), 6.25 (s, 1H), 7.22 (d, 1H), 7.37 (t, 1H), 7.43 (dd, 1H), 7.87 (dd, 1H), 10.02 (s, 1H), 11.11 (s, 1H).

### Beispiel 13A

### 4-Cyano-2-methoxyphenyl-trifluormethansulfonat

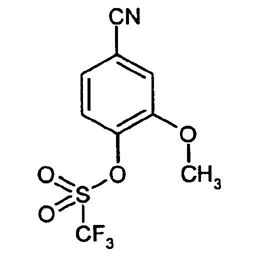

Zu einer Lösung von 20 g (134 mmol) 4-Hydroxy-3-methoxybenzonitril in Pyridin (80 ml) werden langsam 24 ml (141 mmol) Trifluormethansulfonsäureanhydrid getropft, wobei die Reaktionstemperatur mit Hilfe eines Eisbads unter 25°C gehalten wird. Die Suspension wird dann 1 h bei RT gerührt. Eiswasser (400 ml) wird zugegeben und die Suspension noch bis zum Erreichen der Raumtemperatur weiter gerührt. Dann wird filtriert, der Feststoff in Ethylacetat gelöst und diese Lösung mit gesättigter Natriumchlorid-Lösung gewaschen. Die organische Phase wird über Magnesiumsulfat getrocknet und eingeengt. Es werden 37.13 g (92% d. Th.) der Titelverbindung als weißer Feststoff erhalten.
LC-MS (Methode 4): Rₜ = 2.54 min; MS (EIpos): m/z = 282 [M+H]⁺
¹H-NMR (300 MHz, DMSO-d₆): δ = 3.97 (s, 3H), 7.60 (dd, 1H), 7.71 (d, 1H), 7.92 (d, 1H).

### Beispiel 14A

### tert.-Butyl (2E)-3-(4-cyano-2-methoxyphenyl)acrylat

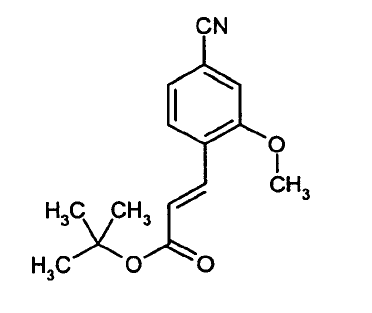

Zu einer entgasten Lösung von 37.13 g (132 mmol) 4-Cyano-2-methoxyphenyl-trifluormethansulfonat, 35 ml (245 mmol) *tert*.-Butylacrylat und 90 ml (645 mmol) Triethylamin in DMF (250 ml) werden 4 g (5.7 mmol) Bis(triphenylphosphin)palladium(II)chlorid hinzugefügt. Die Lösung wird unter Schutzgasatmosphäre 24 h lang bei 100°C gerührt. Anschließend wird Eiswasser (1000 ml) zugegeben und die Suspension mit Ethylacetat (3 x 100 ml) extrahiert. Die organische Phase wird mit gesättigter Natriumchlorid-Lösung gewaschen, über Magnesiumsulfat getrocknet und eingeengt. Der Rückstand wird säulenchromatographisch gereinigt (Kieselgel, Laufmittel: CyclohexanEssigsäureethylester 10:1). Es werden 24.6 g (72% d. Th.) der Titelverbindung als weißer Feststoff erhalten.
LC-MS (Methode 1): Rₜ = 2.59 min; MS (EIpos): m/z = 260 [M+H]⁺
¹H-NMR (300 MHz, DMSO-d₆): δ = 1.48 (s, 9H), 3.93 (s, 3H), 6.65 (d, 1H), 7.42 (d, 1H), 7.58 (s, 1H), 7.74 (d, 1H), 7.89 (d, 1H).

### Beispiel 15A

### 4-Formyl-3-methoxybenzonitril

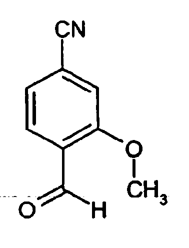

Zu einer kräftig gerührten Lösung von 48 g (185 mmol) *tert*.-Butyl (2*E*)-3-(4-cyano-2-methoxyphenyl)acrylat, 207 mg (0.81 mmol) Osmiumtetroxid und 1.4 g (6.14 mmol) Benzyltriethylammoniumchlorid in 750 ml Wasser/THF (2:1) werden 79 g (370 mmol) Natriummetaperiodat portionsweise zugegeben, wobei die Reaktionstemperatur unter 30°C gehalten wird. Die Lösung wird 1 h bei RT weitergerührt. Wasser (2000 ml) wird zugegeben und die Mischung anschließend filtriert. Der verbleibende Feststoff wird in Ethylacetat gelöst und die Lösung mit gesättigter Natriumchlorid-Lösung gewaschen. Die organische Phase wird über Magnesiumsulfat getrocknet und eingeengt. Der Rückstand wird mit Petrolether verrührt. Es werden 21.18 g (71% d. Th.) der Titelverbindung als weißer Feststoff erhalten. LC-MS (Methode 4): Rₜ = 1.87 min; MS (EIpos): m/z = 162 [M+H]⁺
¹H-NMR (300 MHz, DMSO-d₆): δ = 3.98 (s, 3H), 7.53 (d, 1H), 7.80 (s, 1H), 7.81 (d, 1H), 10.37 (s, 1H).

### Beispiel 16A

### 4-(2-Acetyl-3-oxobut-1-en-1-yl)-3-methoxybenzonitril

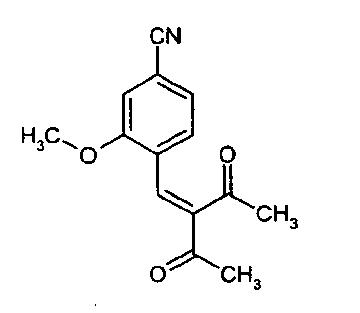

21 g (130 mmol) 4-Formyl-3-methoxybenzonitril, 14.7 ml (143 mmol) 2,4-Pentandion, 11.2 ml (195 mmol) Essigsäure und 2.6 ml (26 mmol) Piperidin in 400 ml trockenem Dichlormethan werden 24 h lang am Wasserabscheider unter Rückfluss gerührt. Nach dem Abkühlen wird die Reaktionslösung nacheinander mit gesättigter Natriumhydrogencarbonat-Lösung und gesättigter Natriumchlorid-Lösung gewaschen. Die organische Phase wird über Magnesiumsulfat getrocknet und eingeengt. Der Rückstand wird aus Diethylether umkristallisiert. Man erhält 23.2 g (92% d. Th.) der Titelverbindung als leicht braunen Feststoff.
LC-MS (Methode 4): Rₜ = 2.05 min; [M+H]⁺ = 244
¹H-NMR (300 MHz, DMSO-d₆): δ = 2.20 (s, 3H), 2.42 (s, 3H), 3.89 (s, 3H), 7.37 (d, 1H), 7.46 (dd, 1H), 7.60 (d, 1H), 7.68 (s, 1H).

### Beispiel 17A

### 4-(3-Acetyl-2-methyl-5-oxo-1,4,5,6-tetrahydro-1,6-naphthyridin-4-yl)-3-methoxybenzonitril

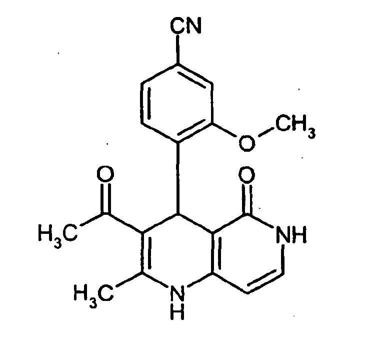

18 g (74 mmol) 4-(2-Acetyl-3-oxobut-1-en-1-yl)-3-methoxybenzonitril und 8.14 g (74 mmol) 4-Aminopyridin-2(1*H*)-on [Searls, T., McLaughlin, L.W., Tetrahedron 55, 11985-11996 (1999)] werden in 40 ml Isopropanol gelöst und unter Argon 4 Tage lang unter Rückfluss erhitzt. Das Gemisch wird danach eingeengt und der Rückstand aus Methanol umkristallisiert. Man erhält 18.1 g (70% d. Th.) der Titelverbindung als leicht gelben Feststoff.
LC-MS (Methode 4): Rₜ = 1.46 min; MS (EIpos): m/z = 336 [M+H]⁺
¹H-NMR (300 MHz, DMSO-d₆): δ = 2.14 (s, 3H), 2.15 (s, 3H), 3.79 (s, 3H), 5.32 (s, 1H), 5.92 (d, 1H), 7.10 (d, 1H), 7.14 (d, 1H), 7.27 (d, 1H), 7.37 (s, 1H), 9.18 (s, 1H), 10.90 (br. s, 1H).

### Beispiel 18A

### 4-(2-Acetyl-3-oxobut-1-en-1-yl)-benzonitril

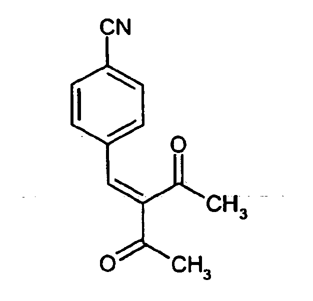

2.3 g (17.5 mmol) 4-Formylbenzonitril, 1.98 ml (19.29 mmol) 2,4-Pentandion, 1 ml (26 mmol) Essigsäure und 0.34 ml (3.5 mmol) Piperidin in 40 ml wasserfreiem Dichlormethan werden 24 h lang am Wasserabscheider unter Rückfluss gerührt. Nach dem Abkühlen wird die Reaktionslösung nacheinander mit gesättigter Natriumhydrogencarbonat-Lösung und gesättigter Natriumchlorid-Lösung gewaschen. Die organische Phase wird über Magnesiumsulfat getrocknet und eingeengt. Der Rückstand wird aus Diethylether umkristallisiert. Man erhält 3.18 g (85% d. Th.) der Titelverbindung als leicht braunen Feststoff.
¹H-NMR (300 MHz, DMSO-d₆): δ = 2.26 (s, 3H), 2.46 (s, 3H), 7.60 (d, 2H), 7.76 (s, 1H), 7.93 (d, 2H).

### Beispiel 19A

### 4-(3-Acetyl-2-methyl-5-oxo-1,4,5,6-tetrahydro-1,6-naphthyridin-4-yl)-benzonitril

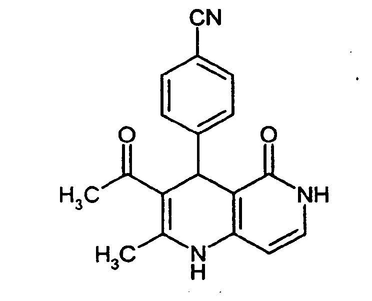

1.5 g (7.04 mmol) 4-(2-Acetyl-3-oxobut-1-en-1-yl)-benzonitril werden mit 0.77 g (7.04 mmol) 4-Aminopyridin-2(1*H*)-on [Searls, T., McLaughlin, L.W., Tetrahedron 55, 11985-11996 (1999)] versetzt, in 40 ml Isopropanol gelöst und unter Argon 4 Tage lang unter Rückfluss erhitzt. Das Gemisch wird danach eingeengt und der Rückstand aus Methanol umkristallisiert. Man erhält 0.67 g (31% d. Th.) der Titelverbindung als leicht gelben Feststoff.
LC-MS (Methode 1): Rₜ = 1.22 min; MS (EIpos): m/z = 306 [M+H]^{+ 1}H-NMR (300 MHz, DMSO-d₆): δ = 2.10 (s, 3H), 2.38 (s, 3H), 5.13 (s, 1H), 5.91 (d, 1H), 7.13 (t, 1H), 7.44 (d, 2H), 7.68 (d, 2H), 9.32 (s, 1H), 11.05 (br. s, 1H).

### Beispiel 20A

### 9-Oxo-9H-fluoren-4-carbaldehyd

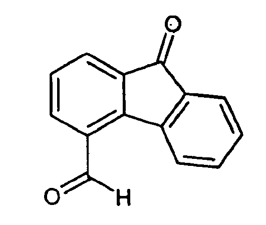

Unter Argon wird 9-Oxo-9*H*-fluoren-4-carbonsäuremethylester (9.85 g, 41.3 mmol) in 180 ml wasserfreiem THF vorgelegt. Bei RT wird innerhalb von 90 min RED-AL^{®} (38 ml, 136 mmol) [Natrium-bis-(2-methoxyethoxy)aluminiumdihydrid, 70%-ige Lösung in Toluol] zugetropft und das Reaktionsgemisch 1 h nachgerührt. Der Ansatz wird durch vorsichtige, tropfenweise Zugabe von 15 ml Wasser hydrolysiert. Anschließend gibt man 60 ml 6 N Salzsäure zu und extrahiert mit Essigsäureethylester (4 x je 150 ml). Die vereinigten organischen Phasen werden mit gesättigter Natriumchlorid-Lösung gewaschen (2 x je 100 ml), über Natriumsulfat getrocknet und am Rotationsverdampfer eingeengt. Man erhält 12.1 g des korrespondierenden Alkohols. Von diesem werden 8.77 g (41.3 mmol) in 200 ml Dioxan gelöst und mit aktiviertem Mangandioxid (25.1 g, 289 mmol) versetzt. Der Ansatz wird 1 h bei RT und danach 30 min bei 50°C gerührt. Man saugt vom Oxidationsmittel ab, wäscht den Filterrückstand mit Dioxan (3 x je 50 ml) und engt das Filtrat am Rotationsverdampfer ein. Das erhaltene Rohmaterial wird an Kieselgel (Laufmittelgradient: Cyclohexan → Cyclohexan/Essigsäureethylester 3:1) chromatographisch gereinigt. Man erhält 6.50 g (76% d. Th.) der Titelverbindung.
LC-MS (Methode 5): Rₜ = 2.14 min; MS (ESIpos): m/z = 209 [M+H]⁺
¹H-NMR (400 MHz, DMSO-d₆): δ = 7.50 (dd, 1H), 7.62 (dd, 1H), 7.69 (m, 2H), 7.90 (d, 1H), 8.12 (d, 1H), 8.39 (d, 1H), 10.5 (s, 1H).

### Beispiele 21A

### 3-Oxo-2-[(9-oxo-9H-fluoren-4-yl)methylen]butannitril

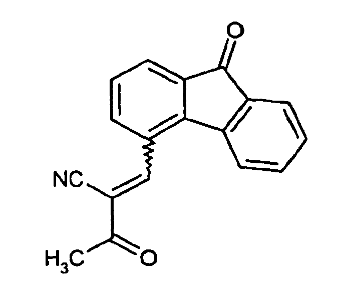

Die Verbindung aus Beispiel 11A (5.21 g, 25.0 mmol) wird in 180 ml Dichlormethan vorgelegt, und die Verbindung aus Beispiel 20A (2.89 g, 27.5 mmol), Essigsäure (1.72 ml, 30.0 mmol) und Piperidin (0.25 ml, 2.50 mmol) werden zugegeben. Der Ansatz wird 4 h lang am Wasserabscheider in der Siedehitze gerührt. Nach dem Abkühlen auf RT wird mit 30 ml Dichlormethan verdünnt, mit Wasser (2 x 50 ml) gewaschen, die organische Phase über Natriumsulfat getrocknet und das Lösungsmittel am Rotationsverdampfer entfernt. Das erhaltene Rohprodukt wird über Kieselgel-60 mit Dichlormethan als Laufmittel chromatographisch gereinigt. Man erhält nach Vereinigen der Produktfraktionen und Entfernen des Lösungsmittels 5.40 g (79% d. Th.) der Titelverbindung als *E*/*Z*-Isomerengemisch.
LC-MS (Methode 5): Rₜ = 2.24 min; MS (ESIpos): m/z = 274 [M+H]⁺
¹H-NMR (400 MHz, DMSO-d₆): δ = 2.64 (s, 3H), 7.49 (t, 1H), 7.59 (t, 1H), 7.64 (d, 1H), 7.69 (d, 1H), 7.73 (d, 1H), 7.81 (d, 1H), 7.90 (d, 1H), 8.88 (s, 1H).

### Beispiel 22A

### 3-Acetyl-6-({[(1R,4S)-7,7-dimethyl-2-oxobicyclo[2.2.1]hept-1-yl]methyl}sulfonyl)-2-methyl-4-(2-methyl-4-oxo-4H-chromen-8-yl)4,6-dihydro-1,6-naphthyridin-5(1H)-on (Diastereomer A und Diastereomer B)

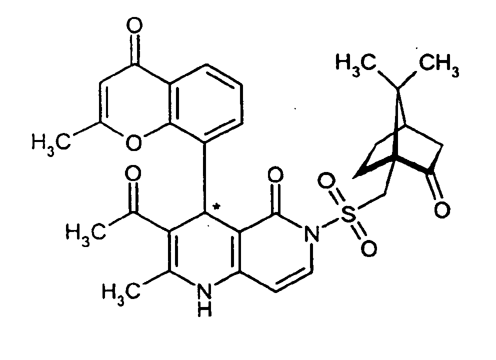

700 mg (1.9 mmol) 3-Acetyl-2-methyl-4-(2-methyl-4-oxo-4*H*-chromen-8-yl)-4,6-dihydro-1,6-naphthyridin-5(1*H*)-on werden in DMF (4 ml) suspendiert und mit 247 mg (3 mmol) Lithium-*tert*.-butylat versetzt. Zu der dunkelroten Lösung werden dann 824 mg (3.1 mmol) (1*S*)-(+)-Campher-10-sulfonsäurechlorid in 2 ml DMF getropft. Die entfärbte Lösung wird für 10 min weitergerührt, dann mit Methanol solvolysiert und mit 40 ml 0.1 M Salzsäure versetzt. Es wird filtriert und der zurückbleibende Niederschlag mit Wasser gewaschen. Man reinigt den Feststoff weiter durch präparative HPLC und trennt dabei in die diastereomerenreinen Titelverbindungen auf.

### Diastereomer A:

### Ausbeute: 254 mg (23% d.Th.)

LC-MS (Methode 4): Rₜ = 2.01 min; [M+H]⁺ = 577
¹H-NMR (400 MHz, DMSO-d₆): δ = 0.30 (t, 3H), 0.87 (t, 3H), 1.24-1.45 (m, 2H), 1.50 (dd, 1H), 1.58 (m, 1H), 1.73 (d, 1H), 1.85 (m, 1H), 2.18 (s, 3H), 2.24 (m, 1H), 2.29 (s, 3H), 2.35 (s, 3H), 3.91 (AB-System, 2H), 5.50 (s, 1H), 6.20 (s, 1H), 6.24 (d, 1H), 7.33 (t, 1H), 7.70 (m, 2H), 7.80 (dd, 1H), 9.76 (s, 1H).

### Diastereomer B:

### Ausbeute: 245 mg (22% d. Th.)

### LC-MS (Methode 4): Rₜ = 2.05 min; [M+H]⁺ = 577

¹H-NMR (400 MHz, DMSO-d₆): δ = 0.55 (t, 3H), 0.87 (t, 3H), 1.24-1.45 (m, 2H), 1.73 (d, 1H), 1.94 (m, 3H), 2.09 (m, 1H), 2.18 (s, 3H), 2.29 (s, 3H), 2.35 (s, 3H), 3.65 (d, 1H), 4.15 (d, 1H), 5.48 (s, 1H), 6.16 (s, 1H), 6.2 (d, 1H), 7.29 (t, 1H), 7.68 (d, 2H), 7.77 (dd, 1H), 9.74 (s, 1H).

### Beispiel 23A

ent-3-Acetyl-2-methyl-4-(2-methyl-4-oxo-4*H*-chromen-8-yl)-4,6-dihydro-1,6-naphthyridin-5(1*H*)-on 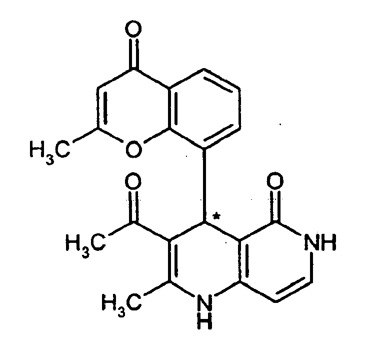 240 mg (0.41 mmol) des Diastereomers B von 3-Acetyl-6-({[(1*R*,4*S*)-7,7-dimethyl-2-oxobicyclo-[2.2.1]hept-1-yl]methyl}sulfonyl)-2-methyl-4-(2-methyl-4-oxo-4*H*-chromen-8-yl)-4,6-dihydro-1,6-naphthyridin-5(1*H*)-on aus Beispiel 22A werden in Methanol (5 ml) gelöst und mit 4 ml wässriger Natriumcarbonat-Lösung (0.2 M, 0.8 mmol) versetzt. Die Reaktionsmischung wird 12 h bei RT gerührt. Man stellt dann mit 1 N Salzsäure neutral und reinigt die Lösung direkt mittels präparativer HPLC. Man erhält 105 mg (70% d. Th.) der enantiomerenreinen Titelverbindung als weißen Feststoff.
LC-MS (Methode 1): Rₜ = 1.07 min; [M+H]⁺ = 363 ¹H-NMR (300 MHz, DMSO-d₆): δ = 2.13 (s, 3H), 2.28 (s, 3H), 2.36 (s, 3H), 5.45 (s, 1H), 5.93 (d, 1H), 6.18 (s, 1H), 7.12 (m, 1H), 7.31 (t, 1H), 7.64 (dd, 1H), 7.78 (dd, 1H), 9.35 (s, 1H), 10.82 (s, 1H).

### Beispiel 24A

### 4-Formyl-3-hydroxybenzonitril

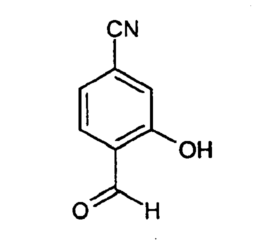

Zu einer Lösung von 8 g (49.64 mmol) 4-Formyl-3-methoxybenzonitril in 80 ml wasserfreiem Dichlormethan werden unter Argonatmosphäre bei -78°C 100 ml einer Bortribromid-Lösung in Dichlormethan (1 M, 100 mmol) getropft. Das Reaktionsgemisch wird bis zur vollen Umsetzung des Eduktes bei RT gerührt (ca. 5 Tage). Die Reaktionslösung wird dann bei 0°C mit gesättigter Natriumhydrogencarbonat-Lösung neutral gestellt. Die Phasen werden getrennt und die organische Phase wird mit gesättigter Natriumchlorid-Lösung gewaschen, über Magnesiumsulfat getrocknet und eingeengt. Der Rückstand wird mittels Säulenchromatographie an Kieselgel gereinigt (Laufmittel: Cyclohexan/Ethylacetat 3:1). Es werden 4.5 g (61% d. Th.) der Titelverbindung als gelber Feststoff erhalten.
LC-MS (Methode 1): Rₜ = 1.38 min; [M-H]⁻ = 146
¹H-NMR (300 MHz, CDCl₃): δ = 7.38 (d, 1H), 7.38 (s, 1H), 7.77 (d, 1H), 10.33 (s, 1H), 11.38 (s, 1H).

### Beispiel 25A

### 5-Cyano-2-formylphenyl-trifluormethansulfonat

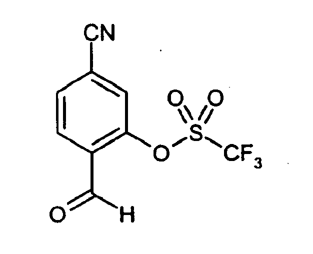

Zu einer Lösung von 2 g (13.59 mmol) 4-Formyl-3-hydroxybenzonitril und 2.5 ml (14.27 mmol) *N,N*-Diisopropylethylamin in 37 ml wasserfreiem Dichlormethan werden unter Argonatmosphäre bei 0°C 2.4 ml (14.27 mmol) Trifluormethansulfonsäureanhydrid getropft. Die Reaktionsmischung wird 1 h bei RT nachgerührt, dann mit 70 ml Dichlormethan verdünnt und nacheinander mit 1 M Salzsäure, gesättigter Natriumhydrogencarbonat-Lösung und gesättigter Natriumchlorid-Lösung gewaschen. Die organische Lösung wird über Magnesiumsulfat getrocknet und eingeengt. Der Rückstand wird mittels Säulenchromatographie an Kieselgel gereinigt (Laufmittel: Cyclohexan/Ethylacetat 7:1). Es werden 2.36 g (62% d. Th.) der Titelverbindung als weißer Feststoff erhalten.
LC-MS (Methode 4): Rₜ = 2.34 min; [M+H]⁺ = 280
¹H-NMR (300 MHz, CDCl₃): δ = 8.27 (m, 2H), 8.33 (s, 1H), 10.13 (s, 1H).

### Beispiel 26A

### 4-Formyl-3-vinylbenzonitril

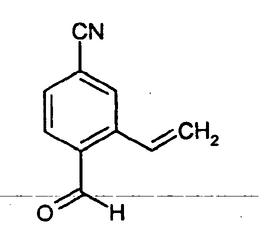

Zu einer Lösung von 1 g (3.58 mmol) 5-Cyano-2-formylphenyl-trifluormethansulfonat und 1.15 ml (3.94 mmol) Tri-n-butylvinylstannan in 6 ml wasserfreiem und entgastem DMF werden unter Argonatmosphäre 125 mg (0.18 mmol) Bis(triphenylphosphin)palladium(II)chlorid gegeben. Die Reaktionsmischung wird anschließend 90 min bei 80°C gerührt. Danach werden 100 ml 10%-ige Kaliumfluorid-Lösung zugesetzt und das Gemisch 1 h bei RT nachgerührt. Die Suspension wird dreimal mit jeweils 20 ml Ethylacetat extrahiert und die vereinigten organischen Phasen nacheinander mit gesättigter Natriumhydrogencarbonat-Lösung und gesättigter Natriumchlorid-Lösung gewaschen. Die organische Lösung wird über Magnesiumsulfat getrocknet und eingeengt. Der Rückstand (0.6 g) wird ohne weitere Reinigung in der nächsten Stufe eingesetzt.
GC-MS (Methode 7): Rₜ = 5.02 min; [M]⁺ = 157
¹H-NMR (300 MHz, CDCl₃): δ = 5.62 (d, 1H), 6.05 (d, 1H), 7.58 (dd, 1H), 7.95 (d, 1H), 8.00 (d, 1H), 8.24 (s, 1H), 10.32 (s, 1H).

### Beispiel 27A

### 3-Ethyl-4-formylbenzonitril

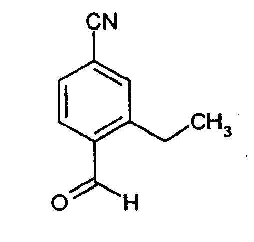

Eine Lösung von 1.3 g (8.27 mmol) 4-Formyl-3-vinylbenzonitril in 35 ml Ethanol wird mit 880 mg 10%-igem Palladium auf Kohle versetzt und 2 h lang unter einer Wasserstoff-Atmosphäre kräftig gerührt. Die Suspension wird durch eine Kieselgurschicht filtriert, der Rückstand mit Ethanol gewaschen und das Filtrat eingeengt. Der Rückstand (890 mg) wird ohne weitere Reinigung in der Folgestufe eingesetzt.
¹H-NMR (300 MHz, CDCl₃): δ = 1.2 (t, 1H), 3.07 (q, 2H), 7.88 (d, 1H), 7.90 (s, 1H), 7.97 (d, 1H), 10.32 (s, 1H).

### Beispiel 28A

### 4-(2-Acetyl-3-oxobut-1-en1-yl)-3-ethylbenzonitril

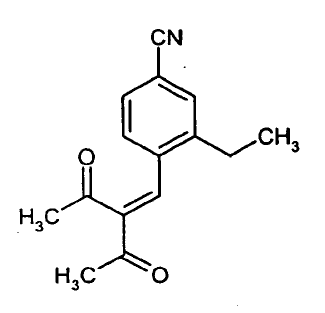

885 mg (5.55 mmol) 3-Ethyl-4-formylbenzonitril, 0.62 ml (6.11 mmol) 2,4-Pentandion, 0.47 ml (8.33 mmol) Essigsäure und 0.11 ml (1.11 mmol) Piperidin in 40 ml wasserfreiem Dichlormethan werden 24 h lang am Wasserabscheider unter Rückfluss gerührt. Nach dem Abkühlen wird die Reaktionslösung nacheinander mit gesättigter Natriumhydrogencarbonat-Lösung und gesättigter Natriumchlorid-Lösung gewaschen. Die organische Phase wird über Magnesiumsulfat getrocknet und eingeengt. Der Rückstand (1.07 g) wird ohne weitere Reinigung weiter umgesetzt.
¹H-NMR (300 MHz, CDCl₃): δ = 1.17 (t, 1H), 2.10 (s, 3H), 2.46 (s, 3H), 2.76 (q, 2H), 7.24 (d, 1H), 7.68 (d, 1H), 7.79 (s, 1H), 7.90 (s, 1H).

### Beispiel 29A

### 4-(3-Acetyl-2-methyl-5-oxo-1,4,5,6-tetrahydro-1,6-naphthyridin-4-yl)-3-ethylbenzonitril

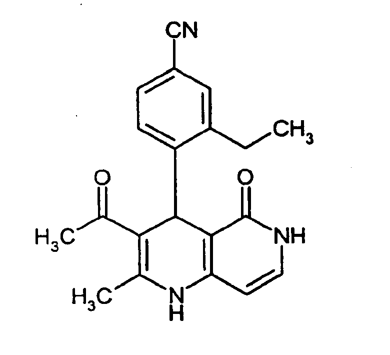

1 g (4.14 mmol) 4-(2-Acetyl-3-oxobut-1-en-1-yl)-3-ethylbenzonitril und 0.45 g (4.14 mmol) 4-Aminopyridin-2(1*H*)-on [Searls, T., McLaughlin, L.W., Tetrahedron 55, 11985-11996 (1999)] werden in 20 ml Isopropanol gelöst und 4 Tage lang unter Argon bei Rückflusstemperatur gerührt. Das Gemisch wird dann eingeengt und der Rückstand mittels Säulenchromatographie an Kieselgel gereinigt (Laufmittel: Dichlormethan/Methanol 10:1). Man erhält 277 mg (20% d. Th.) der Titelverbindung als leicht gelben Feststoff.
LC-MS (Methode 1): Rₜ = 1.44 min; [M+H]⁺ = 334
¹H-NMR (300 MHz, CDCl₃): δ = 1.26 (t, 3H), 2.14 (s, 3H), 2.34 (s, 3H), 3.19 (m, 1H), 3.40 (m, 1H), 5.24 (s, 1H), 5.92 (d, 1H), 7.11 (t, 1H), 7.29 (d, 1H), 7.46 (m, 2H), 9.26 (s, 1H), 10.87 (s, 1H).

### Beispiel 30A

### 4-(4-Cyano-2-methoxyphenyl)-2-methyl-5-oxo-1,4,5,6-tetrahydro-1,6-naphthyridin-3-carbonitril

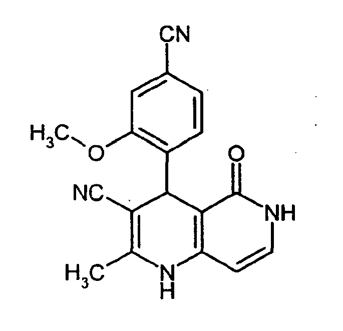

1.05 g (6.51 mmol) 4-Formyl-3-methoxybenzonitril werden mit 684 mg (6.51 mmol) Natrium-1-cyanoprop-1-en-2-olat, 789 mg (7.16 mmol) 4-Aminopyridin-2(1*H*)-on [Searls, T., McLaughlin, L.W., Tetrahedron 55, 11985-11996 (1999)] und 600 µl (9.77 mmol) Essigsäure in 30 ml Isopropanol gelöst und unter Argon 12 h lang unter Rückfluss erhitzt. Nach dem Abkühlen wird filtriert und der verbleibende Feststoff mit Diethylether (40 ml) gewaschen. Es werden 1.96 g (94% d. Th.) der Titelverbindung als weißer Feststoff erhalten.
LC-MS (Methode 4): Rₜ = 1.17 min; [M+H]⁺ = 319
¹H-NMR (300 MHz, DMSO-d₆): δ = 2.01 (s, 3H), 3.83 (s, 3H), 5.02 (s, 1H), 5.94 (d, 1H), 7.03 (d, 1H), 7.19 (d, 1H), 7.32 (d, 1H), 7.45 (s, 1H), 9.88 (s, 1H), 11.06 (s, 1H).

### Beispiel 31A

### 3-[4-Nitro-2-(trifluormethyl)benzyliden]pentan-2,4-dion

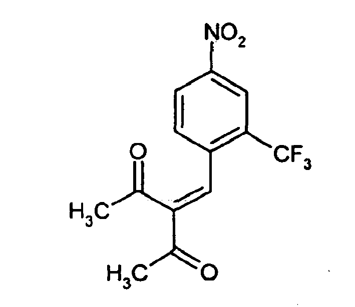

1 g (4.56 mmol) 2-Trifluormethyl-4-nitrobenzaldehyd, 0.51 ml (5.02 mmol) 2,4-Pentandion, 0.4 ml (6.85 mmol) Essigsäure und 90 µl (0.91 mmol) Piperidin in 20 ml wasserfreiem Dichlormethan werden 24 h lang am Wasserabscheider unter Rückfluss gerührt. Nach dem Abkühlen wird die Reaktionslösung nacheinander mit gesättigter Natriumhydrogencarbonat-Lösung und gesättigter Natriumchlorid-Lösung gewaschen. Die organische Phase wird über Magnesiumsulfat getrocknet und eingeengt. Der Rückstand (1.28 g) wird ohne weitere Reinigung in der nächsten Stufe umgesetzt.

LC-MS (Methode 3): Rₜ = 2.34 min; [M+H]⁺ = 202.

### Beispiel 32A

### 3-Acetyl-2-methyl-4-[4-nitro-2-(trifluormethyl)phenyl]-4,6-dihydro-1,6-naphthyridin-5(1H)-on

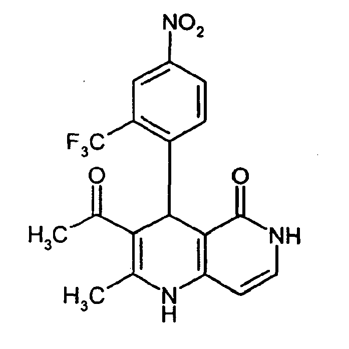

1.28 g (4.24 mmol) 3-[4-Nitro-2-(trifluormethyl)benzyliden]pentan-2,4-dion und 484 mg (4.24 mmol) 4-Aminopyridin-2(1*H*)-on [Searls, T., McLaughlin, L.W., Tetrahedron 55, 11985-11996 (1999)] werden in 25 ml Isopropanol gelöst und unter Argon 12 h lang unter Rückfluss erhitzt. Nach dem Abkühlen wird filtriert und der verbleibende Feststoff mit Diethylether (40 ml) gewaschen. Es werden 990 mg (58% d. Th.) der Titelverbindung als weißer Feststoff erhalten.
LC-MS (Methode 1): Rₜ = 1.51 min; [M+H]⁺ = 394 ¹H-NMR (300 MHz, DMSO-d₆): δ = 2.15 (s, 3H), 2.32 (s, 3H), 5.58 (s, 1H), 5.92 (d, 1H), 7.15 (t, 1H), 7.63 (d, 1H), 8.17 (d, 1H), 8.29 (dd, 1H), 9.32 (s, 1H), 10.84 (d, 1H).

### Beispiel 33A

### Methyl 4-cyano-2-fluorbenzoat

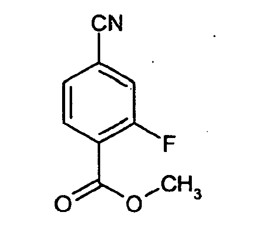

13.20 g (79.9 mmol) 4-Cyano-2-fluorbenzoesäure werden in 300 ml Aceton gelöst. Anschließend werden nacheinander 22.10 g (159.9 mmol) Kaliumcarbonat und 9.08 ml (95.9 mmol) Dimethylsulfat zugesetzt. Es wird 20 h bei Rückflusstemperatur gerührt. Das Reaktionsgemisch wird danach mit 300 ml Wasser versetzt und das Aceton am Rotationsverdampfer entfernt. Es wird mehrfach mit Dichlormethan extrahiert. Die vereinigten organischen Phasen werden mit gesättigter Natriumchlorid-Lösung gewaschen und über Natriumsulfat getrocknet. Dann wird das Lösungsmittel im Vakuum entfernt. Der verbleibende Feststoff wird ohne weitere Aufreinigung weiterverwendet. Es werden 16.1 g (84% d. Th.) der Titelverbindung als farbloser Feststoff erhalten.
GC-MS (Methode 7): Rₜ = 6.23 min; [M]⁺ (EIpos): m/z = 179
¹H-NMR (300 MHz, DMSO-d₆): δ = 3.90 (s, 3H), 7.83 (dd, 1H), 8.01-8.08 (m, 2H).

### Beispiel 34A

### 3-Fluor-4-(hydroxymethyl)benzonitril

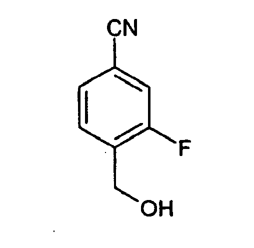

16.10 g (89.9 mmol) Methyl 4-cyano-2-fluorbenzoat werden in 150 ml Methanol gelöst. Anschließend werden portionsweise 3.40 g (89.9 mmol) Natriumborhydrid zugesetzt. Nach erfolgter Umsetzung (DC-Kontrolle) wird mit verdünnter Salzsäure auf pH 3 eingestellt und mehrfach mit Dichlormethan extrahiert. Die vereinigten organischen Phasen werden mit gesättigter Natriumchlorid-Lösung gewaschen und mit Magnesiumsulfat getrocknet. Dann wird das Lösungsmittel im Vakuum entfernt und der Rückstand per Säulenchromatographie aufgereinigt (Kieselgel, Laufmittel: Cyclohexan/Ethylacetat 15:1 → 3:7). Es werden 3.70 g (27.2% d. Th.) der Titelverbindung erhalten.
GC-MS (Methode 7): Rₜ = 6.51 min; [M]⁺ (EIpos): m/z = 151
¹H-NMR (300 MHz, DMSO-d₆): δ = 4.61 (s, 2H), 5.53 (s, 1H), 7.61-7.74 (m, 2H), 7.79 (dd, 1H).

### Beispiel 35A

### 3-Fluor-4-formylbenzonitril

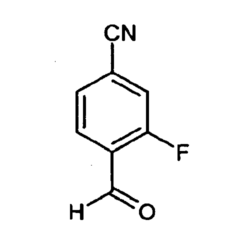

1.00 g (6.62 mmol) 3-Fluor-4-(hydroxymethyl)benzonitril werden in 50 ml Dichlormethan gelöst und mit 9.20 g (105.9 mmol) Mangan(IV)oxid versetzt. Es wird über Nacht bei Raumtemperatur gerührt und dann über eine kurze Kieselgur-Säule filtriert. Das Lösungsmittel wird unter vermindertem Druck abdestilliert und der Rückstand per Säulenchromatographie aufgereinigt (Kieselgel, Laufmittel: Dichlormethan). Es werden 120 mg (12.1% d. Th.) der Titelverbindung erhalten.
GC-MS (Methode 7): Rₜ = 5.11 min; [M]⁺ (EIpos): m/z = 149
¹H-NMR (300 MHz, DMSO-d₆): δ = 7.89 (d, 1H), 8.00 (t, 1H), 8.11 (d, 1H), 10.24 (d, 1H).

### Beispiel 36A

### 4-(3-Acetyl-2-methyl-5-oxo-1,4,5,6-tetrahydro-1,6-naphthyridin-4-yl)-3-fluorbenzonitril

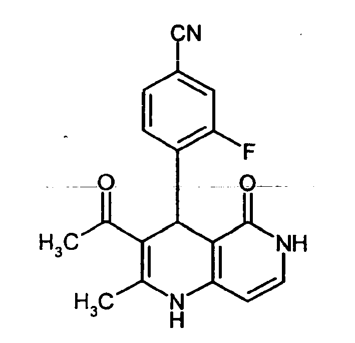

120 mg (0.805 mmol) 3-Fluor-4-formylbenzonitril, 80.6 mg (0.805, mmol) 2,4-Pentandion, 72.5 mg (1.21 mmol) Essigsäure und 14 mg (0.16 mmol) Piperidin werden in 8 ml Dichlormethan gelöst und über Nacht an einem inversen Wasserabscheider unter Rückfluss erhitzt. Der Ansatz zeigt vollständigen Umsatz (DC-Kontrolle, Laufmittel: Cyclohexan/Ethylacetat 5:1). Es wird mit Dichlormethan verdünnt, mit Wasser und gesättigter Natriumchlorid-Lösung gewaschen und mit Natriumsulfat getrocknet. Das Lösungsmittel wird am Rotationsverdampfer entfernt. Es werden 155 mg (83.3% d. Th.) 4-(2-Acetyl-3-oxobut-1-en-1-yl)-3-fluorbenzonitril erhalten, welches ohne weitere Aufreinigung umgesetzt wird.
155 mg (0.670 mmol) der so gewonnenen Benzylidenverbindung werden in 5 ml Isopropanol aufgenommen, mit 73.8 mg (0.67 mmol) 4-Aminopyridin-2(1*H*)-on versetzt und dann 24 h lang in einem verschlossenen Gefäß bei 100°C Badtemperatur umgesetzt. Der Ansatz wird danach eingeengt und der Rückstand mittels präparativer HPLC gereinigt (Eluent: Acetonitril/Wasser mit 0.1% Ameisensäure, Gradient 20:80 → 95:5). Es werden 18 mg (8.3% d. Th., bezogen auf die Zwischenstufe 4-(2-Acetyl-3-oxobut-1-en-1-yl)-3-fluorbenzonitril) der Titelverbindung erhalten.
LC-MS (Methode 4): Rₜ = 1.49 min; [M+H]⁺ (EIpos): m/z = 324
¹H-NMR (300 MHz, DMSO-d₆): δ = 2.13 (s, 3H), 2.30 (s, 3H), 5.25 (s, 1H), 5.91 (d, 1H), 7.15 (t, 1H), 7.41 (t, 1H), 7.54 (d, 1H), 7.65 (d, 1H), 9.34 (s, 1H), 10.98 (s, 1H).

### Beispiel 37A

### 3-Chlor-4-formylbenzonitril

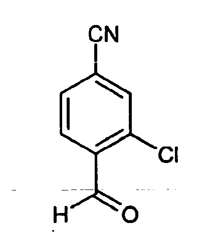

25.0 g (164.91 mmol) 3-Chlor-4-methylbenzonitril werden in 150 ml DMF gelöst und mit 25.55 g (214.39 mmol) *N,N*-Dimethylformamid-dimethylacetal versetzt. Es wird 20 h bei 140°C und dann 4 h bei 180°C Ölbadtemperatur gerührt. Die flüchtigen Komponenten werden am Rotationsverdampfer abgetrennt und der verbleibende Rückstand direkt weiter umgesetzt.

Das so erhaltene, rohe 3-Chlor-4-[2-(dimethylamino)vinyl]benzonitril wird in 500 ml THF/Wasser (1:1) aufgenommen und mit 77.6 g (362.9 mmol) Natriumperiodat versetzt. Es wird 18 h bei Raumtemperatur gerührt und anschließend der ausgefallene Niederschlag per Filtration abgetrennt. Das Filtrat wird mit gesättigter Natriumhydrogencarbonat-Lösung versetzt und dreimal mit Ethylacetat extrahiert. Die vereinigten organischen Phasen werden mit Natriumsulfat getrocknet und das Lösungsmittel am Rotationsverdampfer entfernt. Das Rohprodukt wird säulenchromatographisch aufgereinigt (Kieselgel, Laufmittel: Cyclohexan/Ethylacetat 7:3). Es werden 3.0 g (15% d. Th.) der Titelverbindung erhalten.
GC-MS (Methode 7): R₁ = 6.64 min; [M]⁺ (EIpos): m/z = 165
¹H-NMR (300 MHz, DMSO-d₆): δ = 7.97-8.03 (m, 2H), 8.27 (s, 1H), 10.34 (d, 1H).

### Beispiel 38A

### 4-(3-Acetyl-2-methyl-5-oxo-1,4,5,6-tetrahydro-1,6-naphthyridin-4-yl)-3-chlorbenzonitril

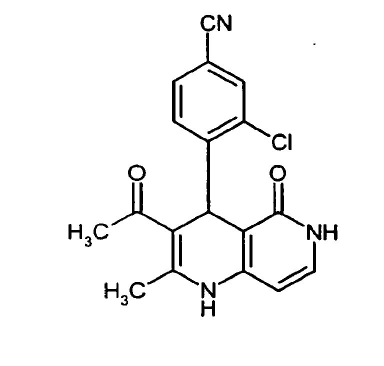

300 mg (1.812 mmol) 3-Chlor-4-formylbenzonitril, 181.4 mg (1.812 mmol) 2,4-Pentandion, 163.2 mg (2.72 mmol) Essigsäure und 30.9 mg (0.36 mmol) Piperidin werden in 15 ml Dichlormethan gelöst und über Nacht an einem inversen Wasserabscheider unter Rückfluss erhitzt. Der Ansatz zeigt vollständigen Umsatz (DC-Kontrolle, Laufmittel: Cyclohexan/Ethylacetat 5:1). Es wird mit Dichlormethan verdünnt, mit Wasser und gesättigter Natriumchlorid-Lösung gewaschen und mit Natriumsulfat getrocknet. Das Lösungsmittel wird am Rotationsverdampfer entfernt. Es werden 403 mg (89.8% d. Th.) 4-(2-Acetyl-3-oxobut-1-en-1-yl)-3-chlorbenzonitril erhalten, welches ohne weitere Aufreinigung umgesetzt wird.
403 mg (1.63 mmol) der so gewonnenen Benzylidenverbindung werden in 8 ml Isopropanol aufgenommen, mit 179.2 mg (1.63 mmol) 4-Aminopyridin-2(1*H*)-on versetzt und 24 h lang auf Rückflusstemperatur erhitzt. Der Ansatz wird danach eingeengt und der Rückstand mittels präparativer HPLC gereinigt (Eluent: Acetonitril/Wasser mit 0.1% Ameisensäure, Gradient 20:80 → 95:5). Es werden 251 mg (45.3% d. Th., bezogen auf die Zwischenstufe 4-(2-Acetyl-3-oxobut-1-en-1-yl)-3-chlorbenzonitril) der Titelverbindung erhalten.
LC-MS (Methode 4): Rₜ = 1.57 min; [M+H]⁺ (Elpos): m/z = 340
¹H-NMR (300 MHz, DMSO-d₆): δ = 2.13 (s, 3H), 2.27 (s, 3H), 5.38 (s, 1H), 5.89 (d, 1H), 7.13 (t, 1H), 7.47 (d, 1H), 7.66 (dd, 1H), 7.82 (d, 1H), 9.31 (s, 1H), 10.89 (d, 1H).

### Beispiel 39A

### 4-(2-Acetyl-3-oxobut-l-en-1-yl)-3-methylbenzonitril

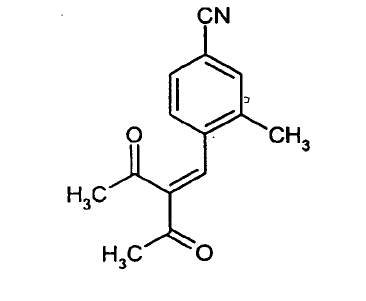

950 mg (6.54 mmol) 3-Methyl-4-formylbenzonitril [S. Levesque et al., Bioorg. Med Chem. Lett. 11, 3161-3164 (2001)], 655.2 mg (6.544 mmol) 2,4-Pentandion, 589.5 mg (9.82 mmol) Essigsäure und 111.5 mg (1.31 mmol) Piperidin werden in 35 ml Dichlormethan gelöst und über Nacht an einem inversen Wasserabscheider unter Rückfluss erhitzt. Nach dem Abkühlen wird zweimal mit Wasser gewaschen und die organische Phase mit Magnesiumsulfat getrocknet. Das Lösungsmittel wird am Rotationsverdampfer entfernt und der Rückstand säulenchromatographisch aufgereinigt (Kieselgel, Laufmittel: zunächst Dichlormethan, dann Isohexan/Ethylacetat 3:1). Nach Einengen der Produktfraktionen wird der Rückstand aus Diethylether kristallisiert und im Hochvakuum getrocknet. Es werden 1.12 g (75.3% d. Th.) der Titelverbindung erhalten.
LC-MS (Methode 3): Rₜ = 1.98 min; [M+H]⁺ (EIpos): m/z = 228
¹H-NMR (300 MHz, DMSO-d₆): δ = 2.11 (s, 3H), 2.40 (s, 3H), 2.47 (s, 3H), 7.25 (d, 1H), 7.68 (d, 1H), 7.80 (s, 1H), 7.84 (s, 1H).

### Beispiel 40A

### 4-(3-Acetyl-2-methyl-5-oxo-1,4,5,6-tetrahydro-1,6-naphthyridin-4-yl)-3-methylbenzonitril

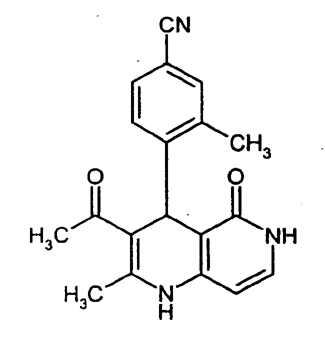

1.10 g (4.84 mmol) 4-(2-Acetyl-3-oxobut-1-en-1-yl)-3-methylbenzonitril werden in 30 ml Isopropanol aufgenommen, mit 592 mg (4.84 mmol) 4-Aminopyridin-2(1*H*)-n versetzt und dann 72 h lang auf Rückflusstemperatur erhitzt. Der Ansatz wird danach eingeengt, der Rückstand erneut in Isopropanol aufgenommen und über Kieselgel mit Isopropanol als Eluent filtriert. Nach Einengen des Filtrats wird der Rückstand mit wenig Methanol versetzt und kristallisiert. Das ausgefallene Produkt wird abfiltriert, mit Diethylether gewaschen und im Hochvakuum getrocknet. Es werden 348 mg (22.5% d. Th.) der Titelverbindung erhalten.
LC-MS (Methode 1): Rₜ = 1.32 min; [M+H]⁺ (EIpos): m/z = 320
¹H-NMR (300 MHz, DMSO-d₆): δ = 2.16 (s, 3H), 2.35 (s, 3H), 2.79 (s, 3H), 5.11 (s, 1H), 5.93 (d, 1H), 7.11 (d, 1H), 7.26 (d, 1H), 7.42-7.49 (m, 2H), 9.25 (s, 1H), 10.89 (d, 1H).

### Beispiele 41

### 4-(3-Acetyl-2-methyl-5-oxo-1,4,5,6-tetrahydro-1,6-naphthyridin-4-yl)-3-(trifluormethyl)benzonitril

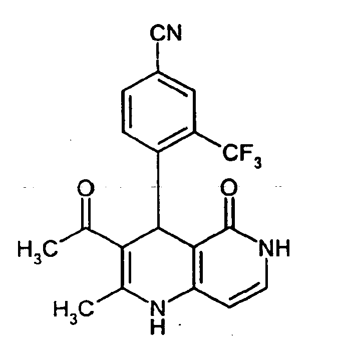

3.00 g (16.20 mmol) 3-Trifluormethyl-4-methylbenzonitril werden in 20 ml DMF gelöst und mit 2.67 g (21.06 mmol) *N,N*-Dimethylformamid-dimethylacetal versetzt. Es wird 20 h bei 140°C gerührt. Die flüchtigen Komponenten werden am Rotationsverdampfer abgetrennt und der verbleibende Rückstand direkt weiter umgesetzt.

Das erhaltene Rohprodukt, welches zu ca. 50% aus 3-Trifluormethyl-4-[2-(dimethylamino)vinyl]-benzonitril besteht, wird in 500 ml THF/Wasser (1:1) aufgenommen und mit 8.20 g (38.3 mmol) Natriumperiodat versetzt. Es wird 18 h bei Raumtemperatur gerührt und anschließend der ausgefallene Niederschlag per Filtration abgetrennt. Das Filtrat wird mit gesättigter Natriumhydrogencarbonat-Lösung versetzt und dreimal mit Ethylacetat extrahiert. Die vereinigten organischen Phasen werden mit Natriumsulfat getrocknet und das Lösungsmittel am Rotationsverdampfer entfernt. Das so erhaltene Produktgemisch (3.07 g) besteht laut GC-MS aus ca. 40% 3-Trifluormethyl-4-formylbenzonitril [GC-MS (Methode 11): Rₜ = 3.29 min; [M]⁺ (EIpos): m/z = 199] und ca. 60% nicht umgesetztem 3-Trifluormethyl-4-methylbenzonitril [GC-MS (Methode 11): Rₜ = 2.88 min; [M]⁺ (Elpos): m/z = 185].
500 mg des so erhaltenen Rohgemisches (Gehalt an 3-Trifluormethyl-4-formylbenzonitril ca. 1 mmol), 251.4 mg (2.511 mmol) 2,4-Pentandion, 226.2 mg (3.77 mmol) Essigsäure und 42.8 mg (0.50 mmol) Piperidin werden in 20 ml Dichlormethan gelöst und über Nacht an einem inversen Wasserabscheider unter Rückfluss erhitzt. Nach dem Abkühlen wird zweimal mit Wasser gewaschen und die organische Phase mit Magnesiumsulfat getrocknet. Das Lösungsmittel wird am Rotationsverdampfer entfernt. Das erhaltene Rohmaterial (288 mg) wird mit 592 mg (4.84 mmol) 4-Aminopyridin-2(1*H*)-on versetzt, in 15 ml Isopropanol gelöst und dann 24 h lang auf Rückflusstemperatur erhitzt. Der Ansatz wird danach eingeengt, der Rückstand erneut in Isopropanol aufgenommen und über Kieselgel mit Isopropanol als Eluent filtriert. Nach Einengen des Filtrats wird der Rückstand mit wenig Methanol versetzt, wobei Kristallisation eintritt. Das ausgefallene Produkt wird abfiltriert, mit Diethylether gewaschen und im Hochvakuum getrocknet. Es werden 50 mg (13.3% d. Th., bezogen auf die eingesetzte Menge an 3-Trifluormethyl-4-formylbenzonitril) der Titelverbindung erhalten.
LC-MS (Methode 1): Rₜ = 1.39 min; [M+H]⁺ (EIpos): m/z = 374
¹H-NMR (300 MHz, DMSO-d₆): δ = 2.14 (s, 3H), 2.29 (s, 3H), 5.54 (s, 1H), 5.90 (d, 1H), 7.14 (t, 1H), 7.53 (d, 1H), 7.91 (d, 1H), 7.97 (d, 1H), 9.24 (s, 1H), 10.83 (d, 1H).

### Beispiel 42A

### 4-Formyl-1-naphthonitril

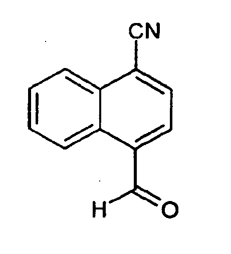

2.50 g (14.95 mmol) 4-Methyl-1-naphthonitril werden in 40 ml Tetrachlormethan gelöst und mit 3.19 g (17.94 mmol) *N*-Bromsuccinimid sowie 245 mg (1.50 mmol) 2,2'-Azobis-2-methylpropannitril versetzt. Es wird über Nacht bei Rückflusstemperatur gerührt. Nach dem Abkühlen wird das Produkt abfiltriert. Es werden 2.75 g (74.7% d. Th.) 4-(Brommethyl)-1-naphthonitril in 90%-iger Reinheit erhalten, welches ohne weitere Aufreinigung umgesetzt wird.
2.75 g (11.17 mmol) des so erhaltenen Bromids werden in 60 ml Acetonitril gelöst und mit 2 g Molekularsieb (3Å) versetzt. Anschließend werden 1.44 g (12.29 mmol) N-Methylmorpholin-N-oxid hinzugefügt und das Gemisch über Nacht bei Raumtemperatur gerührt. Der Ansatz wird danach über Kieselgel filtriert und das Filtrat eingeengt. Der Rückstand wird über eine Biotage-Kartusche (40 M) gereinigt (Eluent: Isohexan/Ethylacetat 3:1). Die Produktfraktionen werden vereinigt, das Lösungsmittel am Rotationsverdampfer entfernt und der Rückstand anschließend mit Diethylether verrührt, wobei Kristallisation eintritt. Es wird mit wenig Diethylether gewaschen und im Hochvakuum getrocknet. Es werden 254 mg (12.6% d. Th.) der Titelverbindung erhalten.
LC-MS (Methode 4): Rₜ = 2.27 min; [M+H]⁺ (EIpos): m/z = 182
¹H-NMR (300 MHz, CDCl₃): δ = 7.79-7.87 (m, 2H), 8.05 (d, 1H), 8.09 (d, 1H), 8.37 (m, 1H), 9.27 (m, 1H), 10.51 (s, 1H).

### Beispiel 43A

### 4-(2-Acetyl-3-oxobut-1-en-1-yl)-1-naphthonitril

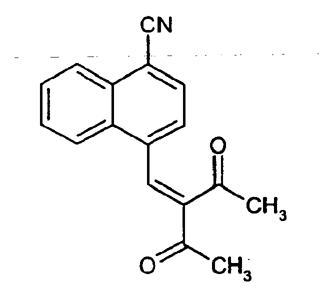

240 mg (1.33 mmol) 4-Formyl-1-naphthonitril, 132.6 mg (1.33 mmol) 2,4-Pentandion, 119.3 mg (1.99 mmol) Essigsäure und 22.6 mg (0.27 mmol) Piperidin werden in 20 ml Dichlormethan gelöst und über Nacht an einem inversen Wasserabscheider unter Rückfluss erhitzt. Nach dem Abkühlen wird zweimal mit Wasser gewaschen und die organische Phase mit Magnesiumsulfat getrocknet. Das Lösungsmittel wird am Rotationsverdampfer entfernt und der Rückstand säulenchromatographisch aufgereinigt (Kieselgel, Laufmittel: zunächst Dichlormethan, dann Isohexan/Ethylacetat 3:1). Nach Einengen der Produktfraktionen wird der Rückstand aus Diethylether kristallisiert und im Hochvakuum getrocknet. Es werden 340 mg (97.5% d. Th.) der Titelverbindung erhalten.
LC-MS (Methode 1): Rₜ = 2.03 min; [M+H]⁺ (EIpos): m/z = 264
¹H-NMR (300 MHz, CDCl₃): δ = 2.09 (s, 3H), 2.60 (s, 3H), 7.49 (d, 1H), 7.84 (t, 1H), 7.91 (t, 1H), 8.19 (t, 2H), 8.31 (d, 1H), 8.36 (s, 1H).

### Beispiel 44A

### 4-(3-Acetyl-2-methyl-5-oxo-1,4,5,6-tetrahydro-1,6-naphthyridirin-4-yl)-1-naphthonitril

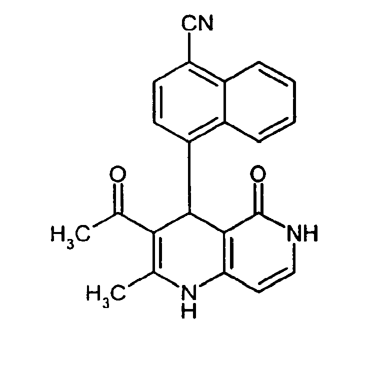

325 mg (1.23 mmol) 4-(2-Acetyl-3-oxobut-1-en-1-yl)-1-naphthonitril werden in 20 ml Isopropanol aufgenommen, mit 151 mg (1.23 mmol) 4-Aminopyridin-2(1*H*)-on versetzt und dann 72 h lang auf Rückflusstemperatur erhitzt. Der Ansatz wird danach eingeengt und der Rückstand mittels präparativer HPLC gereinigt (Eluent: Acetonitril/Wasser mit 0.1% Ameisensäure, Gradient 20:80 → 95:5). Die Produktfraktionen werden vereinigt, das Lösungsmittel entfernt und der Rückstand mit Diethylether verrührt. Das ausgefallene Produkt wird abfiltriert und im Hochvakuum getrocknet. Es werden 91 mg (21.2% d. Th.) der Titelverbindung erhalten.
LC-MS (Methode 1): Rₜ = 1.51 min; [M+H]⁺ (EIpos): m/z = 356
¹H-NMR (300 MHz, DMSO-d₆): δ = 2.03 (s, 3H), 2.39 (s, 3H), 5.90 (s, 1H), 5.97 (d, 1H), 7.11 (d, 1H), 7.49 (d, 1H), 7.73 (m, 2H), 8.03 (d, 2H), 9.10 (d, 1H), 9.39 (s, 1H), 10.84 (s, 1H).

### Beispiel 45A

### Natrium 1-cyano-3,3,3-trifluorprop-1-en-2-olat

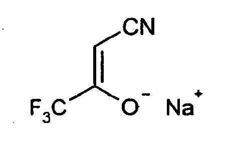

350 mg (14.6 mmol) Natrium werden unter Argon langsam in 20 ml absolutes Methanol eingetragen. Nachdem sich das Natrium vollständig gelöst hat, werden innerhalb von 5 min 2 g (14.6 mmol) 5-Trifluormethylisoxazol [I. I. Gerus et al., J. Org. Chem. USSR (Engl. Transl.) 26, 1623-1628 (1990); Zh. Org. Khim. 26, 1877-1883 (1990)] zugetropft. Man rührt über Nacht bei Raumtemperatur. Nach Entfernen des Lösungsmittels im Vakuum verbleibt das Produkt als farbloser Feststoff. Es werden 2.31 g (90% d. Th.) erhalten, welche ohne weitere Reinigung eingesetzt werden.
¹H-NMR (400 MHz, DMSO-d₆): δ = 3.68 (s, 1H).

### Beispiel 46A

### 4-(4-Cyano-2-methoxyphenyl)-5-oxo-2-(trifluormethyl)-1,4,5,6-tetrahydro-1,6-naphthyridin-3-carbonitril

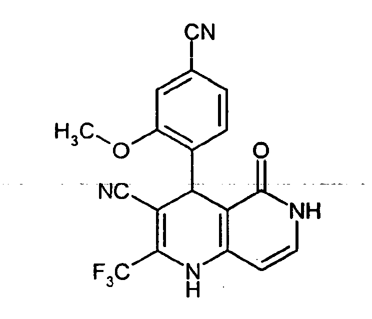

500 mg (3.1 mmol) 4-Formyl-3-methoxybenzonitril werden mit 493 mg (3.1 mmol) Natrium 1-cyano-3,3,3-trifluorprop-1-en-2-olat, 341 mg (3.1 mmol) 4-Aminopyridin-2(1*H*)-on [Searls, T., McLaughlin, L.W., Tetrahedron 55, 11985-11996 (1999)] und 266 µl (4.65 mmol) Essigsäure in 40 ml Isopropanol gelöst und unter Argon 12 h lang unter Rückfluss erhitzt. Nach dem Abkühlen wird die Lösung eingeengt, und der Rückstand wird in 45 ml Essigsäure erneut über Nacht unter Rückfluss gerührt. Die Lösung wird nach dem Abkühlen konzentriert und der Rückstand über präparative HPLC gereinigt. Es werden 392 mg (34% d. Th.) der Titelverbindung als weißer Feststoff erhalten.
LC-MS (Methode 1): Rₜ = 1.63 min; [M+H]⁺= 373
¹H-NMR (300 MHz, DMSO-d₆): δ = 3.83 (s, 3H), 5.12 (s, 1H), 6.12 (d, 1H), 7.21 (d, 1H), 7.25 (d, 1H), 7.38 (dd, 1H), 7.51 (d, 1H), 10.29 (s, 1H), 11.22 (s, 1H).

### Beispiel 47A

### 3-Methoxy-4-(2-methyl-3-nitro-5-oxo-1,4,5,6-tetrahydro-1,6-naphthyridin-4-yl)-benzonitril

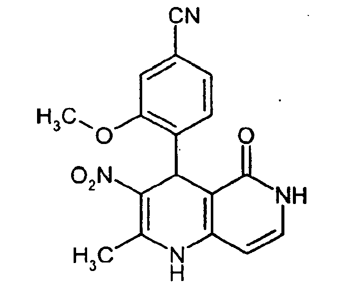

688 mg (4.26 mmol) 4-Formyl-3-methoxybenzonitril werden mit 440 mg (4.26 mmol) 1-Nitroaceton, 470 mg (4.26 mmol) 4-Aminopyridin-2(1*H*)-on [Searls, T., McLaughlin, L.W., Tetrahedron 55, 11985-11996 (1999)] und 367 µl (6.40 mmol) Essigsäure in 10 ml Isopropanol gelöst und unter Argon 12 h lang unter Rückfluss erhitzt. Nach dem Abkühlen wird DMSO (5 ml) hinzugefügt und die Reaktionslösung direkt mittels präparativer HPLC gereinigt. Es werden 41 mg (2% d. Th.) der Titelverbindung als brauner Feststoff erhalten.
LC-MS (Methode 1): Rₜ = 1.30 min; [M+H]⁺ = 339.

### Beispiele 48A

### 4-(4-Cyano-2-methoxyphenyl)-2,7-dimethyl-5-oxo-1,4,5,6-tetrahydro-1,6-naphthyridin-3-carbonitril

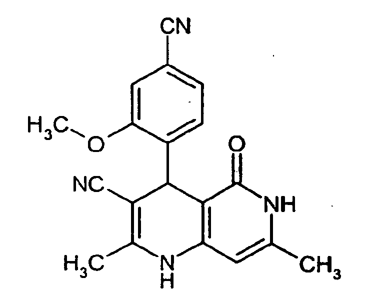

908 mg (5.64 mmol) 4-Formyl-3-methoxybenzonitril werden mit 592 mg (5.64 mmol) Natrium 1-cyanoprop-1-en-2-olat, 700 mg (5.64 mmol) 4-Amino-6-methylpyndin-2(1*H*)-on [Bisagni, E., Hung, N.C., Synthesis, 765-766 (1984)] und 484 µl (8.46 mmol) Essigsäure in 30 ml 2-Propanol gelöst und unter Argon 12 h lang auf Rückflusstemperatur erhitzt. Nach dem Abkühlen wird die Reaktionsmischung filtriert und der verbleibende Feststoff mit Diethylether (20 ml) gewaschen. Es werden 1798 mg (96% d. Th.) der Titelverbindung als weißer Feststoff erhalten.
LC-MS (Methode 4): Rₜ = 1.70 min; [M+H]⁺ (EIpos): m/z = 333
¹H-NMR (300 MHz, DMSO-d₆): δ = 2.00 (s, 3H), 2.09 (s, 3H), 3.84 (s, 3H), 4.99 (s, 1H), 5.71 (s, 1H), 7.02 (d, 1H), 7.32 (dd, 1H), 7.45 (d, 1H), 9.79 (s, 1H), 11.04 (s, 1H).

### Beispiel 49A

### 4-(4-Cyano-2-methoxyphenyl)-2,8-dimethyl-5-oxo-1,4,5,6-tetrahydro-1,6-naphthyridin-3-carbonitril

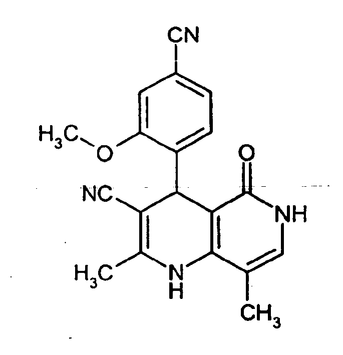

1773 mg (11.0 mmol) 4-Formyl-3-methoxybenzonitril werden mit 1156 mg (11.0 mmol) Natrium 1-cyanoprop-1-en-2-olat, 1366 mg (11.0 mmol) 4-Amino-5-methylpyridin-2(1*H*)-on [Bisagni, E., Hung, N.C., Synthesis, 765-766 (1984)] und 945 µl (16.5 mmol) Essigsäure in 50 ml 2-Propanol gelöst und unter Argon 20 h lang auf Rückflusstemperatur erhitzt. Nach dem Abkühlen wird die Reaktionsmischung filtriert und das Präzipitat mit 2-Propanol und Diethylether nachgewaschen. Das Rohprodukt wird in 800 ml Dichlormethan/Methanol in der Wärme gelöst, mit 500 ml Essigsäureethylester versetzt und anschließend wieder auf ein Volumen von 50 ml eingeengt. Das ausgefallene Produkt wird abfiltriert und mit Essigsäureethylester und Diethylether gewaschen. Nach dem Trocknen im Hochvakuum werden 3.63 g (99% d. Th.) der Titelverbindung in Form gelblicher Kristalle erhalten.
LC-MS (Methode 4): Rₜ = 1.71 min; [M+H]⁺ (EIpos): m/z = 333.

### Ausführungsbeispiele:

### Beispiel 1

### 8-(3-Acetyl-5-ethoxy-2-methyl-1,4-dihydro-1,6-naphthyridin-4-yl)-2-methyl-4H-chromen-4-on

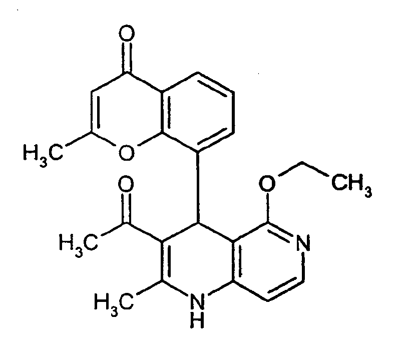

60 mg (0.16 mmol) 3-Acetyl-2-methyl-4-(2-methyl-4-oxo-4*H*-chromen-8-yl)-4,6-dihydro-1,6-naphthyridin-5(1*H*)-on werden in THF (4 ml) suspendiert und mit 0.18 ml (0.18 mmol) *N'''-(tert.-*Butyl)-*N,N',N''*-tris[tris(dimethylamino)phosphoranyliden]phosphorimid-triamid (Phosphazen-Base P₄-t-Bu; 1 M in Hexan) versetzt. Zu der dunkelroten Lösung werden dann 25 µl (0.199 mmol) Ethyltrifluormethansulfonat gegeben. Die entfärbte Lösung wird 10 min nachgerührt, dann mit Methanol versetzt und eingeengt. Man reinigt den Rückstand mittels präparativer HPLC und erhält 35 mg (54% d. Th.) der Titelverbindung als weißen Feststoff.
LC-MS (Methode 1): Rₜ = 1.59 min; [M+H]⁺ = 391
¹H-NMR (300 MHz, DMSO-d₆): δ = 1.03 (t, 3H), 2.14 (s, 3H), 2.34 (s, 3H), 2.39 (s, 3H), 4.06 (m, 2H), 5.63 (s, 1H), 6.21 (s, 1H), 6.53 (d, 1H), 7.32 (t, 1H), 7.63 (dd, 1H), 7.75 (m, 1H), 7.79 (dd, 1H), 9.55 (s, 1H).

### Beispiel 2

### 4-(3-Acetyl-5-ethoxy-2-methyl-1,4-dihydro-1,6-naphthyridin-4-yl)-3-methoxybenzonitril

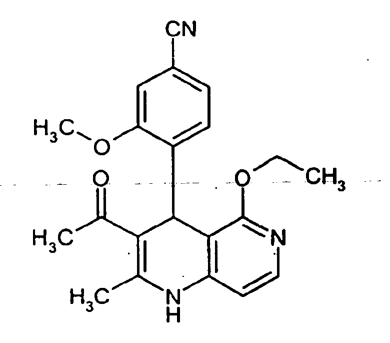

Die Titelverbindung wird analog zu Beispiel 1 aus 2.13 g (6.37 mmol) 4-(3-Acetyl-2-methyl-5-oxo-1,4,5,6-tetrahydro-1,6-naphthyridin-4-yl)-3-methoxybenzonitril hergestellt. Man erhält 970 mg (42% d. Th.) des Produkts als weißen Feststoff.
LC-MS (Methode 1): Rₜ = 1.84 min; [M+H]⁺= 364
¹H-NMR (300 MHz, DMSO-d₆): δ = 1.14 (t, 3H), 2.10 (s, 3H), 2.26 (s, 3H), 3.83 (s, 3H), 4.11 (q, 2H), 5.48 (s, 1H), 6.50 (d, 1H), 7.21 (d, 1H), 7.28 (dd, 1H), 7.37 (d, 1H), 7.74 (d, 1H), 9.39 (s, 1H).

### Beispiel 3

### ent-4-(3-Acetyl-5-ethoxy-2-methyl-1,4-dihydro-1,6-naphthyridin-4-yl)-3-methoxybenzonitril (Enantiomer A und Enantiomer B)

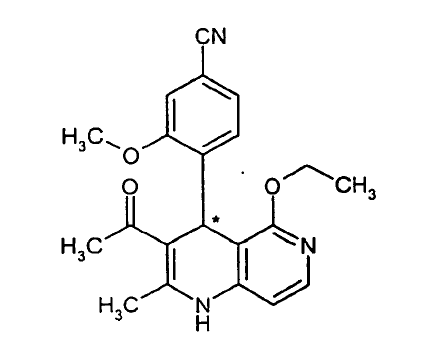

390 mg des Racemats aus Beispiel 2 werden mittels HPLC an chiraler Phase in die Enantiomere getrennt (Säule: 670 mm x 40 mm; Kieselgel-Phase basierend auf dem chiralen Selektor Poly(N-methacryloyl-D-leucin-*tert.*-butylamid; Eluent: Isohexan/Essigsäureethylester 1:1; Temperatur: 24°C; Fluss: 80 ml/min; UV-Detektion: 270 nm).

### Enantiomer A:

Ausbeute: 192 mg

HPLC (Methode 9): Rₜ = 2.35 min.

### Enantiomer B:

Ausbeute: 183 mg

HPLC (Methode 9): Rₜ = 2.71 min.

### Beispiel 4

### 5-Ethoxy-2-methyl-4-(2-methyl-4-oxo-4H-chromen-8-yl)-1,4-dihydro-1,6-naphthyridin-3-carbonitril

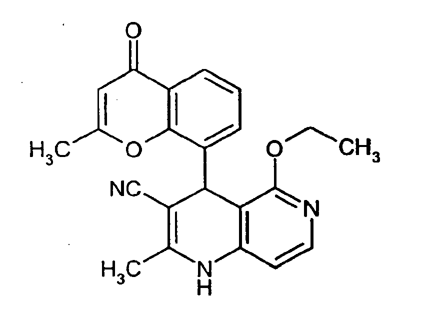

Die Titelverbindung wird analog zu Beispiel 1 aus 71 mg (0.20 mmol) 2-Methyl-4-(2-methyl-4-oxo-4*H*-chromen-8-yl)-5-oxo-1,4,5,6-tetrahydro-1,6-naphthyridin-3-carbonitril hergestellt. Man erhält 14 mg (18% d. Th.) des Produkts als weißen Feststoff.
LC-MS (Methode 3): Rₜ = 1.96 min; [M+H]⁺ = 374
¹H-NMR (300 MHz, DMSO-d₆): δ = 0.89 (t, 3H), 2.11 (s, 3H), 2.34 (s, 3H), 3.99 (m, 2H), 5.27 (s, 1H), 6.23 (s, 1H), 6.50 (d, 1H), 7.39 (t, 1H), 7.54 (dd, 1H), 7.81 (d, 1H), 7.88 (dd, 1H), 9.87 (s, 1H).

### Beispiel 5

### 4-(3-Acetyl-5-ethoxy-2-methyl-1,4-dihydro-1,6-naphthyridin-4-yl)-benzonitril

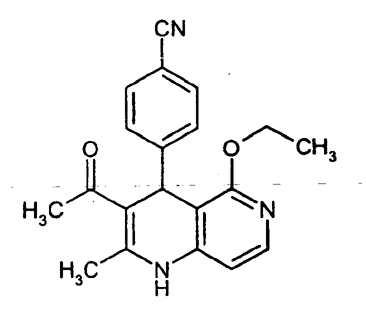

Die Titelverbindung wird analog zu Beispiel 1 aus 103 mg (0.33 mmol) 4-(3-Acetyl-2-methyl-5-oxo-1,4,5,6-tetrahydro-1,6-naphthyridin-4-yl)-benzonitril hergestellt. Man erhält 27 mg (24% d. Th.) des Produkts als weißen Feststoff.
LC-MS (Methode 4): Rₜ = 2.27 min; [M+H]⁺ = 334
¹H-NMR (300 MHz, DMSO-d₆): δ = 1.11 (t, 3H), 2.10 (s, 3H), 2.37 (s, 3H), 3.69 (m, 1H), 3.86 (m, 1H), 5.17 (s, 1H), 5.96 (d, 1H), 7.44 (d, 2H), 7.46 (d, 1H), 7.67 (d, 2H), 9.34 (s, 1H).

### Beispiel 6

### 4-(3-Acetyl-5-isopropoxy-2-methyl-1,4-dihydro-1,6-naphthyridin-4-yl)-benzonitril

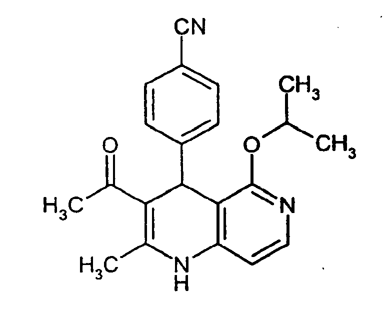

100 mg (0.32 mmol) 4-(3-Acetyl-2-methyl-5-oxo-1,4,5,6-tetrahydro-1,6-naphthyridin-4-yl)-benzonitril werden in DMF (4 ml) suspendiert und mit 14 mg (0.36 mmol) Natriumhydrid (60% in Mineralöl) versetzt. Zu der dunkelroten Lösung werden dann 34 µl (0.34 mmol) 2-Iodpropan gegeben. Die Lösung wird 24 h bei RT gerührt, dann mit Methanol versetzt und mittels präparativer HPLC gereinigt. Man erhält 18 mg (16% d. Th.) der Titelverbindung als weißen Feststoff.
LC-MS (Methode 3): Rₜ = 2.24 min; [M+H]⁺ = 348 ¹H-NMR (300 MHz, DMSO-d₆): δ = 1.09 (d, 3H), 1.31 (d, 3H), 2.15 (s, 3H), 2.39 (s, 3H), 5.14 (m, 1H), 5.15 (s, 1H), 6.51 (d, 1H), 7.39 (d, 2H), 7.68 (d, 1H), 7.77 (d, 2H), 9.51 (s, 1H).

### Beispiel 7

### 8-(3-Acetyl-5-propoxy-2-methyl-1,4-dihydro-1,6-naphthyridin-4-yl)-2-methyl-4H-chromen-4-on

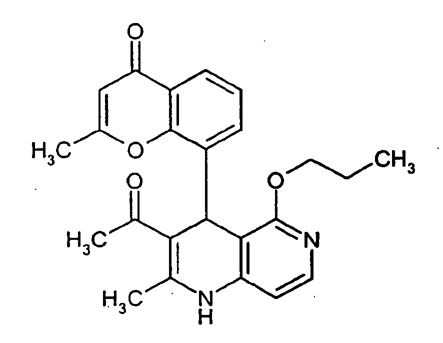

130 mg (0.35 mmol) 3-Acetyl-2-methyl-4-(2-methyl-4-oxo-4*H*-chromen-8-yl)-4,6-dihydro-1,6-naphthyridin-5(1*H*)-on werden in THF (8 ml) suspendiert und mit 0.39 ml (0.39 mmol) *N'''*(*tert.-*Butyl)-*N,N',N''*-tris[tris(dimethylamino)phosphoranyliden]phosphorimid-triamid (Phosphazen-Base P₄-t-Bu; 1 M in Hexan) versetzt. Zu der dunkelroten Lösung werden dann 68 µl (0.41 mmol) Di-*n*-propylsulfat gegeben. Die entfärbte Lösung wird 10 min nachgerührt, dann mit Methanol versetzt und eingeengt. Man reinigt den Rückstand mittels präparativer HPLC und erhält 63 mg (43% d. Th.) der Titelverbindung als weißen Feststoff.
LC-MS (Methode 1): Rₜ = 1.63 min; [M+H]⁺ = 405
¹H-NMR (300 MHz, DMSO-d₆): δ = 0.80 (t, 3H), 1.56 (m, 2H), 2.14 (s, 3H), 2.34 (s, 3H), 2.38 (s, 3H), 4.04 (m, 2H), 5.62 (s, 1H), 6.21 (s, 1H), 6.53 (d, 1H), 7.32 (t, 1H), 7.63 (dd, 1H), 7.75 (m, 1H), 7.79 (dd, 1H), 9.54 (s, 1H).

### Beispiel 8

### 4-(3-Acetyl-5-propoxy-2-methyl-1,4-dihydro-1,6-naphthyridin-4-yl)-3-methoxybenzonitril

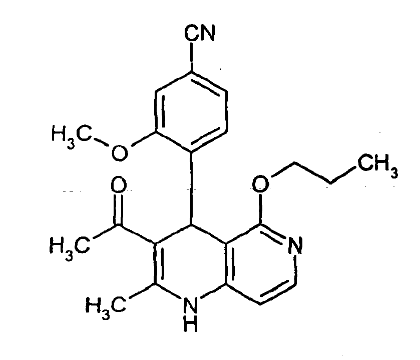

Die Titelverbindung wird analog zu Beispiel 7 aus 428 mg (1.27 mmol) 4-(3-Acetyl-2-methyl-5-oxo-1,4,5,6-tetrahydro-1,6-naphthyridin-4-yl)-3-methoxybenzonitril hergestellt. Man erhält 142 mg (29% d. Th.) des Produkts als weißen Feststoff.
LC-MS (Methode 4): Rₜ = 2.27 min; [M+H]⁺ = 378
¹H-NMR (300 MHz, DMSO-d₆): δ = 0.81 (t, 3H), 1.56 (m, 2H), 2.11 (s, 3H), 2.25 (s, 3H), 3.82 (s, 3H), 4.04 (m, 2H), 5.49 (s, 1H), 6.51 (d, 1H), 7.20 (d, 1H), 7.27 (d, 1H), 7.37 (s, 1H), 7.75 (d, 1H), 9.42 (s, 1H).

### Beispiel 9

### ent-8-[3-Acetyl-5-ethoxy-2-methyl-1,4-dihydro-1,6-naphthyzidin-4-yl]-2-methyl-4H-chromen-4-on

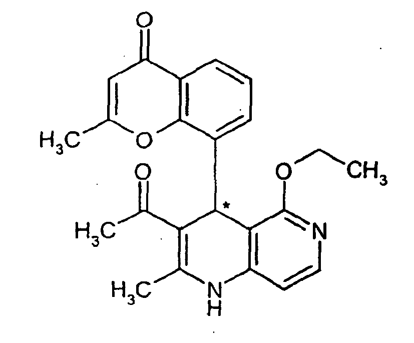

940 mg (2.59 mmol) *ent*-3-Acetyl-2-methyl-4-(2-methyl-4-oxo-4*H*-chromen-8-yl)-4,6-dihydro-1,6-naphthyridin-5(1*H*)-on (Beispiel 23A) werden unter Argonatmosphäre in Dichlormethan (30 ml) suspendiert, mit 985 mg (5.18 mmol) Triethyloxoniumtetrafluoroborat versetzt und 2 h bei RT gerührt. Die Reaktionsmischung wird dann mit Methanol versetzt und eingeengt. Man reinigt den Rückstand durch präparative HPLC und erhält 300 mg (30% d. Th.) der enantiomerenreinen Titelverbindung als weißen Feststoff.
HPLC (Methode 10): Rₜ = 3.20 min
LC-MS (Methode 1): Rₜ = 1.59 min; [M+H]⁺ = 391
¹H-NMR (300 MHz, DMSO-d₆): δ = 1.03 (t, 3H), 2.14 (s, 3H), 2.34 (s, 3H), 2.39 (s, 3H), 4.06 (m, 2H), 5.63 (s, 1H), 6.21 (s, 1H), 6.53 (d, 1H), 7.32 (t, 1H), 7.63 (dd, 1H), 7.75 (m, 1H), 7.79 (dd, 1H), 9.55 (s, 1H).

### Beispiel 10

### 3-Acetyl-2-methyl-4-(2-methyl-4-oxo-4H-chromen-8-yl)-1,4-dihydro-1,6-naphthyridin-5-yl ethansulfonat

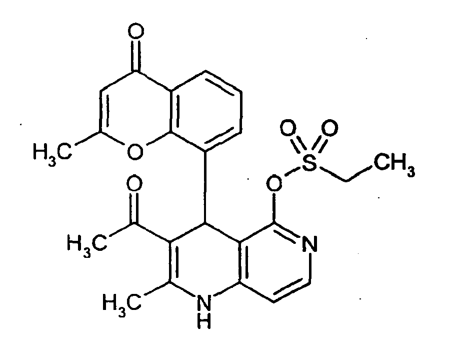

133 mg (0.367 mmol) 3-Acetyl-2-methyl-4-(2-methyl-4-oxo-4*H*-chromen-8-yl)-4,6-dihydro-1,6-naphthyridin-5(1*H*)-on werden in Pyridin (5 ml) suspendiert und mit 70 µl (0.73 mmol) Ethansulfonylchlorid versetzt. Die Suspension wird 3 h bei RT gerührt (wobei das Edukt sich löst), dann mit Methanol versetzt und eingeengt. Man reinigt den Rückstand durch präparative HPLC und erhält 63 mg (43% d. Th.) der Titelverbindung als weißen Feststoff.
LC-MS (Methode 4): Rₜ = 1.88 min; [M+H]⁺ = 455
¹H-NMR (300 MHz, DMSO-d₆): δ = 1.18 (t, 3H), 2.18 (s, 3H), 2.36 (s, 3H), 2.39 (s, 3H), 3.69 (m, 2H), 5.76 (s, 1H), 6.19 (s, 1H), 6.91 (d, 1H), 7.32 (t, 1H), 7.62 (dd, 1H), 7.81 (dd, 1H), 7.95 (m, 1H), 9.94 (s, 1H).

### Beispiel 11

### 3-Acetyl-2-methyl-4-(2-methyl-4-oxo-4H-chromen-8-yl)-1,4-dihydro-1,6-naphthyridin-5-yl methansulfonat

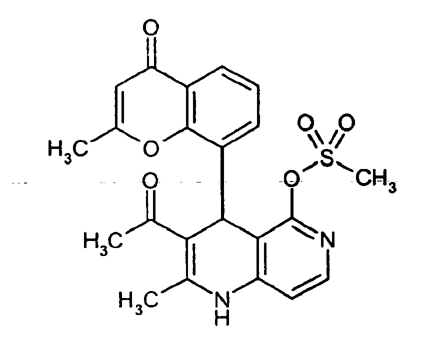

Die Titelverbindung wird analog zu Beispiel 10 aus 50 mg (0.13 mmol) 3-Acetyl-2-methyl-4-(2-methyl-4-oxo-4*H*-chromen-8-yl)-4,6-dihydro-1,6-naphthyridin-5(1*H*)-on und 12 µl (0.15 mmol) Methansulfonsäurechlorid hergestellt. Man erhält 10.4 mg (15% d. Th.) des Produkts als hellgelben Feststoff.
LC-MS (Methode 2): Rₜ = 1.84 min; [M+H]⁺ = 425
¹H-NMR (300 MHz, DMSO-d₆): δ = 2.18 (s, 3H), 2.36 (s, 3H), 2.39 (s, 3H), 3.52 (s, 3H), 5.74 (s, 1H), 6.19 (s, 1H), 6.92 (d, 1H), 7.32 (t, 1H), 7.62 (dd, 1H), 7.81 (dd, 1H), 7.97 (d, 1H), 9.96 (s, 1H).

### Beispiel 12

### 3-Acetyl-2-methyl-4-(2-methyl-4-oxo-4H-chromen-8-yl)-1,4-dihydro-1,6-naphthyridin-5-yl propansulfonat

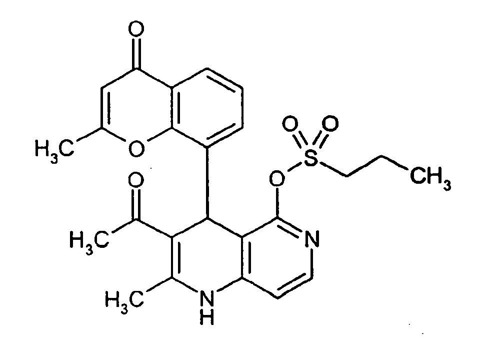

Die Titelverbindung wird analog zu Beispiel 10 aus 50 mg (0.13 mmol) 3-Acetyl-2-methyl-4-(2-methyl-4-oxo-4*H*-chromen-8-yl)-4,6-dihydro-1,6-naphthyridin-5(1*H*)-on und 17 µl (0.15 mmol) Propansulfonsäurechlorid hergestellt. Man erhält 46 mg (71% d. Th.) des Produkts als hellgelben Feststoff.
LC-MS (Methode 3): Rₜ = 1.95 min; [M+H]⁺ = 469
¹H-NMR (300 MHz, DMSO-d₆): δ = 0.89 (t, 3H), 1.64 (m, 2H), 2.18 (s, 3H), 2.36 (s, 3H), 2.38 (s, 3H), 3.68 (m, 2H), 5.76 (s, 1H), 6.19 (s, 1H), 6.90 (d, 1H), 7.32 (t, 1H), 7.62 (dd, 1H), 7.81 (dd, 1H), 7.96 (d, 1H), 9.93 (s, 1H).

### Beispiel 13

### 3-Acetyl-2-methyl-4-(2-methyl-4-oxo-4H-chromen-8-yl)-1,4-dihydro-1,6-naphthyridin-5-yl butansulfonat

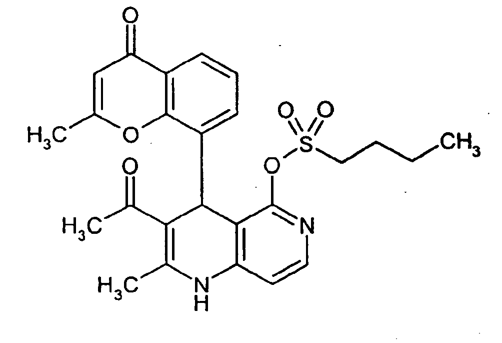

Die Titelverbindung wird analog zu Beispiel 10 aus 50 mg (0.13 mmol) 3-Acetyl-2-methyl-4-(2-methyl-4-oxo-4*H*-chromen-8-yl)-4,6-dihydro-1,6-naphthyridin-5(1*H*)-on und 24 mg (0.15 mmol) Butansulfonsäurechlorid hergestellt. Man erhält 48 mg (72% d. Th.) des Produkts als hellgelben Feststoff.
LC-MS (Methode 3): Rₜ = 2.09 min; [M+H]⁺ = 483
¹H-NMR (400 MHz, DMSO-d₆): δ = 0.76 (t, 3H), 1.27 (m, 2H), 1.53 (m, 2H), 2.18 (s, 3H), 2.36 (s, 3H), 2.39 (s, 3H), 3.70 (t, 2H), 5.76 (s, 1H), 6.19 (s, 1H), 6.90 (d, 1H), 7.32 (t, 1H), 7.62 (dd, 1H), 7.81 (dd, 1H), 7.95 (d, 1H), 9.94 (s, 1H).

### Beispiel 14

### 3-Acetyl-2-methyl-4-(2-methyl-4-oxo-4H-chromen-8-yl)-1,4-dihydro-1,6-naphthyridin-5-yl cyclopropansulfonat

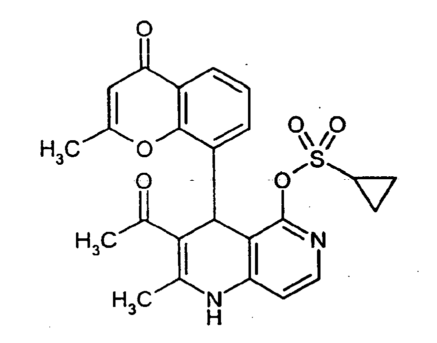

Die Titelverbindung wird analog zu Beispiel 10 aus 50 mg (0.13 mmol) 3-Acetyl-2-methyl-4-(2-methyl-4-oxo-4*H*-chromen-8-yl)-4,6-dihydro-1,6-naphthyridin-5(1*H*)-on und 22 mg (0.15 mmol) Cyclopropansulfonsäurechlorid hergestellt. Man erhält 54 mg (84% d. Th.) des Produkts als hellgelben Feststoff.
LC-MS (Methode 1): Rₜ = 1.65 min; [M+H]⁺ = 467.

### Beispiel 15

### 3-Acetyl-2-methyl-4-(2-methyl-4-oxo-4H-chromen-8-yl)-1,4-dihydro-1,6-naphthyridin-5-yl thiophen-2-sulfonat

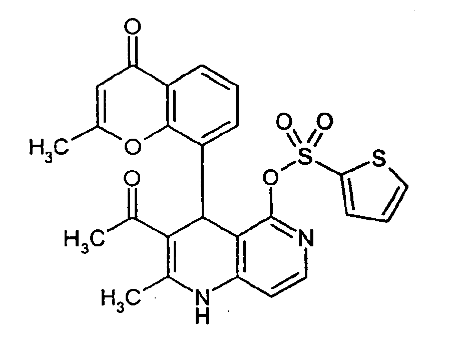

Die Titelverbindung wird analog zu Beispiel 10 aus 50 mg (0.13 mmol) 43-Acetyl-2-methyl-4-(2-methyl-4-oxo-4*H*-chromen-8-yl)-4,6-dihydro-1,6-naphthyridin-5(1*H*)-on und 28 mg (0.15 mmol) Thiophen-2-sulfonsäurechlorid hergestellt. Man erhält 57 mg (81% d. Th.) des Produkts als hellgelben Feststoff.
LC-MS (Methode 1): Rₜ = 1.78 min; [M+H]⁺ = 509 ¹H-NMR (400 MHz, DMSO-d₆): δ = 2.17 (s, 3H), 2.35 (s, 3H), 2.39 (s, 3H), 5.72 (s, 1H), 6.20 (s, 1H), 6.86 (d, 1H), 7.18 (dd, 1H), 7.30 (t, 1H), 7.52 (dd, 1H), 7.74 (dd, 1H), 7.83 (dd, 1H), 7.86 (d, 1H), 8.12 (dd, 1H), 9.92 (s, 1H).

### Beispiel 16

### 3-Acetyl-4-(4-cyano-2-methoxyphenyl)-2-methyl-1,4-dihydro-1,6-naphthyridin-5-yl ethansulfonat

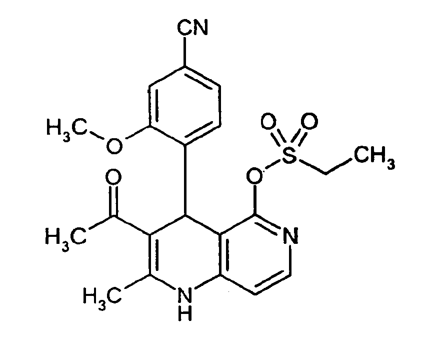

Die Titelverbindung wird analog zu Beispiel 10 ausgehend von 5.05 g (15.05 mmol) 4-(3-Acetyl-2-methyl-5-oxo-1,4,5,6-tetrahydro-1,6-naphthyridin-4-yl)-3-methoxybenzonitril hergestellt. Man erhält 4.97 g (77% d. Th.) des Produkts als hellgelben Feststoff.
LC-MS (Methode 3): Rₜ = 2.03 min; [M+H]⁺ = 428
¹H-NMR (300 MHz, DMSO-d₆): δ = 1.28 (t, 3H), 2.15 (s, 3H), 2.28 (s, 3H), 3.70 (m, 2H), 3.79 (s, 3H), 5.60 (s, 1H), 6.87 (d, 1H), 7.20 (d, 1H), 7.29 (dd, 1H), 7.40 (d, 1H), 7.94 (d, 1H), 9.80 (s, 1H).

### Beispiel 17

### ent-3-Acetyl-4-(4-cyano-2-methoxyphenyl)-2-methyl-1,4-dihydro-1,6-naphthyridin-5-yl ethansulfonat (Enantiomer A und Enantiomer B)

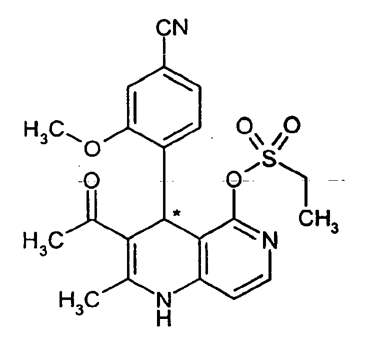

1.8 g des Racemats aus Beispiel 16 werden durch HPLC an chiraler Phase in die Enantiomere getrennt (Säule: 670 mm x 40 mm; Kieselgel-Phase basierend auf dem chiralen Selektor Poly(*N-*methacryloyl-D-leucin-*tert.*-butylamid; Eluent: Isohexan/Essigsäureethylester 1:1; Temperatur: 24°C; Fluss: 80 ml/min; UV-Detektion: 270 nm).

### Enantiomer A:

Ausbeute: 870 mg

HPLC (Methode 9): Rₜ = 3.12 min.

### Enantiomer B:

Ausbeute: 857 mg
HPLC (Methode 9): Rₜ = 4.88 min.

### Beispiel 18

### 3-Acetyl-2-methyl-4-(2-methyl-4-oxo-4H-chromen-8-yl)-1,4-dihydro-1,6-naphthyridin-5-yl trifluormethansulfonat

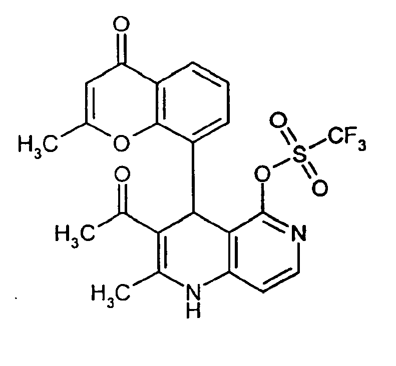

134 mg (0.37 mmol) 3-Acetyl-2-methyl-4-(2-methyl-4-oxo-4*H*-chromen-8-yl)-4,6-dihydro-1,6-naphthyridin-5(1*H*)-on werden in 5 ml Pyridin vorgelegt, mit 80 µl (0.75 mmol) Trifluormethansulfonsäureanhydrid versetzt und 1 h bei Raumtemperatur gerührt. Der Ansatz wird danach eingeengt und der Rückstand mittels präparativer HPLC gereinigt (Eluent: Acetonitril/Wasser mit 0.1% Ameisensäure, Gradient 20:80 → 95:5). Es werden 129 mg (70% d. Th.) der Titelverbindung erhalten.
LC-MS (Methode 4): Rₜ = 2.40 min; MS (EIpos): m/z = 495 [M+H]⁺
¹H-NMR (300 MHz, DMSO-d₆): δ = 2.22 (s, 3H), 2.25 (s, 3H), 2.37 (s, 3H), 5.75 (s, 1H), 6.20 (s, 1H), 7.03 (d, 1H), 7.36 (t, 1H), 7.64 (dd, 1H), 7.85 (dd, 1H), 8.00 (d, 1H), 10.13 (s, 1H).

### Beispiel 19

### 3-Acetyl-4-(4-cyano-2-methoxyphenyl)-2-methyl-1,4-dihydro-1,6-naphthyridin-5-yl trifluormethansulfonat

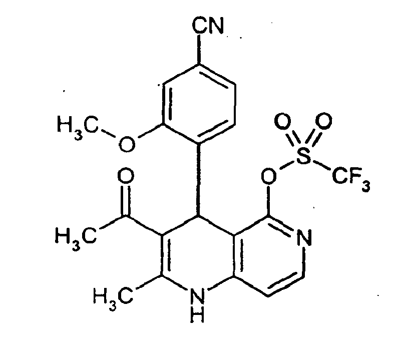

Die Titelverbindung wird analog zu Beispiel 18 aus 75 mg (0.224 mmol) 4-(3-Acetyl-2-methyl-5-oxo-1,4,5,6-tetrahydro-1,6-naphthyridin-4-yl)-3-methoxybenzonitril hergestellt. Man erhält 61 mg (58% d. Th.) des Produkts als hellgelben Feststoff.
LC-MS (Methode 3): Rₜ = 2.47 min; [M+H]⁺ = 468
¹H-NMR (300 MHz, DMSO-d₆): δ = 2.17 (s, 3H), 2.31 (s, 3H), 3.80 (s, 3H), 5.60 (s, 1H), 7.00 (d, 1H), 7.23 (d, 1H), 7.32 (dd, 1H), 7.44 (d, 1H), 7.99 (d, 1H), 10.02 (s, 1H).

### Beispiel 20

### 3-Acetyl-4-(4-cyanophenyl)-2-methyl-1,4-dihydro-1,6-naphthyridin-5-yl trifluormethansulfonat

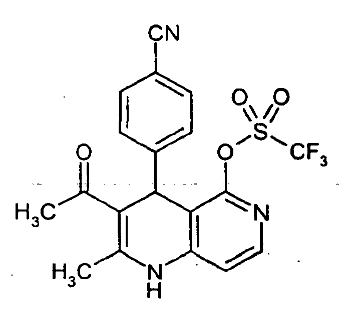

Die Titelverbindung wird analog zu Beispiel 18 aus 107 mg (0.35 mmol) 4-(3-Acetyl-2-methyl-5-oxo-1,4,5,6-tetrahydro-1,6-naphthyridin-4-yl)-benzonitril hergestellt. Man erhält 120 mg (58% d. Th.) des Produkts als weißen Feststoff.
LC-MS (Methode 3): Rₜ = 2.42 min; [M+H]⁺ = 438
¹H-NMR (300 MHz, DMSO-d₆): δ = 2.25 (s, 3H), 2.42 (s, 3H), 5.31 (s, 1H), 7.07 (d, 1H), 7.35 (d, 2H), 7.75 (d, 2H), 8.05 (d, 1H), 10.14 (s, 1H).

### Beispiel 21

### 4-(3-Acetyl-5-ethoxy-2-methyl-1,4-dihydro-1,6-naphthyridin-4-yl)-3-(trifluormethoxy)-benzonitril

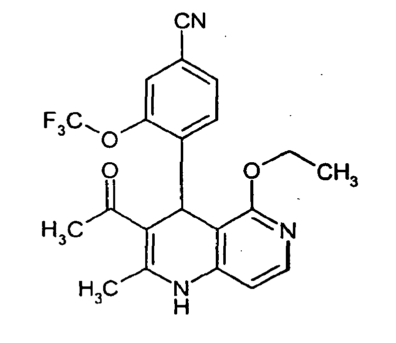

2.40 g (6.16 mmol) 4-(3-Acetyl-2-methyl-5-oxo-1,4,5,6-tetrahydro-1,6-naphthyridin-4-yl)-3-(trifluormethoxy)-benzonitril (Beispiel 10A) werden in 5 ml abs. THF suspendiert, mit 6.78 ml (6.78 mmol) *N'''*-(*tert.*-Butyl)-*N,N',N''*-tris[tris(dimethylamino)phosphoranyliden]phosphorimid-triamid (Phosphazen-Base P₄-t-Bu; 1 M in Hexan) versetzt und anschließend für 5 min gerührt. Dann werden 1.32 g (7.40 mmol) Ethyltrifluormethansulfonat hinzugefügt. Nach weiteren 5 min Rühren wird mit gesättigter Natriumhydrogencarbonat-Lösung hydrolysiert und zweimal mit Essigsäureethylester extrahiert. Die vereinigten organischen Phasen werden mit gesättigter Natriumchlorid-Lösung gewaschen, mit Natriumsulfat getrocknet und anschließend am Rotationsverdampfer eingeengt. Der Rückstand wird säulenchromatographisch aufgereinigt (Kieselgel, Laufmittel: Cyclohexan/Essigsäureethylester 3:7). Umkristallisation aus Acetonitril liefert schließlich 745 mg (29% d. Th.) der Titelverbindung.
LC-MS (Methode 1): Rₜ = 2.18 min; MS (Elpos): m/z = 418 [M+H]⁺
¹H-NMR (300 MHz, DMSO-d₆): δ = 1.13 (t, 3H), 2.17 (s, 3H), 2.34 (s, 3H), 4.12 (m, 2H), 5.44 (s, 1H), 6.53 (d, 1H), 7.55 (d, 1H), 7.65-7.71 (m, 2H), 7.78 (d, 1H), 9.58 (s, 1H).

### Beispiel 22

### ent-4-(3-Acetyl-5-ethoxy-2-methyl-1,4-dihydro-1,6-naphthyridin-4-yl)-3-(trifluormethoxy)-benzonitril (Enantiomer A und Enantiomer B)

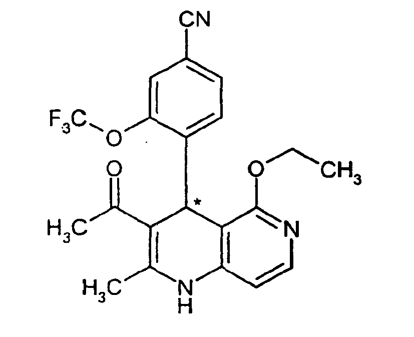

700 mg des Racemats aus Beispiel 21 werden mittels HPLC an chiraler Phase in die Enantiomere getrennt (Säule: 670 mm x 40 mm; Kieselgel-Phase basierend auf dem chiralen Selektor Poly(N-methacryloyl-D-leucin-*tert.*-butylamid; Eluent: Isohexan/Essigsäureethylester 1:1; Temperatur. 24°C; Fluss: 80 ml/min; UV-Detektion: 270 nm).

### Enantiomer A:

Ausbeute: 276 mg
HPLC (Methode 9): Rₜ = 2.18 min.

### Enantiomer B:

Ausbeute: 283 mg .
HPLC (Methode 9): Rₜ = 3.41 min.

### Beispiel 23

### 3-Cyano-2-methyl-4-(2-methyl-4-oxo-4H-chromen-8-yl)-1,4-dihydro-1,6-naphthyridin-5-yl trifluormethansulfonat

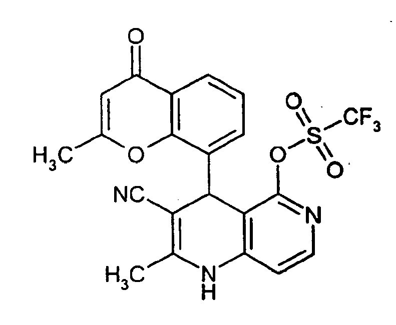

100 mg (0.290 mmol) 2-Methyl-4-(2-methyl-4-oxo-4*H*-chromen-8-yl)-5-oxo-1,4,5,6-tetrahydro-1,6-naphthyridin-3-carbonitril werden in 5 ml Pyridin vorgelegt, mit 163 mg (0.579 mmol) Trifluormethansulfonsäureanhydrid versetzt und 1 h bei Raumtemperatur gerührt. Der Ansatz wird eingeengt und der Rückstand mittels präparativer HPLC gereinigt (Eluent: Acetonitril/Wasser mit 0.1% Ameisensäure, Gradient 20:80 → 95:5). Es werden 80 mg (58% d. Th.) der Titelverbindung erhalten.
LC-MS (Methode 4): Rₜ = 2.42 min; MS (EIpos): m/z = 478 [M+H]⁺
¹H-NMR (300 MHz, DMSO-d₆): δ = 2.14 (s, 3H), 2.29 (s, 3H), 5.50 (s, 1H), 6.25 (s, 1H), 6.99 (d, 1H), 7.45 (t, 1H), 7.57 (dd, 1H), 7.95 (dd, 1H), 8.07 (d, 1H), 10.44 (s, 1H).

### Beispiel 24

### 4-(3-Acetyl-5-ethoxy-2-methyl-1,4-dihydro-1,6-naphthyridin-4-yl)-3-ethylbenzonitril

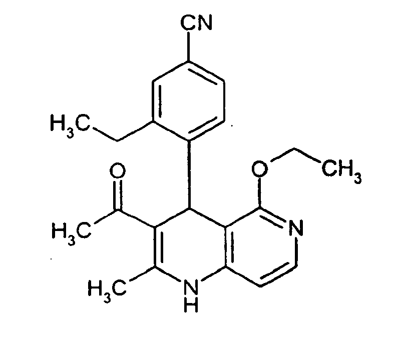

275 mg (0.82 mmol) 4-(3-Acetyl-2-methyl-5-oxo-1,4,5,6-tetrahydro-1,6-naphthyridin-4-yl)-3-ethylbenzonitril werden unter Argonatmosphäre in Dichlormethan (10 ml) suspendiert, mit 313 mg (1.65 mmol) Triethyloxoniumtetrafluoroborat versetzt und 12 h lang bei RT gerührt. Die Reaktionsmischung wird dann mit Methanol versetzt und eingeengt. Man reinigt den Rückstand durch präparative HPLC und erhält so 152 mg (50% d. Th.) der Titelverbindung als weiBen Feststoff.
LC-MS (Methode 4): Rₜ = 2.33 min; [M+H]⁺ = 362
¹H-NMR (300 MHz, CDCl₃): δ = 1.15 (t, 3H), 1.27 (t, 3H), 2.19 (s, 3H), 2.37 (s, 3H), 3.13 (m, 1H), 3.30 (m, 1H), 4.16 (m, 2H), 5.38 (s, 1H), 6.56 (d, 1H), 7.28 (d, 1H), 7.45 (dd, 1H), 7.51 (d, 1H), 7.61 (d, 1H), 9.50 (s, 1H).

### Beispiel 25

### 4-(4-Cyano-2-methoxyphenyl)-5-ethoxy-2-methyl-1,4-dihydro-1,6-naphthyridin-3-carbonitril

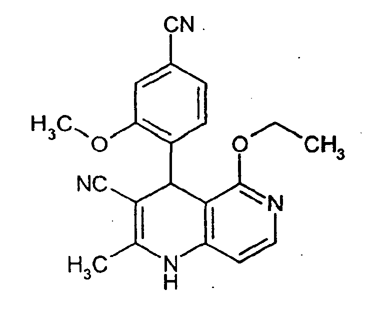

505 mg (1.58 mmol) 4-(4-Cyano-2-methoxyphenyl)-2-methyl-5-oxo-1,4,5,6-tetrahydro-1,6-naphthyridin-3-carbonitril werden in THF (20 ml) suspendiert und mit 1.9 ml (1.9 mmol) *N'''*-(*tert-*Butyl)-*N,N',N''*-tris[tris(dimethylamino)phosphoranyliden]phosphorimid-triamid (Phosphazen-Base P₄-t-Bu; 1 M in Hexan) versetzt. Zu der dunkelroten Lösung werden anschließend 240 µl (1.9 mmol) Ethyl-trifluormethansulfonat gegeben. Die entfärbte Lösung wird 10 min weiter gerührt, dann mit Methanol versetzt und eingeengt. Man reinigt den Rückstand mittels präparativer HPLC und erhält 124 mg (21% d. Th.) der Titelverbindung als weißen Feststoff.
LC-MS (Methode 1): Rₜ = 1.98 min; [M+H]⁺ = 347
¹H-NMR (300 MHz, DMSO-d₆): δ = 0.96 (t, 3H), 2.05 (s, 3H), 3.83 (s, 3H), 4.02 (m, 2H), 5.17 (s, 1H), 6.47 (d, 1H), 7.08 (d, 1H), 7.33 (dd, 1H), 7.47 (d, 1H), 7.80 (d, 1H), 9.72 (s, 1H).

### Beispiel 26

### ent-4-(4-Cyano-2-methoxyphenyl)-5-ethoxy-2-methyl-1,4-dihydro-1,6-naphthyridin-3-carbonitril (Enantiomer A und Enantiomer B)

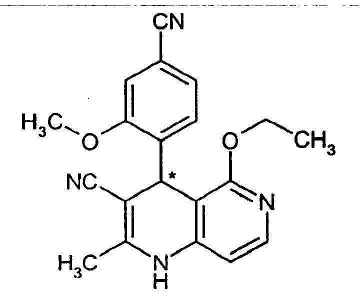

1 g des Racemats aus Beispiel 25 werden mittels HPLC an chiraler Phase in die Enantiomere getrennt (Säule: 630 mm x 30 mm; Kieselgel-Phase basierend auf dem chiralen Selektor Poly(*N-*methacryloyl-D-leucin-*tert.*-butylamid; Eluent: Isohexan/Essigsäureethylester 1:1; Temperatur: 24°C; Fluss: 25 ml/min; UV-Detektion: 260 nm).

### Enantiomer A:

Ausbeute: 427 mg
HPLC (Methode 9): Rₜ = 5.72 min.

### Enantiomer B:

Ausbeute: 494 mg
HPLC (Methode 9): Rₜ = 7.32 min.

### Beispiel 27

### 1-(5-Ethoxy-2-methyl-4-[4-nitro-2-(trifluormethyl)phenyl]-1,4-dihydro-1,6-naphthyridin-3-yl)-ethanon

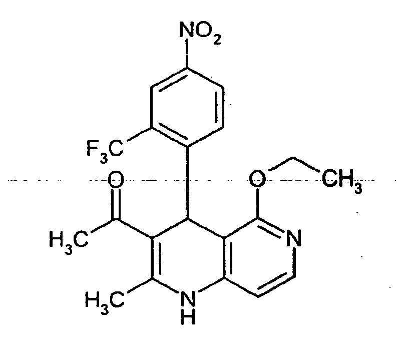

976 mg (2.48 mmol) 3-Acetyl-2-methyl-4-[4-nitro-2-(trifluormethyl)phenyl]-4,6-dihydro-1,6-naphthyridin-5(1*H*)-on werden in THF (60 ml) suspendiert und mit 3 ml (3 mmol) *N"'*-(*tert*.-Butyl)-*N,N',N*"-tris[tris(dimethylamino)phosphoranyliden]phosphorimid-triamid (Phosphazen-Base P₄-t-Bu; 1 M in Hexan) versetzt. Zu der dunkelroten Lösung werden anschließend 389 µl (2.97 mmol) Ethyl-trifluormethansulfonat gegeben. Die entfärbte Lösung wird 10 min weiter gerührt, dann mit Methanol versetzt und eingeengt. Man reinigt den Rückstand mittels präparativer HPLC und erhält 240 mg (23% d. Th.) der Titelverbindung als weißen Feststoff.
LC-MS (Methode 4): Rₜ = 2.47 min; [M+H]⁺ = 422
¹H-NMR (300 MHz, DMSO-d₆): δ = 1.07 (t, 3H), 2.20 (s, 3H), 2.37 (s, 3H), 4.15 (m, 2H), 5.74 (s, 1H), 6.57 (d, 1H), 7.59 (d, 1H), 7.80 (d, 1H), 8.23 (d, 1H), 8.28 (dd, 1H), 9.57 (s, 1H).

### Beispiel 28

### 4-(3-Acetyl-5-ethoxy-2-methyl-1,4-dihydro-1,6-naphthyridin-4-yl)-3-fluorbenzonitril

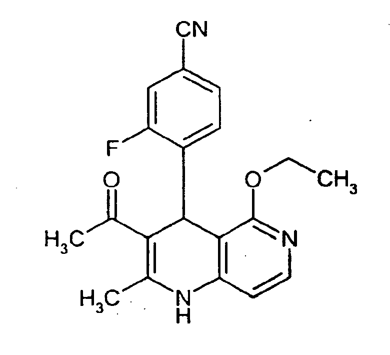

18 mg (0.056 mmol) 4-(3-Acetyl-2-methyl-5-oxo-1,4,5,6-tetrahydro-1,6-naphthyridin-4-yl)-3-fluorbenzonitril werden unter Argonatmosphäre in 5 ml abs. Dichlormethan gelöst und mit 21.2 mg (0.111 mmol) Triethyloxoniumtetrafluoroborat versetzt. Nach einer zweistündigen Reaktionszeit bei Raumtemperatur (Reaktionskontrolle mittels HPLC) wird der Ansatz mit 5 ml Methanol sowie 0.5 ml Wasser versetzt und nochmals für 2 h gerührt. Danach wird mit 10 ml Wasser verdünnt und dreimal mit Dichlormethan extrahiert. Die vereinigten organischen Phasen werden mit Natriumsulfat getrocknet und das Lösungsmittel am Rotationsverdampfer entfernt. Der Rückstand wird per Säulenchromatographie gereinigt (Kieselgel, Laufmittel: Cyclohexan/Ethylacetat 3:7). Es werden 7 mg (35.4% d. Th.) der Titelverbindung erhalten.
LC-MS (Methode 3): Rₜ = 2.10 min; [M+H]⁺ (EIpos): m/z = 352
¹H-NMR (300 MHz, DMSO-d₆): δ = 1.17 (t, 3H), 2.15 (s, 3H), 2.36 (s, 3H), 4.13 (m, 2H), 5.40 (s, 1H), 6.52 (d, 1H), 7.38 (t, 1H), 7.53 (dd, 1H), 7.68 (dd, 1H), 7.77 (d, 1H), 9.54 (s, 1H).

### Beispiel 29

### 4-(3-Acetyl-5-ethoxy-2-methyl-1,4-dihydro-1,6-naphthyridin-4-yl)-3-chlorbenzonitril

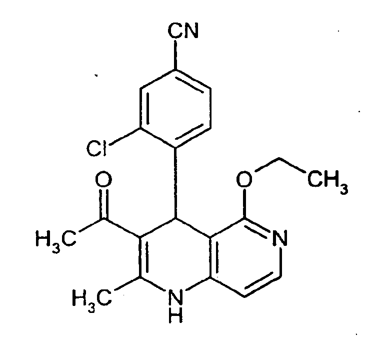

245 mg (0.721 mmol) 4-(3-Acetyl-2-methyl-5-oxo-1,4,5,6-tetrahydro-1,6-naphthyridin-4-yl)-3-chlorbenzonitril werden unter Argonatmosphäre in 15 ml abs. Dichlormethan gelöst und mit 273 mg (1.442 mmol) Triethyloxoniumtetrafluoroborat versetzt. Nach einer zweistündigen Reaktionszeit bei Raumtemperatur (Reaktionskontrolle mittels HPLC) wird der Ansatz mit 5 ml Methanol sowie 0.5 ml Wasser versetzt und nochmals für 2 h gerührt. Danach wird mit 20 ml Wasser verdünnt und dreimal mit Dichlormethan extrahiert. Die vereinigten organischen Phasen werden mit Natriumsulfat getrocknet und das Lösungsmittel am Rotationsverdampfer entfernt. Der Rückstand wird mittels präparativer HPLC gereinigt (Eluent: Acetonitril/Wasser mit 0.1% Ameisensäure, Gradient 20:80 → 95:5). Es werden 127 mg (47.9% d. Th.) der Titelverbindung erhalten.
LC-MS (Methode 3): Rₜ = 2.20 min; [M+H]⁺ (EIpos): m/z = 368
¹H-NMR (300 MHz, DMSO-d₆): δ = 1.17 (t, 3H), 2.17 (s, 3H), 2.34 (s, 3H), 4.16 (m, 2H), 5.52 (s, 1H), 6.54 (d, 1H), 7.44 (d, 1H), 7.66 (dd, 1H), 7.78 (d, 1H), 7.87 (d, 1H), 9.57 (s, 1H).

### Beispiel 30

### 4-(3-Acetyl-5-ethoxy-2-methyl-1,4-dihydro-1,6-naphthyridin-4-yl)-3-methyltbenzonitril

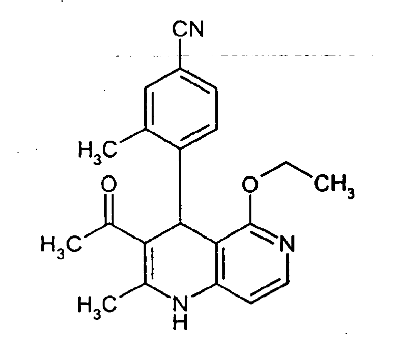

150 mg (0.470 mmol) 4-(3-A cetyl-2-methyl-5-oxo-1,4,5,6-tetrahydro-1,6-naphthyridin-4-yl)-3-methylbenzonitril werden unter Argonatmosphäre in 10 ml abs. Dichlormethan gelöst und mit 178 mg (0.939 mmol) Triethyloxoniumtetrafluoroborat versetzt. Nach einer zweistündigen Reaktionszeit bei Raumtemperatur (Reaktionskontrolle mittels HPLC) wird der Ansatz mit 5 ml Methanol sowie 0.5 ml Wasser versetzt und nochmals für 2 h gerührt. Danach wird mit 20 ml Wasser verdünnt und dreimal mit Dichlormethan extrahiert. Die vereinigten organischen Phasen werden mit Natriumsulfat getrocknet und das Lösungsmittel am Rotationsverdampfer entfernt. Der Rückstand wird mittels präparativer HPLC gereinigt (Eluent: Acetonitril/Wasser mit 0.1% Ameisensäure, Gradient 20:80 → 95:5). Es werden 110 mg (67.4% d. Th.) der Titelverbindung erhalten.
LC-MS (Methode 4): Rₜ = 2.20 min; [M+H]⁺ (EIpos): m/z = 348
¹H-NMR (300 MHz, DMSO-d₆): δ = 1.18 (t, 3H), 2.20 (s, 3H), 2.38 (s, 3H), 2.73 (s, 3H), 4.17 (m, 2H), 5.27 (s, 1H), 6.56 (d, 1H), 7.25 (d, 1H), 7.45 (dd, 1H), 7.49 (s, 1H), 7.77 (d, 1H), 9.50 (s, 1H).

### Beispiel 31

### 4-(3-Acetyl-5-ethoxy-2-methyl-1,4-dihydro-1,6-naphthyridin-4-yl)-3-(trifluormethyl)benzonitril

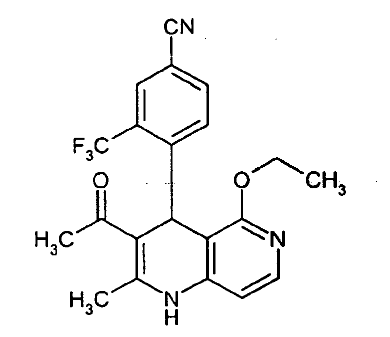

50 mg (0.134 mmol) 4-(3-Acetyl-2-methyl-5-oxo-1,4,5,6-tetrahydro-1,6-naphthyridin-4-yl)-3-(trifluormethyl)benzonitril werden unter Argonatmosphäre in 5 ml abs. Dichlormethan gelöst und mit 50.9 mg (0.268 mmol) Triethyloxoniumtetrafluoroborat versetzt. Nach einer zweistündigen Reaktionszeit bei Raumtemperatur (Reaktionskontrolle mittels HPLC) wird der Ansatz mit 5 ml Methanol sowie 0.5 ml Wasser versetzt und nochmals für 2 h gerührt. Danach wird mit 20 ml Wasser verdünnt und dreimal mit Dichlormethan extrahiert. Die vereinigten organischen Phasen werden mit Natriumsulfat getrocknet und das Lösungsmitteln am Rotationsverdampfer entfernt. Der Rückstand wird mittels präparativer HPLC gereinigt (Eluent: Acetonitril/Wasser mit 0.1% Ameisensäure, Gradient 20:80 → 95:5). Es werden 23 mg (42.8% d. Th.) der Titelverbindung erhalten.
LC-MS (Methode 1): Rₜ = 2.12 min; [M+H]⁺ (EIpos): m/z = 402
¹H-NMR (300 MHz, DMSO-d₆): δ = 1.06 (t, 3H), 2.19 (s, 3H), 2.35 (s, 3H), 4.15 (m, 2H), 5.69 (s, 1H), 6.56 (d, 1H), 7.48 (d, 1H), 7.80 (d, 1H), 7.90 (dd, 1H), 8.05 (d, 1H), 9.54 (s, 1H).

### Beispiel 32

### 4-(3-Acetyl-5-ethoxy-2-methyl-1,4-dihydro-1,6-naphthyridin-4-yl)-1-naphthonitril

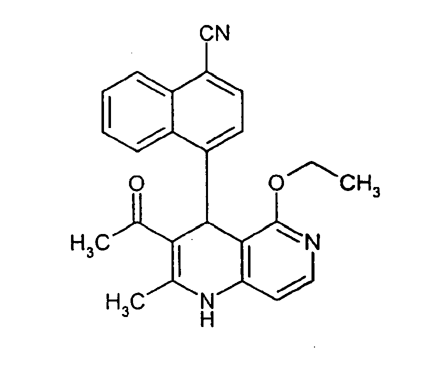

93 mg (0.262 mmol) 4-(3-Acetyl-2-methyl-5-oxo-1,4,5,6-tetrahydro-1,6-naphthyridin-4-yl)-1-naphthonitril werden unter Argonatmosphäre in 5 ml abs. Dichlormethan gelöst und mit 99.4 mg (0.268 mmol) Triethyloxoniumtetrafluoroborat versetzt. Nach einer zweistündigen Reaktionszeit bei Raumtemperatur (Reaktionskontrolle mittels HPLC) wird der Ansatz mit 5 ml Methanol sowie 0.5 ml Wasser versetzt und mochmals für 2 h gerührt.-Danach wird mit 20 ml Wasser verdünnt und dreimal mit Dichlormethan extrahiert. Die vereinigten organischen Phasen werden mit Natriumsulfat getrocknet und das Lösungsmittel am Rotationsverdampfer entfernt. Der Rückstand wird mittels präparativer HPLC gereinigt (Eluent: Acetonitril/Wasser mit 0.1% Ameisensäure, Gradient 20:80 → 95:5). Es werden 60 mg (59.8% d. Th.) der Titelverbindung erhalten.
LC-MS (Methode 4): Rₜ = 2.35 min; [M+H]⁺ (EIpos): m/z = 384
¹H-NMR (300 MHz, DMSO-d₆): δ = 0.87 (t, 3H), 2.10 (s, 3H), 2.43 (s, 3H), 3.88 (m, 1H), 4.02 (m, 1H), 6.05 (s, 1H), 6.59 (d, 1H), 7.49 (d, 1H), 7.74 (d, 1H), 7.78 (m, 2H), 8.01 (d, 1H), 8.06 (m, 1H), 8.05 (m, 1H), 9.59 (s, 1H).

### Beispiel 33

### 4-(4-Cyano-2-methoxyphenyl)-5-ethoxy-2-(trifluormethyl)-1,4-dihydro-1,6-naphthyridin-3-carbonitril

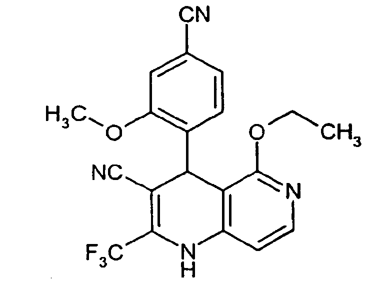

390 mg (1.05 mmol) 4-(4-Cyano-2-methoxyphenyl)-5-oxo-2-(trifluormethyl)-1,4,5,6-tetrahydro-1,6-naphthyridin-3-carbonitril und 398 mg Triethyloxoniumtetrafluoroborat (2.1 mmol) werden unter einer trockenen Argonatmosphäre vorgelegt. Nach Zugabe von Dichlormethan (32 ml) wird die Suspension bis zum Erhalt einer Lösung gerührt (ca. 2 h), dann mit Methanol versetzt und eingeengt. Man reinigt den Rückstand mittels präparativer HPLC und erhält 250 mg (59% d. Th.) der Titelverbindung als weißen Feststoff.
LC-MS (Methode 1): Rₜ = 2.29 min; [M+H]⁺ = 401 ¹H-NMR (300 MHz, DMSO-d₆): δ = 1.02 (t, 3H), 3.81 (s, 3H), 4.05 (m, 2H), 5.28 (s, 1H), 6.72 (d, 1H), 7.28 (d, 1H), 7.40 (dd, 1H), 7.52 (d, 1H), 7.87 (d, 1H), 10.48 (s, 1H).

### Beispiel 34

### 4-(5-Ethoxy-2-methyl-3-nitro-1,4-dihydro-1,6-naphthyridin-4-yl)-3-methoxybenzonitril

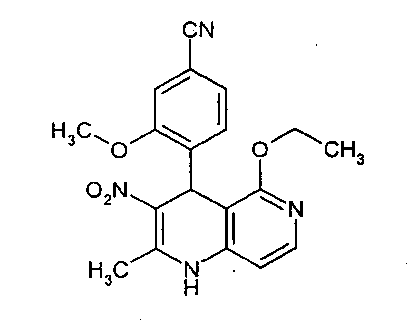

40 mg (0.11 mmol) 3-Methoxy-4-(2-methyl-3-nitro-5-oxo-1,4,5,6-tetrahydro-1,6-naphthyridin-4-yl)-benzonitril und 43 mg Triethyloxoniumtetrafluoroborat (0.23 mmol) werden unter einer trockenen Argonatmosphäre vorgelegt. Nach Zugabe von Dichlormethan (5 ml) wird die Suspension bis zum Erhalt einer Lösung gerührt, dann mit Methanol versetzt und eingeengt. Man reinigt den Rückstand mittels präparativer HPLC und erhält 13 mg (29% d. Th.) der Titelverbindung als weißen Feststoff.
LC-MS (Methode 1): Rₜ = 2.01 min; [M+H]⁺ = 367
¹H-NMR (300 MHz, DMSO-d₆): δ = 1.13 (t, 3H), 2.55 (s, 3H), 3.73 (s, 3H), 4.10 (m, 2H), 5.62 (s, 1H), 6.60 (d, 1H), 7.31 (dd, 1H), 7.37 (d, 1H), 7.40 (d, 1H), 7.85 (d, 1H), 10.36 (s, 1H).

### Beispiel 35

### 4-(4-Cyano-2-methoxyphenyl)-5-ethoxy-2,7-dimethyl-1,4-dihydro-1,6-naphthyridin-3-carbonitril

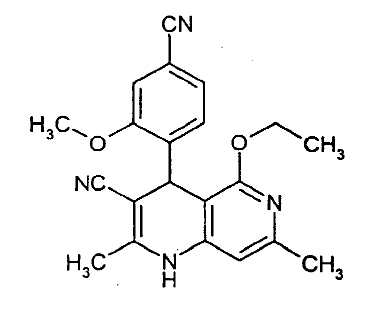

1920 mg (5.77 mmol) 4-(4-Cyano-2-methoxyphenyl)-2,7-dimethyl-5-oxo-1,4,5,6-tetrahydro-1,6-naphthyridin-3-carbonitril werden in 40 ml (240 mmol) wasserfreiem Orthoameisensäuretriethylester suspendiert. Das Reaktionsgemisch wird auf 130°C erhitzt. Anschließend werden stündlich 10 Tropfen konzentrierter Schwefelsäure zugesetzt. Nachdem vollständige Umsetzung detektiert ist (analytische HPLC; Reaktionsdauer ca. 36 h), wird das Reaktionsgemisch auf Raumtemperatur abgekühlt und die flüchtigen Komponenten am Rotationsverdampfer entfernt. Das Rohprodukt wird mittels Säulenchromatographie aufgereinigt (Kieselgel; Laufmittel: Cyclohexan/Essigsäureethylester 1:1). Das so erhaltene Produkt wird aus Essigsäureethylester kristallisiert. Es werden 451 mg (22% d. Th.) der Titelverbindung als weißer Feststoff erhalten.
LC-MS (Methode 12): Rₜ = 3.42 min, [M+H]⁺ (EIpos): m/z = 361
¹H-NMR (300 MHz, DMSO-d₆): δ = 0.95 (t, 3H), 2.05 (s, 3H), 2.24 (s, 3H), 3.85 (s, 3H), 4.00 (m, 2H), 5.14 (s, 1H), 6.30 (s, 1H), 7.06 (d, 1H), 7.33 (d, 1H), 7.47 (s, 1H), 9.63 (s, 1H).

### Beispiel 36

### ent-4-(4-Cyano-2-methoxyphenyl)-5-ethoxy-2,7-dimethyl-1,4-dihydro-1,6-naphthyridin-3-carbonitril [(-)-Enantiomer und (+)-Enantiomer]

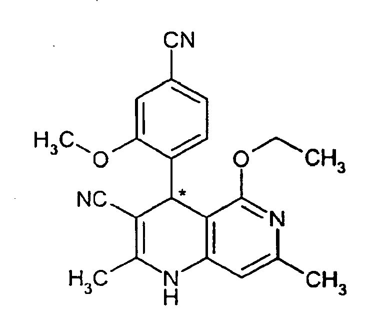

Das Racemat aus Beispiel 35 kann in präparativem Maßstab durch HPLC an chiraler Phase in seine Enantiomere aufgetrennt werden [Säule: 680 mm x 40 mm; Kieselgel-Phase basierend auf dem chiralen Selektor Poly(*N-*methacryloyl-D-leucin-dicyclopropylmethylamid; Eluent: Essigsäureethylester/Isohexan 2:1 (v/v); Temperatur: 24°C; Fluss: 80 ml/min; UV-Detektion: 220 nm].

### (-)-Enantiomer:

HPLC: Rₜ = 3.1 min, ee = 99.5% [Säule: 250 mm x 4.6 mm; Kieselgel-Phase basierend auf dem chiralen Selektor Poly(*N-*methacryloyl-D-leucin-dicyclopropylmethylamid; Eluent: Essigsäureethylester/Isohexan 2:3 (v/v); Temperatur: 25°C; Fluss: 2 ml/min; UV-Detektion: 220 nm];
spezifischer Drehwert (Chloroform, 589 nm, 19.9°C, c = 0.48500 g /100 ml): -466.4°.

### (+)-Enantiomer:

HPLC: Rₜ = 3.96 min, ee = 98% [Säule: 250 mm x 4.6 mm; Kieselgel-Phase basierend auf dem chiralen Selektor Poly(*N-*methacryloyl-D-leucin-dicyclopropylmethylamid; Eluent: Essigsäureethylester/Isohexan 2:3 (v/v); Temperatur: 25°C; Fluss: 2 ml/min; UV-Detektion: 220 nm];
spezifischer Drehwert (Chloroform, 589 nm, 20.1°C, c = 0.34000 g /100 ml): +465.1°.

### Beispiel 37

### 4-(4-Cyano-2-methoxyphenyl)-5-ethoxy-2,8-dimethyl-1,4-dihydro-1,6-naphthyridin-3-carbonitril

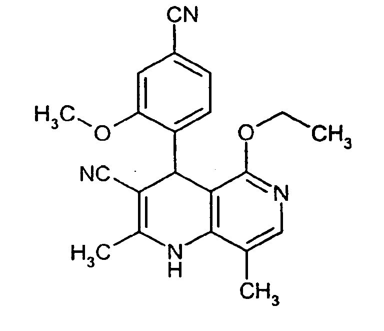

3620 mg (10.83 mmol) 4-(4-Cyano-2-methoxyphenyl)-2,8-dimethyl-5-oxo-1,4,5,6-tetrahydro-1,6-naphthyridin-3-carbonitril werden in 150 ml (901 mmol) wasserfreiem Orthoameisensäuretriethylester suspendiert. Das Reaktionsgemisch wird auf 140°C erhitzt. Anschließend werden stündlich 10 Tropfen konzentrierter Schwefelsäure zugesetzt. Nachdem vollständige Umsetzung detektiert ist (analytische HPLC; Reaktionsdauer ca. 36 h), wird das Reaktionsgemisch auf Raumtemperatur abgekühlt und das ausgefallene Produkt abfiltriert. Das Präzipitat wird mit Essigsäureethylester und Diethylether gewaschen und anschließend per Flash-Chromatographie aufgereinigt (Kieselgel; Laufmittel: zunächst Dichlormethan, dann Isohexan/Essigsäureethylester 1:1). Die Produktfraktionen werden vereinigt, am Rotationsverdampfer eingeengt und der Rückstand anschließend aus Essigsäureethylester kristallisiert. Nach dem Trocknen im Hochvakuum werden 1160 mg (30% d. Th.) der Titelverbindung in Form gelblicher Kristalle erhalten.
LC-MS (Methode 13): Rₜ = 3.21 min; [M+H]^{÷} (EIpos): m/z = 361
¹H-NMR (300 MHz, DMSO-d₆): δ = 0.95 (t, 3H), 2.14 (s, 3H), 2.15 (s, 3H), 3.85 (s, 3H), 3.99 (m, 2H), 5.19 (s, 1H), 7.06 (d, 1H), 7.33 (d, 1H), 7.46 (s, 1H), 7.67 (s, 1H), 8.66 (s, 1H).

### Beispiel 38

### ent-4-(4-Cyano-2-methoxyphenyl)-5-ethoxy-2,8-dimethyl-1,4-dihydro-1,6-naphthyridin-3-carbonitril [(-)-Enantiomer und (+)-Enantiomer]

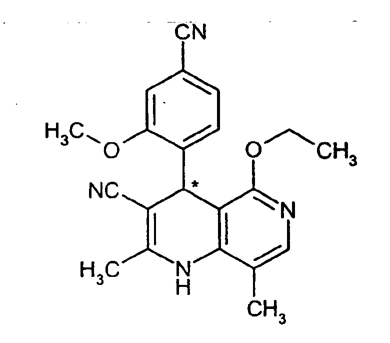

Das Racemat aus Beispiel 37 kann in präparativem Maßstab durch HPLC an chiraler Phase in seine Enantiomere aufgetrennt werden [Säule: 670 mm x 40 mm; Kieselgel-Phase basierend auf dem chiralen Selektor Poly(*N-*methacryloyl-L-leucin-*tert*.-butylamid; Eluent: Essigsäureethylester/Isohexan 7:3 (v/v); Temperatur: 24°C; Fluss: 80 ml/min; UV-Detektion: 220 nm].

### (-)-Enantiomer:

HPLC: Rₜ = 3.80 min, ee >99.8% [Säule: 250 mm x 4.6 mm; Kieselgel-Phase basierend auf dem chiralen Selektor Poly(*N-*methacryloyl-L-leucin-*tert*.-butylamid; Eluent: Essigsäureethylester/Isohexan 1:1 (v/v); Temperatur: 25°C; Fluss: 1 ml/min; UV-Detektion: 220 nm];
spezifischer Drehwert (Chloroform, 589 nm, 19.6°C, c = 0.28000 g / 100 ml): -387.0°.

### (+)-Enantiomer:

HPLC: Rₜ = 4.46 min, ee = 99.6% [Säule: 250 mm x 4.6 mm; Kieselgel-Phase basierend auf dem chiralen Selektor Poly(*N-*methacryloyl-L-leucin-*tert*.-butylamid; Eluent: Essigsäureethylester/Isohexan 1:1 (v/v); Temperatur: 25°C; Fluss: 1 ml/min; UV-Detektion: 220 nm];
spezifischer Drehwert (Chloroform, 589 nm, 20.1°C, c = 0.39500 g / 100 ml): +397.1°.

### B. Bewertung der pharmakologischen Wirksamkeit

### Abkürzungen:

- DMEM: Dulbecco's Modified Eagle Medium
- DNA: Desoxyribo Nucleic Acid
- FCS: Fetal Calf Serum
- HEPES: 4-(2-Hydroxyethyl)-1-piperazin-ethansulfonsäure
- PCR: Polymerase Chain Reaction
- Tris: Tris-(hydroxymethyl)-methylamin

Die vorteilhaften pharmakologischen Eigenschaften der erfindungsgemäßen Verbindungen können in folgenden Assays gezeigt werden:

### 1. Zellulärer in vitro-Test zur Bestimmung der inhibitorischen MR-Aktivität und MR-Selektivität gegenüber anderen Steroidhormon-Rezeptoren

Die Identifizierung von Antagonisten des humanen Mineralokorticoid-Rezeptors (MR) sowie die Quantifizierung der Wirksamkeit der hier beschriebenen Verbindungen erfolgt mit Hilfe einer rekombinanten Zelllinie. Die Zelle leitet sich ursprünglich von einer Ovarepithelzelle des Hamsters ab (Chinese Hamster Ovary, CHO K1, ATCC: American Type Culture Collection, VA 20108, USA).

In dieser CHO K1-Zelllinie wird ein etabliertes Chimärensystem verwendet, in dem die Liganden-Bindungsdomänen humaner Steroidhormon-Rezeptoren an die DNA-Bindungsdomäne des Hefe-Transkriptionsfaktors GAL4 fusioniert werden. Die so entstehenden GAL4-Steroidhormonrezeptor-Chimären werden in den CHO-Zellen mit einem Reporterkonstrukt co-transfiziert und stabil exprimiert.

### Klonierungen:

Zur Generierung der GAL4-Steroidhormonrezeptor-Chimären wird die GAL4-DNA-Bindungsdomäne (Aminosäuren 1-147) aus dem Vektor pFC2-dbd (Fa. Stratagene) mit den PCR-amplifizierten Liganden-Bindungsdomänen des Mineralokorticoid-Rezeptors (MR, Aminosäuren 734-985), des Glucokorticoid-Rezeptors (GR, Aminosäuren 443-777), des Progesteron-Rezeptors (PR, Aminosäuren 680-933) und des Androgen-Rezeptors (AR, Aminosäuren 667-919) in den Vektor pIRES2 (Fa. Clontech) kloniert. Das Reporterkonstrukt, welches fünf Kopien der GAL4-Bindestelle, vorgeschaltet vor einem Thymidinkinase-Promotor enthält, führt zur Expression der Firefly-Luciferase (*Photinus pyralis*) nach Aktivierung und Bindung der GAL4-Steroidhormonrezeptor-Chimären durch die jeweiligen spezifischen Agonisten Aldosteron (MR), Dexamethason (GR), Progesteron (PR) und Dihydrotestosteron (AR).

### Testablauf:

Die MR-, GR-, PR- und AR-Zellen werden am Tag vor dem Test in Medium (Optimem, 2.5% FCS, 2 mM Glutamin, 10 mM HEPES) in 96- (oder 384- bzw. 1536-) Loch-Mikrotiterplatten ausplattiert und in einem Zellinkubator (96% Luftfeuchtigkeit, 5% v/v CO₂, 37°C) gehalten. Am Testtag werden die zu prüfenden Substanzen in oben genanntem Medium aufgenommen und zu den Zellen hinzugegeben. Etwa 10 bis 30 Minuten nach Zugabe der Testsubstanzen werden die jeweiligen spezifischen Agonisten der Steroidhormon-Rezeptoren hinzugesetzt. Nach einer weiteren Inkubationszeit von 5 bis 6 Stunden wird die Luciferaseaktivität mit Hilfe einer Videokamera gemessen. Die gemessenen relativen Lichteinheiten ergeben in Abhängigkeit von der Substanzkonzentration eine sigmoide Stimulationskurve. Die Berechnung der IC₅₀-Werte erfolgt mit Hilfe des Computerprogramms GraphPad PRISM (Version 3.02).

Tabelle A zeigt die IC₅₀-Werte (MR) repräsentativer Beispielverbindungen:

**Tabelle A**

| **Beispiel-Nr.** | **MR IC₅₀ [nM]** |
|---|---|
| 4 | 273 |
| 10 | 75 |
| 21 | 30 |
| 24 | 31 |
| 25 | 87 |
| 32 | 4 |
| 33 | 35 |
| 35 | 122 |
| 36 | 55 |
| [(-)-Enantiomer] | |
| 37 | 73 |
| 38 | 25 |
| [(-)-Enantiomer) | |

### 2. In vitro-Test zur Bestimmung möglicher Bindungsaktivität am L-Typ Calcium-Kanal

Membranpräparationen des zerebralen Cortex von Wistar-Ratten dienen als Ausgangsmaterial für einen radioaktiven Bindungstest, der als Standardassay in der Literatur ausführlich beschrieben ist [Ehlert, F.J., Roeske, W.R., Itoga E., Yamamura, H.I., Life Sci. 30, 2191-2202 (1982); Gould, R.J., Murphy, K.M.M., Snyder, S.H., Proc. Natl. Acad. Sci. U.S.A. 79, 3656-3660] und von kommerziellen Anbietern (z.B. Fa. MDS Pharma Services) im Rahmen von Auftragsuntersuchungen verwendet wird. In diesem Bindungsassay werden Verdünnungsreihen der Testverbindungen in DMSO typischerweise für 90 Minuten bei 25°C in einem 50 mM TrisHCl-Puffer, pH 7.7, mit den Membranpräparationen und dem Tritium-markierten Liganden Nitrendipin (0.1 nM) inkubiert und die spezifische Bindung der Testverbindungen über Quantifizierung des spezifisch verdrängten, radioaktiv markierten Liganden bestimmt. IC₅₀-Werte werden über eine nicht-lineare Regressionsanalyse ermittelt.

In diesem L-Typ Calcium-Kanal-Bindungsassay wird für einen klassischen Calcium-Antagonisten vom Dihydropyridin-Typ, wie z.B. Nitrendipin, ein IC₅₀-Wert von 0.3 nM bestimmt, während untersuchte Beispiele der hier beschriebenen erfindungsgemäßen Verbindungen IC₅₀-Werte von > 1 µM und somit eine mindestens um den Faktor 3000 verminderte Affinität zum L-Typ Calcium-Kanal aufweisen. Verbindungen mit einer solchen geringen Residualbindungsaffinität zum L-Typ Calcium-Kanal zeigen *in vivo* keine ausgeprägten hämodynamischen Effekte mehr, die über den L-Typ Calcium-Kanal vermittelt sind.

### 3. In vitro-Test zur funktionellen Charakterisierung möglicher Calcium-Kanalagonistischer oder -antagonistischer Wirkungen von Testverbindungen: Kaliumchlorid-induzierte Stimulation der isolierten Kaninchenaorta

Die frisch isolierte Thoraxaorta von männlichen New Zeeland White-Kaninchen wird entfernt und vom umgebenden Gewebe gereinigt. Dann werden Aortenringe von 2 mm Länge unter einer Anfangsspannung von 4 g in 10 ml-Organbäder mit auf 37°C erwärmter Krebs-Henseleit-Lösung gebracht. Kontraktionen von 40 mM KCl (submaximale Kontraktion) und 15 mM KCl (minimale Kontraktion) werden viermal im Abstand von 45 Minuten ausgelöst, um die Gefäße zu trainieren und eine stabile Ruhespannung zu erzeugen. Jeder Kontraktion folgt eine Serie von elf Spülzyklen und eine Ruhezeit von 30 Minuten bei vorheriger Nachspannung. Nach den vier Vorläufen erfolgt ohne weitere Nachspannung jeweils zu Beginn der Ruhephase die Zugabe der Testsubstanzen in die Organbäder. Die Konzentration der Testsubstanzen erhöht sich bei den vier folgenden Kontraktionen jeweils um den Faktor 10. Zur Wirkungsberechnung wird die Differenz zwischen der Basisspannung und dem vierten Vorlauf-Kontraktionswert als 100% gesetzt, die folgenden Kontraktionsspitzen beziehen sich auf diesen Wert. Diese Versuchsdurchführung ermöglicht die Differenzierung von Calcium-agonistischer (leichte Steigerung bei submaximaler Kontraktion, stärkere Steigerung bei minimaler Kontraktion) und Calcium-antagonistischer Substanzwirkung (Senkung bei submaximaler Kontraktion, stärkere Senkung bei minimaler Kontraktion).

In diesem funktionellen Assay am isolierten Organ wird für einen klassischen Calcium-Antagonisten vom Dihydropyridin-Typ, wie z.B. Nifedipin, ein IC₅₀-Wert von 0.1 nM bis 0.4 nM gemessen, während untersuchte Beispiele der hier beschriebenen erfindungsgemäßen Verbindungen IC₅₀-Werte von > 1 µM und somit eine mindestens um den Faktor 2500 verminderte Affinität zum L-Typ Calcium-Kanal aufweisen. Verbindungen mit einer solchen geringen Residualbindungsaffinität zum L-Typ Calcium-Kanal zeigen *in vivo* keine ausgeprägten hämodynamischen Effekte mehr, die über den L-Typ Calcium-Kanal vermittelt sind.

### 4. In vivo-Test zum Nachweis der kardiovaskulären Wirkung: Diurese-Untersuchungen an wachen Ratten in Stoffwechlselkäfigen

Wistar-Ratten (250-350 g Körpergewicht) werden mit freiem Zugang zu Futter (Altromin) und Trinkwasser gehalten. Ab ca. 72 Stunden vor Versuchsbeginn erhalten die Tiere anstelle des normalen Futters ausschließlich Kochsalz-reduziertes Futter mit einem Gehalt von 0.02% Natriumchlorid (ssniff R/M-H, 10 mm mit 0.02% Na, S0602-E081, Fa. ssniff Spezialdiäten Gmbh, D-59494 Soest). Während des Versuches werden die Tiere für ca. 24 Stunden einzeln in für Ratten dieser Gewichtsklasse geeigneten Stoffwechselkäfigen (Fa. Tecniplast Deutschland GmbH, D-82383 Hohenpeißenberg) mit freiem Zugang zu Kochsalz-reduziertem Futter und Trinkwasser gehalten. Am Versuchsbeginn wird den Tieren die zu prüfende Substanz in einem Volumen von 0.5 ml/kg Körpergewicht eines geeigneten Lösemittels mittels einer Schlundsonde in den Magen verabreicht. Als Kontrolle dienende Tiere erhalten nur Lösemittel. Kontrollen und Substanztestungen werden am selben Tag parallel durchgeführt. Kontrollgruppen und Substanz-Dosisgruppen bestehen aus jeweils 3 bis 6 Tieren. Während des Versuchs wird der von den Tieren ausgeschiedene Urin kontinuierlich in einem Auffangbehälter am Käfigboden gesammelt. Für jedes Tier wird gesondert das Urinvolumen pro Zeiteinheit bestimmt und die Konzentration der im Urin ausgeschicdenen Natrium- bzw. Kalium-Ionen mittels flammenphotometrischer Standardmethoden gemessen. Aus den Messwerten wird der Natrium/Kalium-Quotient als ein Maß für die Substanzwirkung berechnet. Die Messintervalle betragen typischerweise den Zeitraum bis zu 8 Stunden nach Versuchsbeginn (Tagintervall) und den Zeitraum von 8 bis 24 Stunden nach Versuchsbeginn (Nachtintervall). In einer abgewandelten Versuchsanordnung wird der Urin während des Tagintervalls im Abstand von zwei Stunden gesammelt und gemessen. Um eine hierfür ausreichende Urinmenge zu erhalten, wird den Tieren zu Versuchsbeginn und dann im Abstand von zwei Stunden per Schlundsonde eine definierte Menge Wasser zugeführt.

### 5. DOCA/Salz-Modell

Die Verabreichung von Desoxycorticosteronacetat (DOCA) in Kombination mit einer Hochsalzdiät und einseitiger Nierenentfernung induziert bei der Ratte einen Hypertonus, der durch relativ niedrige Reninspiegel charakterisiert ist. Als Folge dieser endokrinen Hypertonie (DOCA ist eine direkte Vorstufe von Aldosteron) kommt es in Abhängigkeit von der gewählten DOCA-Konzentration zu einer Hypertrophie des Herzens und weiteren Endorgan-Schäden, z.B. der Niere, die u.a. durch Proteinurie und Glomerulosklerose charakterisiert sind. In diesem Rattenmodell lassen sich somit Testsubstanzen auf vorhandene antihypertrophe und Endorgan-schützende Wirkung hin untersuchen.

Etwa 8 Wochen alte (Körpergewicht zwischen 250 und 300 Gramm), männliche Sprague Dawley (SD)-Ratten werden linksseitig uninephrektomiert. Dazu werden die Ratten mit 1.5-2%-igem Isofluran in einer Mischung aus 66% N₂O und 33% O₂ anästhesiert und die Niere über einen Flankenschnitt entfernt. Als spätere Kontrolltiere dienen sogenannte sham-operierte Tiere, denen keine Niere entfernt wird.

Uninephrektomierte SD-Ratten erhalten 1% Natriumchlorid im Trinkwasser und einmal wöchentlich eine subkutane Injektion von Desoxycorticosteronacetat (gelöst in Sesamöl; Fa. Sigma) zwischen die Schulterblätter gespritzt (Hochdosis: 100 mg/kg/Woche s.c.; Normaldosis: 30 mg/kg/Woche s.c.).

Die Substanzen, die auf ihre protektive Wirkung *in vivo* untersucht werden sollen, werden per Gavage oder über das Futter (Fa. Ssniff) verabreicht. Die Tiere werden einen Tag vor Versuchsbeginn randomisiert und Gruppen mit gleicher Tierzahl, in der Regel n = 10, zugeordnet. Während des gesamten Versuchs steht den Tieren Trinkwasser und Futter *ad libitum* zur Verfügung. Die Substanzen werden einmal täglich 4-8 Wochen lang per Gavage oder per Futter verabreicht. Als Plazebogruppe dienen Tiere, die genauso behandelt werden, aber entweder nur das Lösungsmittel oder das Futter ohne Testsubstanz erhalten.

Die Wirkung der Testsubstanzen wird durch Messung hämodynamischer Parameter [Blutdruck, Herzfrequenz, Inotropie (dp/dt), Relaxationszeit (tau), maximaler linksventrikulärer Druck, linksventrikulärer enddiastolischer Druck (LVEDP)], Gewichtsbestimmung von Herz, Niere und Lunge, Messung der Proteinausscheidung sowie durch Messung der Genexpression von Biomarkern (z.B. ANP, Atrial Natriuretic Peptide, und BNP, Brain Natriuretic Peptide) mittels RT/TaqMan-PCR nach RNA-Isolation aus kardialem Gewebe bestimmt.

Die statistische Auswertung erfolgt mit Student's t-Test nach vorheriger Überprüfung der Varianzen auf Homogenität.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 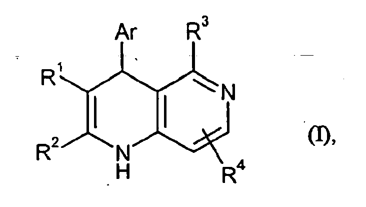 in welcher
Ar für eine Gruppe der Formel 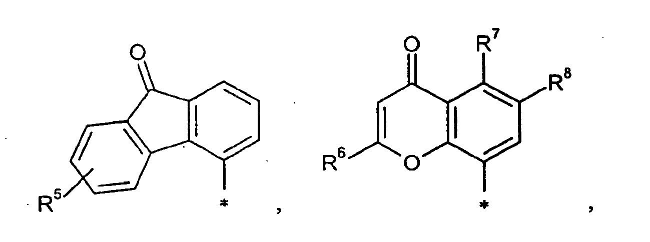 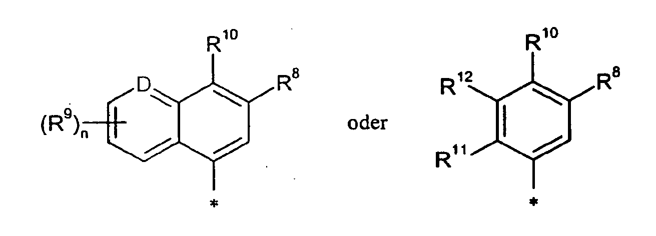
steht, worin
* die Verknüpfungsstelle bedeutet,
R⁵ Wasserstoff oder Halogen bedeutet,
R⁶ Methyl oder Ethyl bedeutet,
R⁷ Wasserstoff, Fluor, Chlor, Cyano, Nitro, Trifluormethyl oder (C₁-C₄)- Alkyl bedeutet,
R⁸ Wasserstoff oder Fluor bedeutet,
R⁹ Halogen, (C₁-C₄)-Alkyl, Trifluormethyl, (C₁-C₄)-Alkoxy oder Trifluor- methoxy bedeutet,
R¹⁰ Cyano oder Nitro bedeutet,
R¹¹ Wasserstoff, Halogen, (C₁-C₄)-Alkyl, (C₁-C₄)-Alkoxy, (C₁-C₄)-Alkylthio oder Di-(C₁-C₄)-alkylamino, wobei die Alkylgruppe in den genannten (C₁- C₄)-Alkyl-, (C₁-C₄)-Alkoxy- und (C₁-C₄)-Alkylthio-Resten jeweils bis zu dreifach mit Fluor substituiert sein kann,
oder
Phenyl, welches mit Halogen, (C₁-C₄)-Alkyl oder Trifluormethyl substituiert sein kann, bedeutet,
R¹² Wasserstoff, Halogen oder (C₁-C₄)-Alkyl bedeutet,
D CH, C-R⁹ oder N bedeutet
und
n die Zahl 0, 1 oder 2 bedeutet,
wobei für den Fall, dass der Substituent R⁹ mehrfach auftritt, seine Bedeutungen gleich oder verschieden sein können,
R¹ für Cyano, Nitro oder eine Gruppe der Formel -C(=O)-R¹³ steht, worin
R¹³ (C₁-C₆)-Alkyl, das mit (C₃-C₇)-Cyloalkyl oder ein- bis dreifach mit Fluor substituiert sein kann, oder Phenyl, das mit Halogen, Cyano, (C₁-C₄)- Alkyl, Trifluormethyl, (C₁-C₄)-Alkoxy oder Trifluormethoxy substituiert sein kann, oder (C₃-C₇)-Cycloalkyl bedeutet,
R² für (C₁-C₄)-Alkyl, Trifluormethyl, Cyclopropyl, Cyclobutyl, (C₁-C₄)-Alkoxy oder (C₁-C₄)-Alkylthio steht,
R³ für (C₁-C₆)-Alkyl, (C₁-C₆)-Alkoxy, Trifluormethoxy, (C₁-C₆)-Alkylthio, Amino, Mono-(C₁-C₆)-alkylamino oder eine Gruppe der Formel -O-SO₂-R¹⁴ steht, wobei die genannten (C₁-C₆)-Alkyl-, (C₁-C₆)-Alkoxy- und (C₁-C₆)-Alkylthio-Reste jeweils mit (C₃-C₇)-Cycloalkyl substituiert sein können
und
R¹⁴ (C₁-C₆)-Alkyl, Trifluormethyl, (C₃-C₇)-Cycloalkyl, Phenyl oder 5- oder 6- gliedriges Heteroaryl mit bis zu zwei Heteroatomen aus der Reihe N, O und/oder S bedeutet, wobei Phenyl und Heteroaryl ihrerseits jeweils ein- oder zweifach, gleich oder verschieden, mit Halogen, Cyano, Nitro, (C₁-C₄)-Alkyl, Trifluor- methyl, (C₁-C₄)-Alkoxy und/oder Trifluormethoxy substituiert sein kön- nen,
und
R⁴ für Wasserstoff, Fluor, (C₁-C₄)-Alkyl, Trifluormethyl, (C₁-C₄)-Alkoxy, Trifluor- methoxy, (C₁-C₄)-Alkylthio, Amino, Mono-(C₁-C₄)-alkylamino oder Di-(C₁-C₄)- alkylamino steht,
sowie ihre Salze, Solvate und Solvate der Salze.

2. Verbindung der Formel (I) nach Anspruch 1, in welcher
Ar für eine Gruppe der Formel 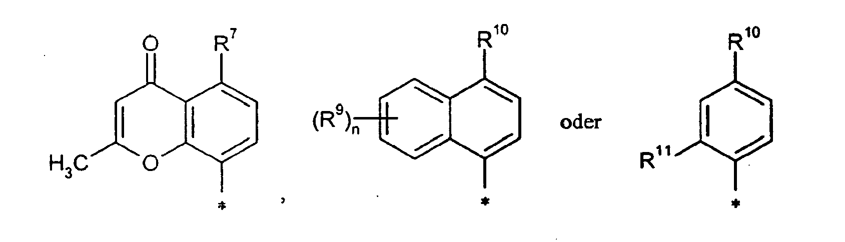
steht, worin
* die Verknüpfungsstelle bedeutet,
R⁷ Wasserstoff, Fluor, Chlor oder Cyano bedeutet,
R⁹ Fluor, Chlor, Methyl oder Ethyl bedeutet,
R¹⁰ Cyano oder Nitro bedeutet,
R¹¹ Chlor, Brom, (C₁-C₄)-Alkyl, Trifluormethyl, (C₁-C₄)-Alkoxy, Trifluor- methoxy, (C₁-C₄)-Alkylthio oder Trifluormethylthio bedeutet,
und
n die Zahl 0 oder 1 bedeutet,
R¹ für Cyano, Acetyl oder Trifluoracetyl steht,
R² für Methyl oder Trifluormethyl steht,
R³ für (C₁-C₄)-Alkoxy, Trifluormethoxy oder eine Gruppe der Formel -O-SO₂-R¹⁴ steht, worin
R¹⁴ (C₁-C₄)-Alkyl, Trifluormethyl, (C₃-C₆)-Cycloalkyl, Phenyl oder Thienyl bedeutet, wobei Phenyl und Thienyl ihrerseits jeweils ein- oder zweifach, gleich oder verschieden, mit Fluor, Chlor, Cyano, Methyl, Trifluormethyl, Methoxy und/oder Trifluormethoxy substituiert sein können,
und
R⁴ für Wasserstoff, Fluor oder Methyl steht,
sowie ihre Salze, Solvate und Solvate der Salze.

3. Verbindung der Formel (I) nach Anspruch 1 oder 2, in welcher
Ar für eine Gruppe der Formel 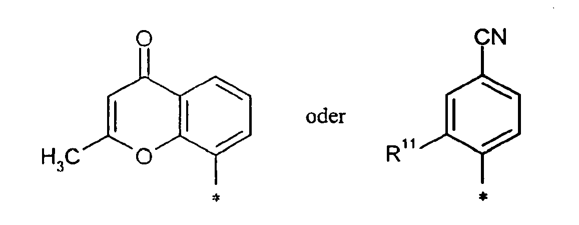
steht, worin
* die Verknüpfungsstelle
und
R¹¹ Ethyl, Methoxy oder Trifluormethoxy bedeutet,
R¹ für Cyano oder Acetyl steht,
R² für Methyl oder Trifluormethyl steht,
R³ für (C₁-C₃)-Alkoxy oder eine Gruppe der Formel -O-SO₂-R¹⁴ steht, worin
R¹⁴ (C₁-C₃)-Alkyl bedeutet,
und
R⁴ für Wasserstoff oder Methyl steht,
sowie ihre Salze, Solvate und Solvate der Salze.

4. Verfahren zur Herstellung von Verbindungen der Formel (I), wie in den Ansprüchen 1 bis 3 definiert, in welchen R³ für gegebenenfalls mit (C₃-C₇)-Cycloalkyl substituiertes (C₁-C₆)-Alkoxy, Trifluormethoxy oder eine Gruppe der Formel -O-SO₂-R¹⁴ steht, worin R¹⁴ die in den Ansprüchen 1 bis 3 angegebene Bedeutung hat,
**dadurch gekennzeichnet, dass** man eine Verbindung der Formel (II) 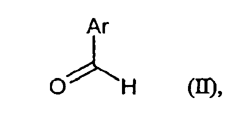 in welcher Ar die in den Ansprüchen 1 bis 3 angegebene Bedeutung hat,
entweder
[A] in einem einstufigen (Eintopf-Reaktion) oder zweistufigen Verfahren mit einer Verbindung der Formel (III) 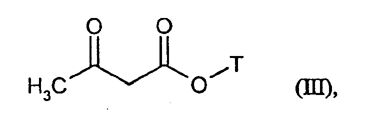 in welcher
T für Methyl oder Ethyl steht,
und einer Verbindung der Formel (IV) 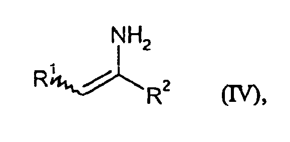 in welcher R¹ und R² die in den Ansprüchen 1 bis 3 angegebenen Bedeutungen haben,
zu einer Verbindung der Formel (V) 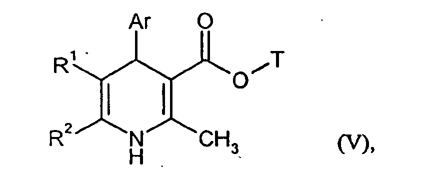 in welcher Ar, R¹, R² und T jeweils die oben angegebenen Bedeutungen haben, umsetzt und diese dann in einem inerten Lösungsmittel mit s-Triazin in Gegenwart einer Base zu einer Verbindung der Formel (VIa) 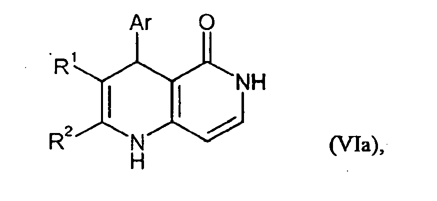 in welcher Ar, R¹ und R² jeweils die oben angegebenen Bedeutungen haben, reagiert
oder
[B] in einem einstufigen (Eintopf-Reaktion) oder zweistufigen Verfahren mit einer Verbindung der Formel (VII) 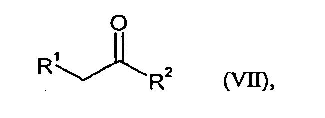 in welcher R¹ und R² die in den Ansprüchen 1 bis 3 angegebenen Bedeutungen haben,
und einer Verbindung der Formel (VIII) 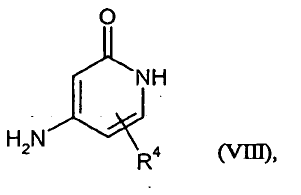 in welcher R⁴ die in den Ansprüchen 1 bis 3 angegebene Bedeutung hat, zu einer Verbindung der Formel (VI) 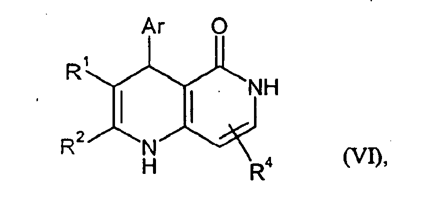 in welcher Ar, R¹, R² und R⁴ jeweils die oben angegebenen Bedeutungen haben, umsetzt
und anschließend die Verbindungen der Formel (VIa) bzw. (VI) in einem inerten Lösungsmittel gegebenenfalls in Gegenwart einer Base mit einer Verbindung der Formel (IX) oder einem Trialkyloxonium-Salz der Formel (X) 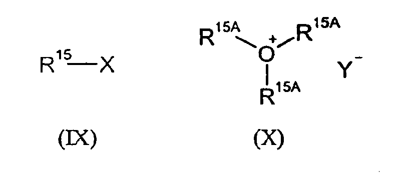 in welchen
R¹⁵ für (C₁-C₆)-Alkyl, das mit (C₃-C₇)-Cycloalkyl substituiert sein kann, oder für Tri- fluormethyl steht,
R^{15A} für Methyl oder Ethyl steht,
X für eine Abgangsgruppe, wie beispielsweise Halogen, Mesylat, Tosylat oder Tri- flat, steht
und
Y⁻ für ein nicht-nukleophiles Anion, wie beispielsweise Tetrafluoroborat, steht,
oder in Gegenwart einer Säure mit einem Orthoameisensäureester der Formel (XI) 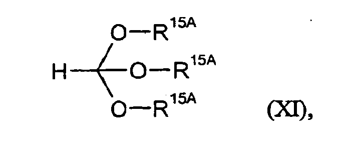 in welcher R^{15A} die oben angegebene Bedeutung hat, zu Verbindungen der Formel (I-A) 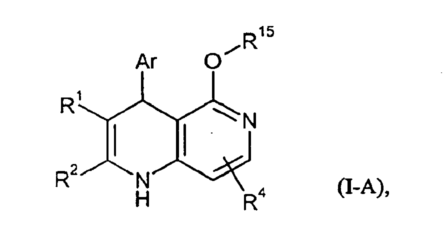 in welcher Ar, R¹, R², R⁴ und R¹⁵ jeweils die oben angegebenen Bedeutungen haben, alkyliert
oder die Verbindungen der Formel (VIa) bzw. (VI) in einem inerten Lösungsmittel in Gegenwart einer Base mit einer Verbindung der Formel (XII) 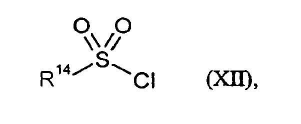 in welcher R¹⁴ die in den Ansprüchen 1 bis 3 angegebene Bedeutung hat, zu Verbindungen der Formel (I-B) 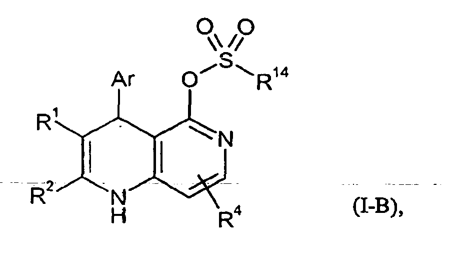 in welcher Ar, R¹, R², R⁴ und R¹⁴ jeweils die oben angegebenen Bedeutungen haben, umsetzt
und gegebenenfalls die resultierenden Verbindungen der Formel (I-A) bzw. (I-B) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

5. Verbindung der Formel (I), wie in einem der Ansprüche 1 bis 3 definiert, zur Behandlung und/oder Prophylaxe von Krankheiten.

6. Verwendung einer Verbindung der Formel (I), wie in einem der Ansprüche 1 bis 3 definiert, zur Herstellung eines Arzneimittels zur Behandlung und/oder Prophylaxe von Aldosteronismus, Bluthochdruck, chronischer Herzinsuffizienz, den Folgen eines Myokardinfarkts, Leberzirrhose, Niereninsuffizienz und Hirnschlag.

7. Arzneimittel enthaltend eine Verbindung der Formel (I), wie in einem der Ansprüche 1 bis 3 definiert, in Kombination mit einem inerten, nicht-toxischen, pharmazeutisch geeigneten Hilfsstoff.

8. Arzneimittel enthaltend eine Verbindung der Formel (I), wie in einem der Ansprüche 1 bis 3 definiert, in Kombination mit einem oder mehreren weiteren Wirkstoffen ausgewählt aus der Gruppe bestehend aus ACE-Inhibitoren, Renin-Inhibitoren, Angiotensin II-Rezeptor-Antagonisten, Beta-Blocker, Acetylsalicylsäure, Diuretika, Kalium-Supplements, Calcium-Antagonisten, Statine, Digitalis (Digoxin)-Derivate, Calcium-Sensitizer, Nitrate sowie Antithrombotika.

9. Arzneimittel nach Anspruch 7 oder 8 zur Behandlung und/oder Prophylaxe von Aldosteronismus, Bluthochdruck, chronischer Herzinsuffizienz, den Folgen eines Myokardinfarkts, Leberzirrhose, Niereninsuffizienz und Hirnschlag.

## Claims

1. Compound of the formula (I) 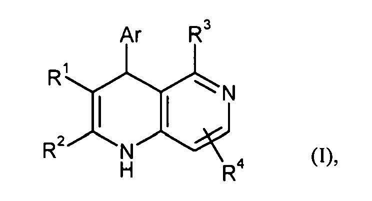 in which
Ar is a group of the formula 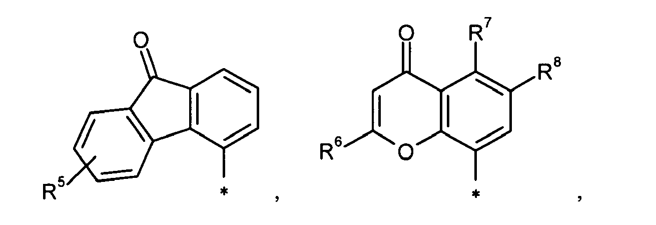 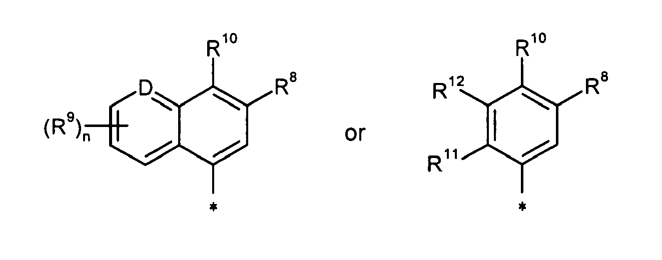
in which
* is the linkage point,
R⁵ is hydrogen or halogen,
R⁶ is methyl or ethyl,
R⁷ is hydrogen, fluorine, chlorine, cyano, nitro, trifluoromethyl or (C₁-C₄)-alkyl,
R⁸ is hydrogen or fluorine,
R⁹ is halogen, (C₁-C₄)-alkyl, trifluoromethyl, (C₁-C₄)-alkoxy or trifluoromethoxy,
R¹⁰ is cyano or nitro,
R¹¹ is hydrogen, halogen, (C₁-C₄)-alkyl, (C₁-C₄)-alkoxy, (C₁-C₄)-alkylthio or di-(C₁-C₄)-alkylamino, it being possible for the alkyl group in said (C₁-C₄)-alkyl, (C₁-C₄)-alkoxy and (C₁-C₄)-alkylthio radicals in each case to be substituted up to three times by fluorine,
or
phenyl, which may be substituted by halogen, (C₁-C₄)-alkyl or trifluoromethyl,
R¹² is hydrogen, halogen or (C₁-C₄)-alkyl,
D is CH, C-R⁹ or N,
and
n is the number 0, 1 or 2, it being possible in the case where the substituent R⁹ occurs more than once for its meanings to be identical or different,
R¹ is cyano, nitro or a group of the formula -C(=O)-R¹³ in which
R¹³ is (C₁-C₆)-alkyl which may be substituted by (C₃-C₇)-cycloalkyl or once to three times by fluorine, or phenyl which may be substituted by halogen, cyano, (C₁-C₄)-alkyl, trifluoromethyl, (C₁-C₄)-alkoxy or trifluoromethoxy, or (C₃-C₇)-cycloalkyl,
R² is (C₁-C₄)-alkyl, trifluoromethyl, cyclopropyl, cyclobutyl, (C₁-C₄)-alkoxy or (C₁-C₄)-alkylthio,
R³ is (C₁-C₆)-alkyl, (C₁-C₆)-alkoxy, trifluoromethoxy, (C₁-C₆)-alkylthio, amino, mono-(C₁-C₆)-alkylamino or a group of the formula -O-SO₂R¹⁴, where said (C₁-C₆)-alkyl, (C₁-C₆)-alkoxy and (C₁-C₆)-alkylthio radicals may in each case be substituted by (C₃-C₇)-cycloalkyl,
and
R¹⁴ is (C₁-C₆)-alkyl, trifluoromethyl, (C₃-C₇)-cycloalkyl, phenyl or 5- or 6- membered heteroaryl having up to two heteroatoms from the series N, O and/or S, it being possible for phenyl and heteroaryl in turn each to be substituted once or twice, identically or differently, by halogen, cyano, nitro, (C₁-C₄)-alkyl, trifluoromethyl, (C₁-C₄)-alkoxy and/or trifluoromethoxy,
and
R⁴ is hydrogen, fluorine, (C₁-C₄)-alkyl, trifluoromethyl, (C₁-C₄)-alkoxy, trifluoromethoxy, (C₁-C₄)-alkylthio, amino, mono-(C₁-C₄)-alkylamino or di- (C₁-C₄)-alkylamino,
and the salts, solvates and solvates of the salts thereof.

2. Compound of the formula (I) according to Claim 1, in which
Ar is a group of the formula 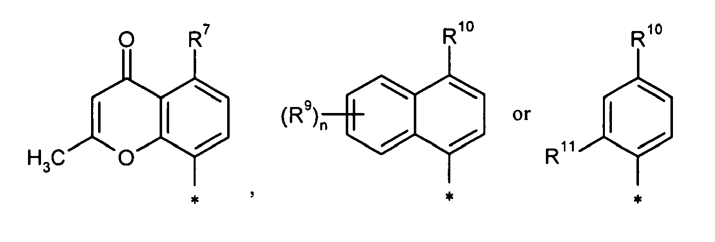
in which
* is the linkage point,
R⁷ is hydrogen, fluorine, chlorine or cyano,
R⁹ is fluorine, chlorine, methyl or ethyl,
R¹⁰ is cyano or nitro,
R¹¹ is chlorine, bromine, (C₁-C₄)-alkyl, trifluoromethyl, (C₁-C₄)-alkoxy, trifluoromethoxy, (C₁-C₄)-alkylthio or trifluoromethylthio,
and
n is the number 0 or 1,
R¹ is cyano, acetyl or trifluoroacetyl,
R² is methyl or trifluoromethyl,
R³ is (C₁-C₄)-alkoxy, trifluoromethoxy or a group of the formula -O-SO₂-R¹⁴ in which
R¹⁴ is (C₁-C₄)-alkyl, trifluoromethyl, (C₃-C₆)-cycloalkyl, phenyl or thienyl,
where phenyl and thienyl in turn may each be substituted once or twice, identically or differently by fluorine, chlorine, cyano, methyl, trifluoromethyl, methoxy and/or trifluoromethoxy,
and
R⁴ is hydrogen, fluorine or methyl,
and the salts, solvates and solvates of the salts thereof.

3. Compound of the formula (I) according to Claim 1 or 2, in which
Ar is a group of the formula 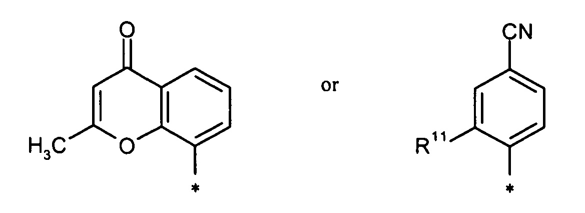
in which
* is the linkage point
and
R¹¹ is ethyl, methoxy or trifluoromethoxy,
R¹ is cyano or acetyl,
R² is methyl or trifluoromethyl,
R³ is (C₁-C₃)-alkoxy or a group of the formula -O-SO₂-R¹⁴ in which
R¹⁴ is (C₁-C₃)-alkyl,
and
R⁴ is hydrogen or methyl,
and the salts, solvates and solvates of the salts thereof.

4. Process for preparing compounds of the formula (I) as defined in Claims 1 to 3, in which R³ is optionally (C₃-C₇)-cycloalkyl-substituted (C₁-C₆)-alkoxy, trifluoromethoxy or a group of the formula -O-SO₂-R¹⁴ in which R¹⁴ has the meaning indicated in Claims 1 to 3,
**characterized in that** a compound of the formula (II) 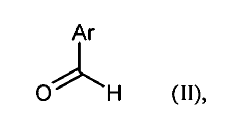 in which Ar has the meaning indicated in Claims 1 to 3,
either
[A] is reacted in a one-stage (one-pot reaction) or two-stage process with a compound of the formula (III) 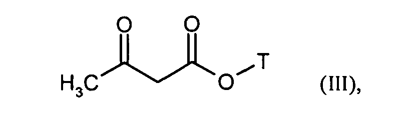
in which
T is methyl or ethyl,
and a compound of the formula (IV) 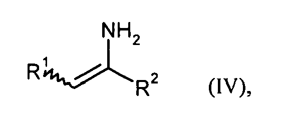 in which R¹ and R² have the meanings indicated in Claims 1 to 3, to give a compound of the formula (V) 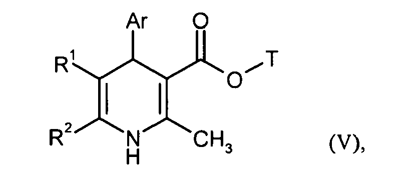 in which Ar, R¹, R² and T each have the meanings indicated above,
and the latter is then reacted in an inert solvent with s-triazine in the presence of a base to give a compound of the formula (VIa) 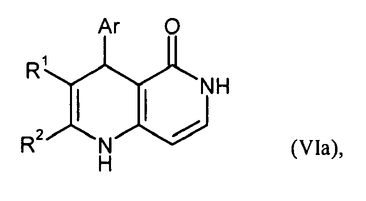 in which Ar, R¹ and R² each have the meanings indicated above,
or
[B] is reacted in a one-stage (one-pot reaction) or two-stage process with a compound of the formula (VII) 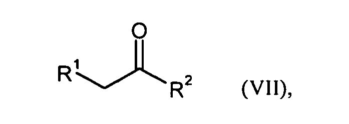
in which R¹ and R² have the meanings indicated in Claims 1 to 3, and a compound of the formula (VIII) 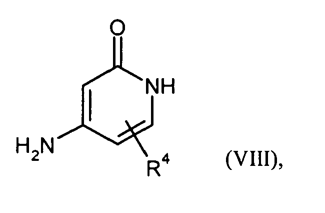 in which R⁴ has the meaning indicated in Claims 1 to 3, to give a compound of the formula (VI) 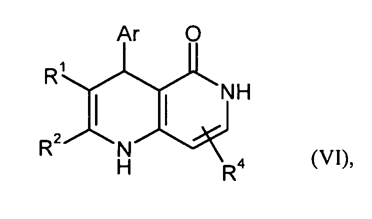 in which Ar, R¹, R² and R⁴ each have the meanings indicated above,
and then the compounds of the formula (VIa) or (VI) are alkylated in an inert solvent, where appropriate in the presence of a base, with a compound of the formula (IX) or a trialkyloxonium salt of the formula (X) 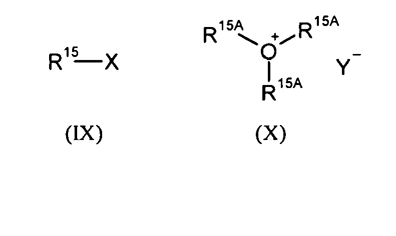 in which
R¹⁵ is (C₁-C₆)-alkyl which may be substituted by (C₃-C₇)-cycloalkyl or is trifluoromethyl,
R^{15A} is methyl or ethyl,
X is a leaving group such as, for example, halogen, mesylate, tosylate or triflate,
and
Y⁻ is a non-nucleophilic anion such as, for example, tetrafluoroborate,
or in the presence of an acid with an orthoformic ester of the formula (XI) 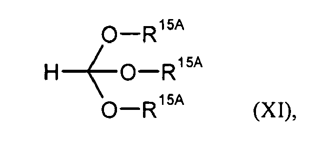 in which R^{15A} has the meaning indicated above, to give compounds of the formula (I-A) 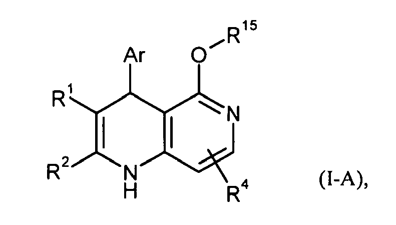 in which Ar, R¹, R², R⁴ and R¹⁵ each have the meanings indicated above,
or the compounds of the formula (VIa) or (VI) are reacted in an inert solvent in the presence of a base with a compound of the formula (XII) 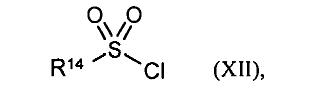 in which R¹⁴ has the meaning indicated in Claims 1 to 3, to give compounds of the formula (I-B) 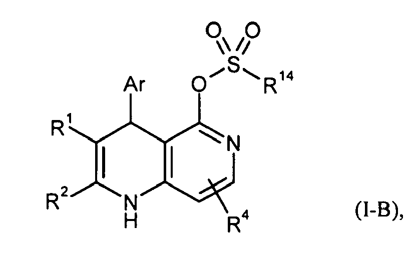 in which Ar, R¹, R², R⁴ and R¹⁴ each have the meanings indicated above,
and where appropriate the resulting compounds of the formula (I-A) or (I-B) are separated by methods known to the skilled worker into their enantiomers and/or diastereomers, and/or converted with the appropriate (i) solvents and/or (ii) bases or acids into the solvates, salts and/or solvates of the salts thereof.

5. Compound of the formula (I) as defined in any of Claims 1 to 3 for the treatment and/or prophylaxis of diseases.

6. Use of a compound of the formula (I) as defined in any of Claims 1 to 3 for the manufacture of a medicament for the treatment and/or prophylaxis of aldosteronism, high blood pressure, chronic heart failure, the sequelae of a myocardial infarction, cirrhosis of the liver, renal failure and stroke.

7. Medicament comprising a compound of the formula (I) as defined in any of Claims 1 to 3 in combination with an inert, non-toxic, pharmaceutically suitable excipient.

8. Medicament comprising a compound of the formula (I) as defined in any of Claims 1 to 3 in combination with one or more further active ingredients selected from the group consisting of ACE inhibitors, renin inhibitors, angiotensin II receptor antagonists, beta-blockers, acetylsalicylic acid, diuretics, potassium supplements, calcium antagonists, statins, digitalis (digoxin) derivatives, calcium sensitizers, nitrates and antithrombotics.

9. Medicament according to Claims 7 or 8 for the treatment and/or prophylaxis of aldosteronism, high blood pressure, chronic heart failure, the sequelae of a myocardial infarction, cirrhosis of the liver, renal failure and stroke.

## Revendications

1. Composé de la formule (I) 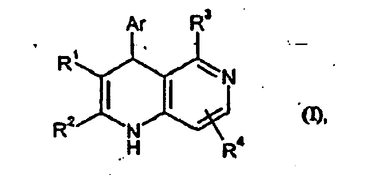 dans laquelle
Ar est un groupe de la formule 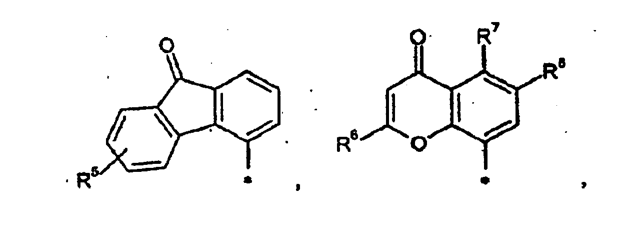 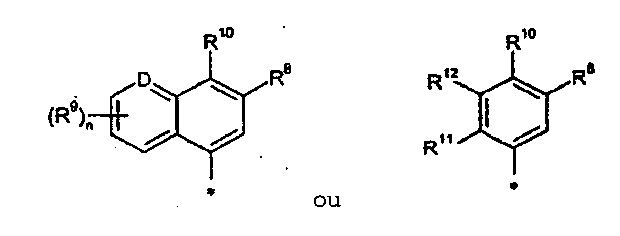 dans laquelle
* est le point de liaison,
R⁵ est un hydrogène ou un halogène,
R⁶ est un méthyle ou un éthyle,
R⁷ est un hydrogène, fluor, chlore, cyano, nitro, trifluorométhyle ou alkyle en (C₁-C₄),
R⁸ est un hydrogène ou fluor,
R⁹ est un halogène, alkyle en (C₁-C₄), trifluorométhyle, alcoxy en (C₁-C₄) ou trifluorométhoxy,
R¹⁰ est un cyano ou nitro,
R¹¹ est un hydrogène, halogène, alkyle en (C₁-C₄), alcoxy en (C₁-C₄), alkylthio en (C₁-C₄) ou dialkylamino en (C₁-C₄), dans lequel le groupe alkyle dans les radicaux alkyle en (C₁-C₄), alcoxy en (C₁-C₄) et alkylthio en (C₁-C₄) peut chaque fois être substitué jusqu'à trois fois par un fluor,
ou
est un phényle, qui peut être substitué par un halogène, alkyle en (C₁-C₄) ou trifluorométhyle,
R¹² est un hydrogène, halogène ou alkyle en (C₁-C₄),
D est un CH, C-R⁹ ou N
et
n est le nombre 0, 1 ou 2,
dans laquelle, pour le cas où le substituant R⁹ apparaît plusieurs fois, ses significations peuvent être identiques ou différentes,
R¹ est un groupe cyano, nitro ou un groupe de la formule -C(=O)-R¹³, dans laquelle
R¹³ est un alkyle en (C₁-C₆), qui peut être substitué par un cycloalkyle en (C₃-C₇) ou de une à trois fois par un fluor, ou un phényle, qui peut être substitué par un halogène, cyano, alkyle en (C₁-C₄), trifluorométhyle, alcoxy en (C₁-C₄) ou trifluorométhoxy, ou un cycloalkyle en (C₃-C₇),
R² est un alkyle en (C₁-C₄), trifluorométhyle, cyclopropyle, cyclobutyle, alcoxy en (C₁-C₄) ou alkylthio en (C₁-C₄),
R³ est un alkyle en (C₁-C₆) , alcoxy en (C₁-C₆) , trifluorométhoxy, alkylthio en (C₁-C₆), un groupe amino, monoalkylamino en (C₁-C₆) ou un groupe de la formule -O-SO₂-R¹⁴,
dans laquelle les radicaux alkyle en (C₁-C₆), alcoxy en (C₁-C₆) et alkylthio en (C₁-C₆) mentionnés peuvent chaque fois être substitués par un cycloalkyle en (C₃-C₇)
et
R¹⁴ est un alkyle en (C₁-C₆), trifluorométhyle, cycloalkyle en (C₃-C₇), un phényle ou un hétéroaryle à 5 ou 6 chaînons avec jusqu'à deux hétéroatomes de la série N, O et/ou S,
dans laquelle phényle et hétéroaryle peuvent de leur côté être substitués respectivement une ou deux fois, de manière identique ou différente, par un halogène, cyano, nitro, alkyle en (C₁-C₄), trifluorométhyle, alcoxy en (C₁-C₄) et/ou trifluorométhoxy,
et
R⁴ est un hydrogène, fluor, alkyle en (C₁-C₄) trifluorométhyle, alcoxy en (C₁-C₄), trifluorométhoxy, alkylthio en (C₁-C₄), un groupe amino, monoalkylamino en (C₁-C₄) ou dialkylamino en (C₁-C₄),
ainsi que leurs sels, solvates et solvates des sels.

2. Composé de la formule (I) selon la revendication 1, dans laquelle
Ar est un groupe de la formule 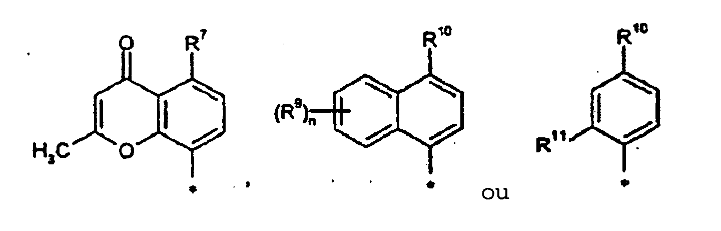 dans laquelle
* est le point de liaison,
R⁷ est un hydrogène, fluor, chlore ou cyano,
R⁹ est un fluor, chlore, méthyle ou éthyle,
R¹⁰ est un groupe cyano ou nitro,
R¹¹ est un chlore, brome, alkyle en (C₁-C₄), trifluorométhyle, alcoxy en (C₁-C₄), trifluorométhoxy, alkylthio en (C₁-C₄) ou trifluorométhylthio,
et
n est le nombre 0 ou 1,
R¹ est un groupe cyano, acétyle ou trifluoroacétyle, R² est un méthyle ou trifluorométhyle,
R³ est un alcoxy en (C₁-C₄), trifluorométhoxy ou un groupe de la formule -O-SO₂-R¹⁴, dans laquelle
R¹⁴ est un alkyle en (C₁-C₄), trifluorométhyle, cycloalkyle en (C₃-C₆), un phényle ou un thiényle,
dans laquelle le phényle et le thiényle peuvent de leur côté être respectivement substitués un ou deux fois, de manière identique ou différente, par un fluor, chlore, cyano, méthyle, trifluorométhyle, méthoxy et/ou trifluorométhoxy,
et
R⁴ est un hydrogène, fluor ou méthyle,
ainsi que leurs sels, solvates et solvates des sels.

3. Composé de la formule (I) selon la revendication 1 ou 2, dans laquelle
Ar est un groupe de la formule 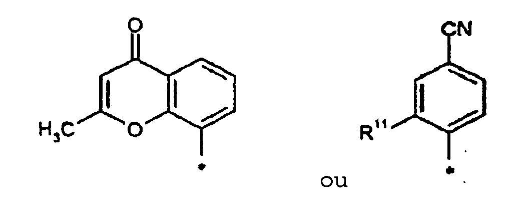 dans laquelle
* est le point de liaison
et
R¹¹ est un éthyle, méthoxy ou trifluorométhoxy,
R¹ est un cyano ou acétyle,
R² est un méthyle ou trifluorométhyle,
R³ est un alcoxy en (C₁-C₃) ou un groupe de la formule -O-SO₂-R¹⁴, dans laquelle R¹⁴ est un alkyle en (C₁-C₃),
et
R⁴ est un hydrogène ou méthyle,
ainsi que leurs sels, solvates et solvates des sels.

4. Procédé pour la fabrication de composés de la formule (I), tels qu'ils sont définis dans les revendications 1 à 3, dans lesquels R³ est un alcoxy en (C₁-C₆) éventuellement substitué par un cycloalkyle en (C₃-C₇), un trifluorométhoxy ou un groupe de la formule -O-SO₂-R¹⁴, dans laquelle R¹⁴ a la signification indiquée dans les revendications 1 à 3,
**caractérisé en ce que** l'on convertit un composé de la formule (II) 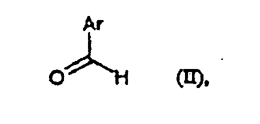 dans laquelle Ar a la signification indiquée dans les revendications 1 à 3,
soit
[A] dans une réaction à une étape (réaction en cuve unique) ou un procédé en deux étapes avec un composé de la formule (III) 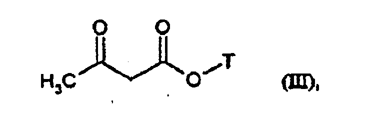
dans laquelle
T est un méthyle ou éthyle,
et avec un composé de la formule (IV) 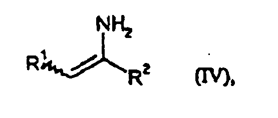 dans laquelle R¹ et R² ont la signification indiquée dans les revendications 1 à 3,
en un composé de la formule (V) 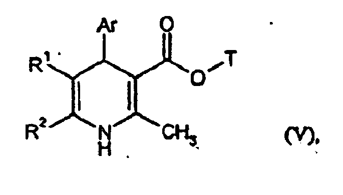 dans laquelle Ar, R¹, R² et T ont respectivement les significations indiquées plus haut,
et on fait ensuite réagir celui-ci dans un solvant inerte avec la s-triazine en présence d'une base en un composé de la formule (VIa) 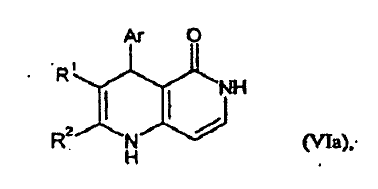 dans laquelle Ar, R¹ et R² ont respectivement les significations indiquées plus haut,
soit
[B] dans une réaction en une étape (réaction en cuve unique) ou un procédé en deux étapes avec un composé de la formule (VII) 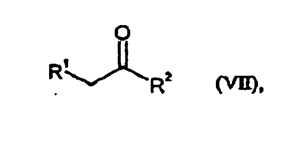
dans laquelle R¹ et R² ont la signification indiquée dans les revendications 1 à 3,
et avec un composé de la formule (VIII) 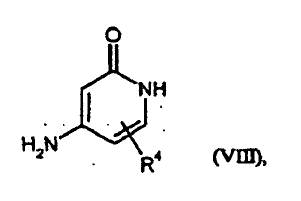 dans laquelle R⁴ a la signification indiquée dans les revendications 1 à 3,
en un composé de la formule (VI) 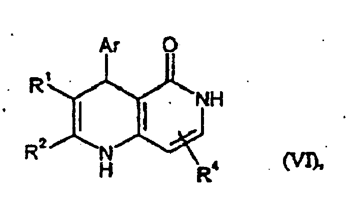 dans laquelle Ar, R¹, R² et R⁴ ont respectivement les significations indiquées plus haut,
et on alkyle ensuite les composés de la formule (VIa) ou (VI) dans un solvant inerte, éventuellement en présence d'une base, avec un composé de la formule (IX) ou un sel de trialkyloxonium de la formule (X) 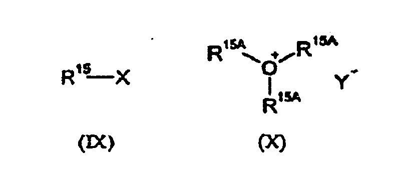 dans lesquelles
R¹⁵ est un alkyle en (C₁-C₆), qui peut être substitué par un cycloalkyle en (C₃-C₇), ou un trifluorométhyle, R^{15A} est un méthyle ou un éthyle,
X est un groupe partant, comme par exemple un halogène, un mésylate, un tosylate ou un triflate,
et
Y⁻ est un anion non nucléophile, comme par exemple le tétrafluoroborate,
ou en présence d'un acide avec un ester de l'acide orthoformique de la formule (XI) 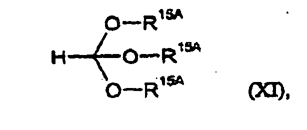 dans laquelle R^{15A} a la signification indiquée plus haut,
en composés de la formule (I-A) 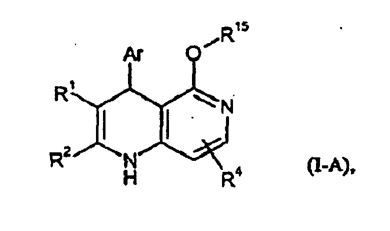 dans laquelle Ar, R¹, R², R⁴ et R¹⁵ ont respectivement les significations indiquées plus haut,
ou on convertit les composés de la formule (VIa) ou (VI) dans un solvant inerte en présence d'une base avec un composé de la formule (XII) 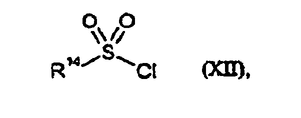 dans laquelle R¹⁴ a la signification indiquée dans les revendications 1 à 3,
en composés de la formule (I-B) 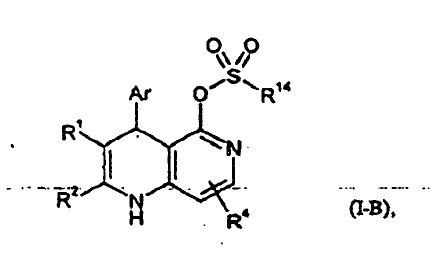 dans laquelle Ar, R¹, R², R⁴ et R¹⁴ ont respectivement les significations indiquées plus haut,
et on sépare éventuellement les composés obtenus de la formule (I-A) ou (I-B), selon des procédés connus de l'homme du métier, en leurs énantiomères et/ou leurs diastéréomères
et/ou on les transforme avec les (i) solvants correspondants et/ou (ii) bases correspondantes ou acides correspondants en leurs solvates, sels et/ou solvates des sels.

5. Composé de la formule (I), tel que défini dans l'une quelconque des revendications 1 à 3, pour le traitement et/ou la prophylaxie de maladies.

6. Utilisation d'un composé de la formule (I), tel que défini dans l'une quelconque des revendications 1 à 3, pour la production d'un médicament pour le traitement et/ou la prophylaxie de l'aldostéronisme, de l'hypertension artérielle, d'une insuffisance cardiaque chronique, des séquelles d'un infarctus du myocarde, de la cirrhose du foie, d'une insuffisance rénale et d'une attaque cérébrale.

7. Médicament contenant un composé de la formule (I), tel que défini dans l'une quelconque des revendications 1 à 3, en combinaison avec un agent accessoire inerte, non toxique, pharmaceutiquement approprié.

8. Médicament contenant un composé de la formule (I), tel que défini dans l'une quelconque des revendications 1 à 3, en combinaison avec une ou plusieurs substances actives choisies dans le groupe composé des inhibiteurs ACE, inhibiteurs de la rénine, antagonistes des récepteurs de l'angiotensine II, bêtabloquants, acide acétylsalicylique, diurétiques, suppléments de potassium, antagonistes de calcium, statine, dérivés digitaliques (digoxine), sensibilisateurs au calcium, nitrates ainsi que antithrombotiques.

9. Médicament selon la revendication 7 ou 8 pour le traitement et/ou la prophylaxie de l'aldostéronisme, de l'hypertension artérielle, d'une insuffisance cardiaque chronique, des séquelles d'un infarctus du myocarde, d'une cirrhose du foie, d'une insuffisance rénale et d'une attaque cérébrale.
